# EUROPEAN PATENT APPLICATION

(11) **EP 4 273 150 A1**
(43) Date of publication of application: **08.11.2023**
(21) Application number: 21914226.2
(22) Date of filing: 27.12.2021
(51) Int. Cl.: C07D 491/056, C07D 491/048, C07D 498/00, C07D 493/00, C07D 471/04, A61K 31/47, A61K 31/423, A61K 31/136, A61P 27/02, A61P 9/00, A61P 11/00, A61P 15/00, A61P 25/00, A61P 35/00, A61P 37/00

(54) **TRICYCLIC COMPOUND, AND PREPARATION METHOD THEREFOR AND MEDICAL USE THEREOF**

(30) Priority: 29.12.2020 CN 202011591150
(71) Applicant: The National Institutes of Pharmaceutical R&D Co., Ltd, Changping District Beijing 102206 (CN)
(72) Inventor: YIN, Huijun, Beijing 102206 (CN); YAN, Xu, Beijing 102206 (CN); LIU, Guobiao, Beijing 102206 (CN); SHI, Jianxin, Beijing 102206 (CN); CHEN, Shizhu, Beijing 102206 (CN)
(74) Representative: EP&C
(86) International application number: PCT/CN2021/141502
(87) International publication number: WO 2022/143489

(57) **Abstract**

A tricyclic compound having the structure represented by general formula (I), a pharmaceutical composition thereof, a preparation method therefor and the use thereof in the prevention and treatment of a variety of diseases caused by toxic aldehydes.

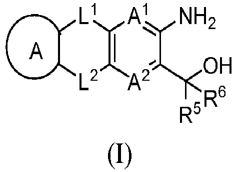

## Description

### TECHNICAL FIELD

The present application relates to a novel tricyclic compound, a pharmaceutical composition comprising the same, a method for preparing the same, and a use thereof in preventing and treating various diseases caused by toxic aldehydes.

### BACKGROUND

The active carbonyl-containing compounds such as aldhhehydes generally refer to any highly active electrophilic compounds containing one or more carbonyl groups. They originate from a wide range of sources, and participate in various life behaviors and physiological activities. Aldehydes are everywhere in the environment from air, water and soil to food, daily necessities and indoor living places (Koren and Bisesi, CRC press, 2002). Natural sources of aldehyde include plants, animals, microorganisms and natural life processes (O'Brien et al., Critical reviews in toxicology, 2005, 35(7): 609-662). Among them, plants are the main source of aldehyde, for example, vanillin, cinnamaldehyde, benzaldehyde, citral and crotonaldehyde are usually plant-derived aldehydes (Koren and Bisesi, CRC press, 2002; Feron et al., Mutation Research/Genetic Toxicology, 1991, 259(3-4): 363-385). Artificial sources mainly include car exhaust, burning of substances, smoking of cigarettes, overcooking of certain foods, industrial emissions and the like, and aldehydes originated from these sources include formaldehyde, acetaldehyde, benzaldehyde, propionaldehyde, acrolein, glyoxal, glutaraldehyde, crotonaldehyde, m-tolualdehyde, 2,5-dimethylbenzaldehyde, 3-hydroxybenzaldehyde and the like (Koren and Bisesi, CRC press, 2002; O'Brien et al., Critical reviews in toxicology, 2005, 35(7): 609-662; Marnett *et al*., Health Effects Institute. National Academy, 1988). In addition to these sources, aldehydes are also produced in the body through various physiological processes, for example, aldehydes are produced in endogenous pathways such as lipid peroxidation, biotransformation of drug and food, intermediates in biosynthetic and catabolic pathways, and final products of enzymatic reactions (Feron et al., Mutation Research/Genetic Toxicology, 1991, 259(3-4): 363-385; O'Brien et al., Critical reviews in toxicology, 2005, 35(7): 609-662). Generally, the aldehydes produced through these metabolic pathways include 4-hydroxy-2-nonenal, nonenal, acetaldehyde, acrolein, glutamic acid γ-semialdehyde, malondialdehyde, glyoxal, methylglyoxal, glycolaldehyde, glyceraldehyde, lactaldehyde and the like (Esterbauer et al., Free radical Biology and medicine, 1991, 11(1): 81-128; Voulgaridou et al., Mutation Research/Fundamental and Molecular Mechanisms of Mutagenesis, 2011, 711(1-2): 13-27; Nelson et al., Current opinion in pharmacology, 2017, 33: 56-63; Barrera et al., Antioxidants & redox signaling, 2015, 22(18): 1681-1702).

Most active carbonyl compounds have high reactivity, and can act as electrophiles to undergo addition reactions with nucleophiles. This electrophilic-nucleophilic reaction and the resulting adducts are the main reasons for the physiological functions and toxicity of aldehydes. The overexpression and elimination disorder involve in, but not limited to, mitochondrial disruption, membrane damage, endoplasmic reticulum stress, activation of inflammatory mediators, immune dysfunction and the like (Moghe et al., Toxicological Sciences, 2015, 143(2): 242-255). For example, 4-hydroxy-2-nonenal and nonenal produced during lipid peroxidation can easily react with low molecular weight compounds or macromolecules (such as proteins and DNA) in physiological system, and there are reports demonstrate that adduct-related diseases include cancer, neurodegenerative disease, chronic inflammatory disease, autoimmune disease and the like (Barrera et al., Antioxidants & redox signaling, 2015, 22(18): 1681-1702). Aldehydes are in equilibrium in normal physiological system, when this equilibrium is disrupted, the production of adducts of aldehydes with proteins, nucleic acids and phospholipids and the production and accumulation of advanced lipid oxidation end products (ALE) and advanced glycation end products (AGE) will occur (Singh et al., The Korean Journal of Physiology & Pharmacology, 2014, 18(1): 1-14). For example, methylglyoxal, a by-product of glucose metabolism, can be adducted with arginine (Arg) and lysine (Lys) on proteins to form imidazolidinone and carboxyethyllysine, and glyoxal, a product of the autoxidation of carbohydrate and ascorbate, can form carboxymethyllysine with Lys. The dicarbonyl group can directly and specifically react with Arg residue, which has the highest probability of being located in the functional site of protein. Therefore, Arg modification will result in the loss of side chain guanidino groups and important functional Arg residues. Under normal conditions, the protein carbonylation level is usually 1 to 5%, but with aging and disease, the level will increase. Proteins modified by methylglyoxal and glyoxal will be recognized by the body as misfolded proteins and directly degraded by the proteasome (Thomalley et al., Nucleic acids research, 2010, 38(16): 5432-5442).

Carbonyl stress caused by reactive carbonyl compound can lead to nonspecific modification of protein and genetic material, resulting in cytotoxicity. Carbonyl stress can mediate mitochondrial protein dysfunction and increased reactive oxygen species formation (Yao and Brownlee, Diabetes, 2010, 59(1): 249-255), expression of crystallin and inflammatory protein (Yao and Brownlee, Diabetes, 2010, 59(1): 249-255; Ahmed et al., Diabetologia, 2005, 48(8): 1590-1603), lipid-related lipoprotein abnormalities (Rabbani et al., Diabetologia. 233 SPRING ST, NEW YORK, NY 10013 USA: SPRINGER, 2009, 52: S498-S499), activation of mitochondrial apoptosis pathway (Chan et al., Journal of cellular biochemistry, 2007, 100(4): 1056-1069), and cell detachment from the extracellular matrix and apoptosis (Dobler et al., Diabetes, 2006, 55(7): 1961-1969). In addition to carbonyl stress, the protein damage caused by reactive carbonyl species can also modify the amino acid side chain residue of specific protein by enhancing the oxidative stress formed by the generation of reactive oxygen species, resulting in protein carbonylation and changes in protein activity and function. The carbonylation of protein reversibly or irreversibly changes the spatial conformation of polypeptide chain, and partially or completely inhibits protein activity, thereby causing cell dysfunction and tissue damage.

Moreover, highly reactive carbonyl compounds can also lead to reduced enzymatic activity through modification of the enzymatic structure. For example, the reductase activity of hepatic mitochondrial cytochrome C is negatively correlated with the carbonylation of protein, indicating that the oxidation of protein can significantly reduce enzyme activity (Bruno et al., Journal of proteome research, 2009, 8(4): 2070-2078). Active carbonyl species increase the oxidation and carbonylation of protein by inhibiting key cellular enzymes such as glutathione reductase and peroxidase (Shangari et al., Biochemical pharmacology, 2006, 71(11): 1610-1618). In human and mouse, the carbonylation of adipocyte fatty acid-binding protein-4 and epidermal fatty acid-binding protein-5 results in reduced ability to bind fatty acids, reduced lipolysis, and obesity. Fatty acid transporter comprises two cysteine (Cys) residues, which are also susceptible to carbonylation (Febbraio et al., Cellular Lipid Binding Proteins. Springer, Boston, MA, 2002: 193-197). Fatty acid-binding protein prevents the lipotoxicity of long-chain fatty acid by binding or cross-linking with oxidized reactive long-chain fatty acid, and hepatic fatty acid-binding protein depletion will convert nonalcoholic fatty liver to nonalcoholic steatohepatitis (Charlton et al., Hepatology, 2009, 49(4): 1375-1384).

Compared with reactive oxygen species, reactive carbonyl species derived from lipid and carbohydrate are more stable, and can integrate into or even escape cellular degradation and attack targets once formed. Therefore, these soluble active mediators and AGE precursors are not only cytotoxic, but also act as the mediator and communicator of oxidative stress and tissue damage, and play the role of "cytotoxic second messengers", which are risk factors for various diseases throughout the body. Studies have reported that human diseases related to active carbonyl species include cardiovascular diseases, such as atherosclerosis, hypertension, cardiopulmonary dysfunction and the like; respiratory system diseases, such as airway neuroinflammation, chronic obstructive pulmonary disease, respiratory allergy, asthma and the like; neurodegenerative diseases, such as Alzheimer's disease; diabetes and its complications; ocular diseases, such as dry eye, cataract, retinopathy, keratoconus, Fukker's endothelial dystrophy, retinitis pigmentosa, glaucoma, allergic conjunctivitis, uveitis; skin diseases, such as psoriasis, psoriasis, contact dermatitis, atopic dermatitis, acne, Sjögren's syndrome and the like; autoimmune diseases, such as lupus erythematosus and the like; neurological diseases, such as autism, central nervous system toxicity, amyotrophic lateral sclerosis and the like; digestive system diseases, such as neurohepatitis, alcoholic liver disease, non-alcoholic fatty liver disease, ulcerative colitis and the like; and obesity, cancer, and aging-related diseases. Therefore, reduction or elimination of active carbonyl species can improve or alleviate these pathological symptoms.

Treatment of various conditions related to reactive carbonyl species by administration of small molecule therapeutics that act as capture agents for reactive carbonyl species (such as malondialdehyde, 4-hydroxynonenal) has not been proposed in the art. Therefore, there is a need to treat, prevent diseases or conditions in which active carbonyl toxicity is involved in the pathogenesis, and/or reduce the risk of such diseases or conditions. The present invention addresses such need.

### SUMMARY OF THE INVENTION

After deep research, the inventors have designed and synthesized a series of tricyclic compounds, which can be used to prevent and treat a disease related to active carbonyl compound.

Thus, an object of the present invention is to provide a compound of formula (I): 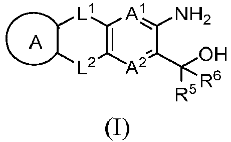 or a mesomer, racemate, enantiomer, diastereomer thereof, or mixture thereof, or a prodrug thereof, or a pharmaceutically acceptable salt thereof,
wherein,
A¹ is selected from the group consisting of N and CR¹;
A² is selected from the group consisting of N and CR²;
L¹ and L² are each independently selected from the group consisting of single bond, CR^{c}R^{d}, NR^{c}, O and S(O)ₘ, and L¹ and L² are not single bonds simultaneously;
ring A is selected from the group consisting of aromatic ring, heteroaromatic ring and heterlcyclic ring, wherein the aromatic ring, heteroaromatic ring and heterlcyclic ring are each optionally further substituted by one or more substituents selected from the group consisting of halogen, amino, nitro, cyano, oxo, hydroxy, thiol, carboxy, ester group, alkyl, alkoxy, alkenyl, alkynyl, cycloalkyl, heterocyclyl, aryl, heteroaryl, -C(O)R^{a}, -O(O)CR^{a}, -C(O)OR^{a}, -C(O)NR^{a}R^{b}, -NHC(O)R^{a}, -S(O)ₘR^{a}, -S(O)ₘNR^{a}R^{b} and -NHS(O)ₘR^{a};
R¹ and R² are each independently selected from the group consisting of hydrogen, halogen, amino, nitro, cyano, hydroxy, thiol, oxo, alkyl, alkoxy, cycloalkyl, heterocyclyl, aryl, heteroaryl, -C(O)R^{a}, -O(O)CR^{a}, -C(O)OR^{a}, -C(O)NR^{a}R^{b}, -NHC(O)R^{a}, -S(O)ₘR^{a}, -S(O)ₘNR^{a}R^{b} and -NHS(O)ₘR^{a};
R^{c} and R^{d} are each independently selected from the group consisting of hydrogen, halogen, amino, nitro, cyano, oxo, hydroxy, thiol, carboxy, ester group, alkyl, alkoxy, alkenyl, alkynyl, cycloalkyl, heterocyclyl, aryl, heteroaryl, -C(O)R^{a}, -O(O)CR^{a}, -C(O)OR^{a}, -C(O)NR^{a}R^{b}, -NHC(O)R^{a}, -S(O)ₘR^{a}, -S(O)ₘNR^{a}R^{b} and -NHS(O)ₘR^{a}, wherein the alkyl, alkoxy, alkenyl, alkynyl, cycloalkyl, heterocyclyl, aryl and heteroaryl are each optionally further substituted by one or more substituents selected from the group consisting of halogen, amino, nitro, cyano, hydroxy, thiol, oxo, alkyl, alkoxy, cycloalkyl, heterocyclyl, aryl and heteroaryl;
R⁵ and R⁶ are each independently selected from the group consisting of hydrogen, halogen, amino, cyano, hydroxy, thiol, alkyl, alkoxy, alkenyl, alkynyl, cycloalkyl, heterocyclyl, aryl and heteroaryl, wherein the alkyl, alkoxy, alkenyl, alkynyl, cycloalkyl, heterocyclyl, aryl and heteroaryl are each optionally further substituted by one or more substituents selected from the group consisting of halogen, amino, nitro, cyano, hydroxy, thiol, carboxy, ester group, oxo, alkyl, alkoxy, alkenyl, alkynyl, cycloalkyl, heterocyclyl, aryl and heteroaryl;
or, R⁵ and R⁶, together with the carbon atom to which they are attached, form a cycloalkyl or heterocyclyl, wherein the cycloalkyl or heterocyclyl is optionally further substituted by one or more substituents selected from the group consisting of halogen, amino, nitro, cyano, hydroxy, thiol, carboxy, ester group, oxo, alkyl, alkoxy, alkenyl, alkynyl, cycloalkyl, heterocyclyl, aryl and heteroaryl;
R^{a} and R^{b} are each independently selected from the group consisting of hydrogen, halogen, hydroxy, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are each optionally further substituted by one or more substituents selected from the group consisting of halogen, amino, nitro, cyano, hydroxy, thiol, carboxy, ester group, oxo, alkyl, alkoxy, alkenyl, alkynyl, cycloalkyl, heterocyclyl, aryl and heteroaryl;
or, R^{a} and R^{b}, together with the nitrogen atom to which they are attached, form a nitrogen-containing heterocyclyl, wherein the nitrogen-containing heterocyclyl is optionally further substituted by one or more substituents selected from the group consisting of halogen, amino, nitro, cyano, oxo, hydroxy, thiol, carboxy, ester group, alkyl, alkoxy, alkenyl, alkynyl, cycloalkyl, heterocyclyl, aryl and heteroaryl;
m is an integer from 0 to 2.

In a preferred embodiment, in the compound of formula (I) or a mesomer, racemate, enantiomer, diastereomer thereof, or mixture thereof, or a prodrug thereof, or a pharmaceutically acceptable salt thereof according to the present invention,
ring A is selected from the group consisting of aromatic ring and heteroaromatic ring, preferably 6-membered aromatic ring or 5- to 6-membered heteroaromatic ring, more preferably benzene ring or pyridine ring, wherein the aromatic ring and heteroaromatic ring are each optionally further substituted by one or more substituents selected from the group consisting of halogen, amino, nitro, cyano, oxo, hydroxy, thiol, carboxy, ester group, alkyl, alkoxy, alkenyl, alkynyl, cycloalkyl, heterocyclyl, aryl, heteroaryl, -C(O)R^{a}, -O(O)CR^{a}, -C(O)OR^{a}, -C(O)NR^{a}R^{b}, -NHC(O)R^{a}, -S(O)ₘR^{a}, -S(O)ₘNR^{a}R^{b} and -NHS(O)ₘR^{a}, preferably substituted by halogen(s);
R^{a} and R^{b} are each independently selected from the group consisting of hydrogen, halogen, hydroxy, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are each optionally further substituted by one or more substituents selected from the group consisting of halogen, amino, nitro, cyano, hydroxy, thiol, carboxy, ester group, oxo, alkyl, alkoxy, alkenyl, alkynyl, cycloalkyl, heterocyclyl, aryl and heteroaryl;
or, R^{a} and R^{b}, together with the nitrogen atom to which they are attached, form a nitrogen-containing heterocyclyl, wherein the nitrogen-containing heterocyclyl is optionally further substituted by one or more substituents selected from the group consisting of halogen, amino, nitro, cyano, oxo, hydroxy, thiol, carboxy, ester group, alkyl, alkoxy, alkenyl, alkynyl, cycloalkyl, heterocyclyl, aryl and heteroaryl;
m is an integer from 0 to 2.

In another preferred embodiment, in the compound of formula (I) or a mesomer, racemate, enantiomer, diastereomer thereof, or mixture thereof, or the prodrug thereof, or the pharmaceutically acceptable salt thereof according to the present invention, ring A is a benzene ring or pyridine ring, wherein the benzene ring or pyridine ring is optionally substituted by one or more substituents selected from the group consisting of halogen, C₁-C₆ alkyl and C₁-C₆ alkoxy, preferably optionally substituted by halogen(s).

In another preferred embodiment, in the compound of formula (I) or a mesomer, racemate, enantiomer, diastereomer thereof, or mixture thereof, or a prodrug thereof, or a pharmaceutically acceptable salt thereof according to the present invention,
A¹ is selected from CR¹; and
A² is selected from the group consisting of N and CR²;
R¹ and R² are each independently selected from the group consisting of hydrogen, halogen, amino, nitro, cyano, hydroxy, thiol, oxo, alkyl, alkoxy, cycloalkyl, heterocyclyl, aryl, heteroaryl, -C(O)R^{a}, -O(O)CR^{a}, -C(O)OR^{a}, -C(O)NR^{a}R^{b}, -NHC(O)R^{a}, -S(O)ₘR^{a}, -S(O)ₘNR^{a}R^{b} and -NHS(O)ₘR^{a};
R^{a}, R^{b} and m are as defined in formula (I).

In another preferred embodiment, in the compound of formula (I) or a mesomer, racemate, enantiomer, diastereomer thereof, or mixture thereof, or a prodrug thereof, or a pharmaceutically acceptable salt thereof according to the present invention, A¹ is selected from CH; and A² is selected from the group consisting of N and CH.

In a preferred embodiment, the compound of formula (I) or a mesomer, racemate, enantiomer, diastereomer thereof, or mixture thereof, or a prodrug thereof, or a pharmaceutically acceptable salt thereof according to the present invention is compound of formula (II) or a mesomer, racemate, enantiomer, diastereomer thereof, or mixture thereof, or a prodrug thereof, or a pharmaceutically acceptable salt thereof, 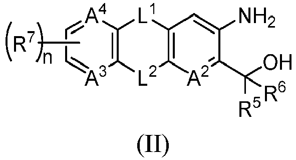 wherein,
A² is N or CH;
A³ is N or CH;
A⁴ is N or CH;
L¹ and L² are each independently selected from the group consisting of single bond, CR^{c}R^{d}, NR^{c}, O and S(O)ₘ, and L¹ and L² are not single bonds simultaneously;
each R⁷ is independently selected from the group consisting of hydrogen, halogen, amino, nitro, cyano, oxo, hydroxy, thiol, carboxy, ester group, alkyl, alkoxy, alkenyl, alkynyl, cycloalkyl, heterocyclyl, aryl, heteroaryl, -C(O)R^{a}, -O(O)CR^{a}, -C(O)OR^{a}, -C(O)NR^{a}R^{b}, -NHC(O)R^{a}, -S(O)ₘR^{a}, -S(O)ₘNR^{a}R^{b} and -NHS(O)ₘR^{a};
n is 0, 1, 2 or 3;
R⁵, R⁶, R^{a}, R^{b}, R^{c}, R^{d} and m are as defined in formula (I).

In a preferred embodiment, in the compound of formula (II) or a mesomer, racemate, enantiomer, diastereomer thereof, or mixture thereof, or a prodrug thereof, or a pharmaceutically acceptable salt thereof according to the present invention, A² is CH.

In another preferred embodiment, in the compound of formula (II) or a mesomer, racemate, enantiomer, diastereomer thereof, or mixture thereof, or a prodrug thereof, or a pharmaceutically acceptable salt thereof according to the present invention, A² is N.

In another preferred embodiment, in the compound of formula (II) or a mesomer, racemate, enantiomer, diastereomer thereof, or mixture thereof, or a prodrug thereof, or a pharmaceutically acceptable salt thereof according to the present invention, A³ is CH.

In another preferred embodiment, in the compound of formula (II) or a mesomer, racemate, enantiomer, diastereomer thereof, or mixture thereof, or a prodrug thereof, or a pharmaceutically acceptable salt thereof according to the present invention, A³ is N.

In another preferred embodiment, in the compound of formula (II) or a mesomer, racemate, enantiomer, diastereomer thereof, or mixture thereof, or a prodrug thereof, or a pharmaceutically acceptable salt thereof according to the present invention, A⁴ is CH.

In another preferred embodiment, in the compound of formula (II) or a mesomer, racemate, enantiomer, diastereomer thereof, or mixture thereof, or a prodrug thereof, or a pharmaceutically acceptable salt thereof according to the present invention, A⁴ is N.

In another preferred embodiment, in the compound of formula (II) or a mesomer, racemate, enantiomer, diastereomer thereof, or mixture thereof, or a prodrug thereof, or a pharmaceutically acceptable salt thereof according to the present invention, A² is N; A³ is CH; A⁴ is CH.

In another preferred embodiment, in the compound of formula (II) or a mesomer, racemate, enantiomer, diastereomer thereof, or mixture thereof, or a prodrug thereof, or a pharmaceutically acceptable salt thereof according to the present invention, A² is CH; A³ is CH; A⁴ is CH.

In another preferred embodiment, in the compound of formula (II) or a mesomer, racemate, enantiomer, diastereomer thereof, or mixture thereof, or a prodrug thereof, or a pharmaceutically acceptable salt thereof according to the present invention, A² is CH; A³ is CH; A⁴ is N.

In another preferred embodiment, in the compound of formula (II) or a mesomer, racemate, enantiomer, diastereomer thereof, or mixture thereof, or a prodrug thereof, or a pharmaceutically acceptable salt thereof according to the present invention, A² is CH; A³ is N; A⁴ is CH.

In another preferred embodiment, in the compound of formula (II) or a mesomer, racemate, enantiomer, diastereomer thereof, or mixture thereof, or a prodrug thereof, or a pharmaceutically acceptable salt thereof according to the present invention, A² is CH; A³ is N; A⁴ is N.

In another preferred embodiment, in the compound of formula (II) or a mesomer, racemate, enantiomer, diastereomer thereof, or mixture thereof, or a prodrug thereof, or a pharmaceutically acceptable salt thereof according to the present invention, R⁷ is independently selected from the group consisting of hydrogen and halogen; n is 0 or 1.

In a preferred embodiment, in the compound of formula (I) or formula (II) or a mesomer, racemate, enantiomer, diastereomer thereof, or mixture thereof, or a prodrug thereof, or a pharmaceutically acceptable salt thereof according to the present invention,
L¹ is selected from the group consisting of single bond, CR^{c}R^{d}, NR^{c} and O;
L² is selected from the group consisting of single bond, NR^{c} and O;
R^{c} and R^{d} are each independently selected from the group consisting of hydrogen and C₁-C₆ alkyl.

In another preferred embodiment, in the compound of formula (I) or formula (II) or a mesomer, racemate, enantiomer, diastereomer thereof, or mixture thereof, or a prodrug thereof, or a pharmaceutically acceptable salt thereof according to the present invention,
L¹ is selected from the group consisting of single bond, CR^{c}R^{d}, NR^{c} and O;
L² is selected from the group consisting of single bond, NR^{c} and O;
R^{c} and R^{d} are each independently selected from the group consisting of hydrogen, halogen, C₁-C₆ alkyl, C₁-C₆ alkoxy, -C(O)OR^{a} and benzyl; R^{a} is selected from C₁-C₆ alkyl.

In another preferred embodiment, in the compound of formula (I) or formula (II) or a mesomer, racemate, enantiomer, diastereomer thereof, or mixture thereof, or a prodrug thereof, or a pharmaceutically acceptable salt thereof according to the present invention,
L¹ is selected from the group consisting of single bond, NR^{c} and O;
L² is selected from the group consisting of NR^{c} and O;
R^{c} is selected from the group consisting of hydrogen, C₁-C₆ alkyl, -C(O)OR^{a} and benzyl; R^{a} is selected from C₁-C₆ alkyl.

In another preferred embodiment, in the compound of formula (I) or formula (II) or a mesomer, racemate, enantiomer, diastereomer thereof, or mixture thereof, or a prodrug thereof, or a pharmaceutically acceptable salt thereof according to the present invention,
L¹ is selected from the group consisting of single bond, NR^{c} and O;
L² is selected from O;
R^{c} is selected from the group consisting of hydrogen, C₁-C₆ alkyl, -C(O)OR^{a} and benzyl, preferably C₁-C₆ alkyl, -C(O)OR^{a} and benzyl; R^{a} is selected from C₁-C₆ alkyl.

In another preferred embodiment, in the compound of formula (I) or formula (II) or a mesomer, racemate, enantiomer, diastereomer thereof, or mixture thereof, or a prodrug thereof, or a pharmaceutically acceptable salt thereof according to the present invention,
L¹ is selected from the group consisting of single bond and O;
L² is selected from NR^{c};
R^{c} is selected from the group consisting of hydrogen, C₁-C₆ alkyl, -C(O)OR^{a} and benzyl, preferably C₁-C₆ alkyl, -C(O)OR^{a} and benzyl; R^{a} is selected from C₁-C₆ alkyl.

In another preferred embodiment, in the compound of formula (I) or formula (II) or a mesomer, racemate, enantiomer, diastereomer thereof, or mixture thereof, or a prodrug thereof, or a pharmaceutically acceptable salt thereof according to the present invention,
L¹ is selected from the group consisting of single bond and O;
L² is selected from NR^{c};
R^{c} is selected from the group consisting of hydrogen and C₁-C₆ alkyl, preferably C₁-C₆ alkyl.

In another preferred embodiment, in the compound of formula (I) or formula (II) or a mesomer, racemate, enantiomer, diastereomer thereof, or mixture thereof, or a prodrug thereof, or a pharmaceutically acceptable salt thereof according to the present invention,
L¹ is selected from the group consisting of NR^{c} and O;
L² is selected from single bond;
R^{c} is selected from the group consisting of hydrogen, C₁-C₆ alkyl, -C(O)OR^{a} and benzyl, preferably C₁-C₆ alkyl, -C(O)OR^{a} and benzyl; R^{a} is selected from C₁-C₆ alkyl.

In another preferred embodiment, in the compound of formula (I) or formula (II) or a mesomer, racemate, enantiomer, diastereomer thereof, or mixture thereof, or a prodrug thereof, or a pharmaceutically acceptable salt thereof according to the present invention,
L¹ is selected from the group consisting of NR^{c} and O;
L² is selected from single bond;
R^{c} is selected from the group consisting of hydrogen and C₁-C₆ alkyl, preferably C₁-C₆ alkyl.

In another preferred embodiment, in the compound of formula (I) or formula (II) or a mesomer, racemate, enantiomer, diastereomer thereof, or mixture thereof, or a prodrug thereof, or a pharmaceutically acceptable salt thereof according to the present invention,
L¹ is selected from CR^{c}R^{d};
L² is selected from the group consisting of single bond and O;
R^{c} and R^{d} are each independently selected from the group consisting of hydrogen, halogen, C₁-C₆ alkyl and C₁-C₆ alkoxy, preferably halogen, C₁-C₆ alkyl and C₁-C₆ alkoxy.

In another preferred embodiment, in the compound of formula (I) or formula (II) or a mesomer, racemate, enantiomer, diastereomer thereof, or mixture thereof, or a prodrug thereof, or a pharmaceutically acceptable salt thereof according to the present invention,
L¹ is selected from the group consisting of single bond and O;
L² is selected from CR^{c}R^{d};
R^{c} and R^{d} are each independently selected from the group consisting of hydrogen, halogen, C₁-C₆ alkyl and C₁-C₆ alkoxy, preferably halogen, C₁-C₆ alkyl and C₁-C₆ alkoxy.

In another preferred embodiment, in the compound of formula (I) or formula (II) or a mesomer, racemate, enantiomer, diastereomer thereof, or mixture thereof, or a prodrug thereof, or a pharmaceutically acceptable salt thereof according to the present invention,
L¹ is selected from NR^{c};
L² is selected from CR^{c}R^{d};
R^{c} is selected from the group consisting of hydrogen, halogen and C₁-C₆ alkyl, preferably halogen and C₁-C₆ alkyl;
R^{d} is selected from the group consisting of hydrogen, halogen, C₁-C₆ alkyl and C₁-C₆ alkoxy.

In another preferred embodiment, in the compound of formula (I) or formula (II) or a mesomer, racemate, enantiomer, diastereomer thereof, or mixture thereof, or a prodrug thereof, or a pharmaceutically acceptable salt thereof according to the present invention,
L¹ is selected from CR^{c}R^{d};
L² is selected from NR^{c};
R^{c} is selected from the group consisting of hydrogen, halogen and C₁-C₆ alkyl, preferably halogen and C₁-C₆ alkyl;
R^{d} is selected from the group consisting of hydrogen, halogen, C₁-C₆ alkyl and C₁-C₆ alkoxy.

In another preferred embodiment, in the compound of formula (I) or formula (II) or a mesomer, racemate, enantiomer, diastereomer thereof, or mixture thereof, or a prodrug thereof, or a pharmaceutically acceptable salt thereof according to the present invention,
R⁵ and R⁶ are each independently selected from the group consisting of hydrogen, halogen, amino, C₁-C₆ alkyl and C₃-C₆ cycloalkyl, wherein the C₁-C₆ alkyl is optionally further substituted by one or more substituents selected from halogen;
or, R⁵ and R⁶, together with the carbon atom to which they are attached, form a C₃-C₆ cycloalkyl or 5- to 7-membered heterocyclyl, wherein the cycloalkyl or heterocyclyl is optionally further substituted by one or more substituents selected from the group consisting of halogen, amino, nitro, cyano, hydroxy, thiol, carboxy, ester group, oxo, alkyl, alkoxy, alkenyl, alkynyl, cycloalkyl, heterocyclyl, aryl and heteroaryl.

In another preferred embodiment, in the compound of formula (I) or formula (II) or a mesomer, racemate, enantiomer, diastereomer thereof, or mixture thereof, or a prodrug thereof, or a pharmaceutically acceptable salt thereof according to the present invention, R⁵ and R⁶ are each independently selected from C₁-C₆ alkyl.

Typical compounds of the present invention include, but are not limited to:

| Example No. | Structure and name |
|---|---|
| 1 | 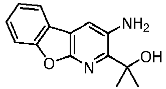 |
| | 2-(3-Aminobenzofuro[2,3-*b*]pyridin-2-yl)propan-2-ol |
| 2 | 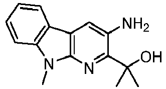 |
| | 2-(3-Amino-9-methyl-9*H*-pyrido[2,3-*b*]indol-2-yl)propan-2-ol |
| 3 | 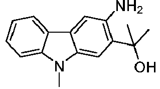 |
| | 2-(3-Amino-9-methyl-9*H*-carbazol-2-yl)propan-2-ol |
| 4 | 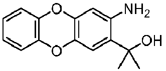 |
| | 2-(3-Aminodibenzo[*b*,*e*][1,4]dioxin-2-yl)propan-2-ol |
| 5 | 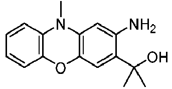 |
| | 2-(2-Amino-10-methyl-10*H*-phenoxazi-n-3-yl)propan-2-ol |
| 6 | 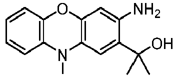 |
| | 2-(3-Amino-10-methyl-10*H*-phenoxazin-2-yl)propan-2-ol |
| 7 | 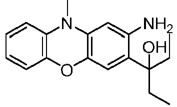 |
| | 3-(2-Amino-10-methyl-10*H*-phenoxazin-3-yl)pentan-3-ol |
| 8 | 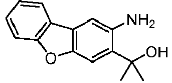 |
| | 2-(2-Aminodibenzo[*b*,*d*]furan-3-yl)propan-2-ol |
| 9 | 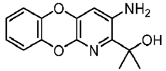 |
| | 2-(3-Aminobenzo[5,6][1,4]dioxino[2,3-*b*]pyridin-2-yl)propan-2-ol |
| 10 | 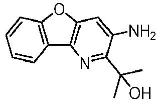 |
| | 2-(3-Aminobenzofuro[3,2-*b*]pyridin-2-yl)propan-2-ol |
| 11 | 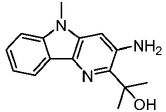 |
| | 2-(3-Amino-5-methyl-5*H*-pyrido[3,2-*b*]indol-2-yl)propan-2-ol |
| 12 | 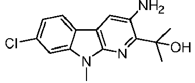 |
| | 2-(3-Amino-7-chloro-9-methyl-9*H*-pyrido[2,3-*b*]indol-2-yl)propan-2-ol |
| 13 | 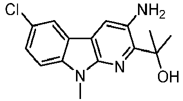 |
| | 2-(3-Amino-6-chloro-9-methyl-9*H*-pyrido[2,3-*b*]indol-2-yl)propan-2-ol |
| 14 | 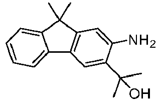 |
| | 2-(2-Amino-9,9-dimethyl-9*H*-fluoren-3-yl)propan-2-ol |
| 15 | 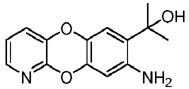 |
| | 2-(8-Aminobenzo[5,6][1,4]dioxino[2,3-*b*]pyridin-7-yl)propan-2-ol |
| 16 | 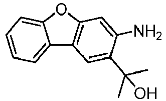 |
| | 2-(3-Aminodibenzo[*b*,*d*]furan-2-yl)propan-2-ol |
| 17 | 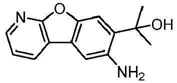 |
| | 2-(6-Aminobenzofuro[2,3-*b*]pyridin-7-yl)propan-2-ol |
| 18 | 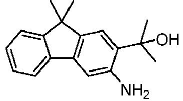 |
| | 2-(3-Amino-9,9-dimethyl-9*H*-fluoren-2-yl)propan-2-ol |
| 19 | 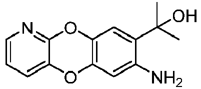 |
| | 2-(7-Aminobenzo[5,6][1,4]dioxino[2,3-*b*]pyridin-8-yl)propan-2-ol |
| 20 | 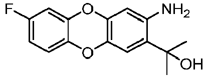 |
| | 2-(3-Amino-7-fluorodibenzo[*b,e*][1,4]dioxin-2-yl)propanol |
| 21 | 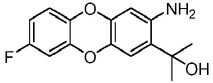 |
| | 2-(3-Amino-8-fluorodibenzo[*b,e*][1,4]dioxin-2-yl)propanol |
| 22 | 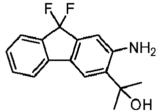 |
| | 2-(2-Amino-9,9-difluoro-9*H*-fluoren-3-yl)propanol |
| 23 | 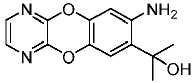 |
| | 2-(8-Aminobenzo[5,6][1,4]dioxino[2,3*-b*]pyrazin-7-yl)propan-2-ol |
| 24 | 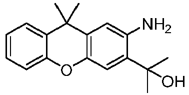 |
| | 2-(2-Amino-9,9-dimethyl-9*H*-xanthen-3-yl)propan-2-ol |
| 25 | 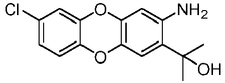 |
| | 2-(3-Amino-7-chlorodibenzo[*b,e*][1,4]dioxin-2-yl)propanol |
| 26 | 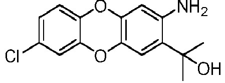 |
| | 2-(3-Amino-8-chlorodibenzo[*b,e*][1,4]dioxin-2-yl)propanol |
| 27 | 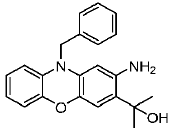 |
| | 2-(2-Amino-10-benzyl-10*H*-phenoxazin-3-yl)propan-2-ol |
| 28 | 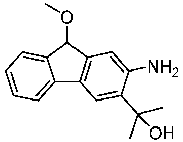 |
| | 2-(2-Amino-9-methoxy-9*H*-fluoren-3-yl)propan-2-ol |
| 29 | 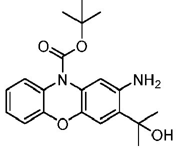 |
| | *tert*-Butyl 2-amino-3-(2-hydroxypropan-2-yl)-10*H*-phenoxazine-10-carboxylate |

or a mesomer, racemate, enantiomer, diastereomer thereof, or mixture thereof, or a prodrug thereof, or a pharmaceutically acceptable salt thereof.

The present invention further provides a method for preparing the compound of formula (I) or a mesomer, racemate, enantiomer, diastereomer thereof, or mixture thereof, or a prodrug thereof, or a pharmaceutically acceptable salt thereof according to the present invention, comprising the following step of: 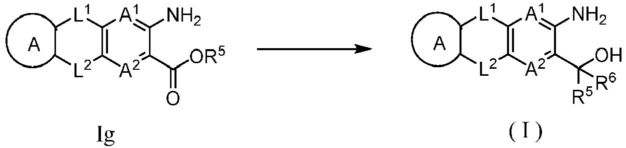
reacting compound Ig with an alkyl Grignard reagent to obtain the compound of formula (I), wherein the alkyl Grignard reagent is preferably methylmagnesium chloride or methylmagnesium bromide; the reaction is preferably carried out in a solvent, the solvent is preferably anhydrous tetrahydrofuran;
wherein A¹, A², ring A, L¹, L², R⁵ and R⁶ are as defined in formula (I).

The present invention further provides a method for preparing the compound of formula (II) or a mesomer, racemate, enantiomer, diastereomer thereof, or mixture thereof, or a prodrug thereof, or a pharmaceutically acceptable salt thereof according to the present invention, comprising the following step of: 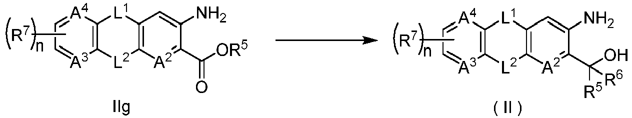
reacting compound IIg with an alkyl Grignard reagent to obtain the compound of formula (II), wherein the alkyl Grignard reagent is preferably methylmagnesium chloride or methylmagnesium bromide; the reaction is preferably carried out in a solvent, the solvent is preferably anhydrous tetrahydrofuran;
wherein A², A³, A⁴, L¹, L², R⁵, R⁶, R⁷ and n are as defined in formula (II).

The present invention further provides a pharmaceutical composition comprising the compound of formula (I) or a mesomer, racemate, enantiomer, diastereomer thereof, or mixture thereof, or a prodrug thereof, or a pharmaceutically acceptable salt thereof according to the present invention, and a pharmaceutically acceptable carrier.

The present invention further provides a use of the compound of formula (I) or a mesomer, racemate, enantiomer, diastereomer thereof, or mixture thereof, or a prodrug thereof, or a pharmaceutically acceptable salt thereof, or the pharmaceutical composition comprising the same according to the present invention in the preparation of a toxic aldehyde capture agent.

The present invention further provides a use of the compound of formula (I) or a mesomer, racemate, enantiomer, diastereomer thereof, or mixture thereof, or a prodrug thereof, or a pharmaceutically acceptable salt thereof, or a pharmaceutical composition comprising the same according to the present invention in the preparation of medicaments for the prevention and/or treatment of diseases related to active carbonyl compound; the disease is preferably ocular disease, skin disease, autoimmune disease, digestive system disease, cardiovascular disease, respiratory system disease, neurodegenerative disease, obesity, cancer, and aging-related disease; and the ocular disease is preferably noninfectious uveitis, allergic conjunctivitis and dry eye.

In another aspect, the present invention provides the compound of formula (I) or a mesomer, racemate, enantiomer, diastereomer thereof, or mixture thereof, or a prodrug thereof, or a pharmaceutically acceptable salt thereof, or the pharmaceutical composition comprising the same according to the present invention, for use as a toxic aldehyde capture agent.

In another aspect, the present invention provides the compound of formula (I) or a mesomer, racemate, enantiomer, diastereomer thereof, or mixture thereof, or a prodrug thereof, or a pharmaceutically acceptable salt thereof, or the pharmaceutical composition comprising the same according to the present invention, for use as a medicament; preferably, the medicament is used for preventing and/or treating a disease related to active carbonyl compound; the disease is preferably ocular disease, skin disease, autoimmune disease, digestive system disease, cardiovascular disease, respiratory system disease, neurodegenerative disease, obesity, cancer, and aging-related disease; specifically, the medicament is used for preventing and/or treating an ocular disease such as noninfectious uveitis, allergic conjunctivitis and dry eye.

In another aspect, the present invention provides the compound of formula (I) or a mesomer, racemate, enantiomer, diastereomer thereof, or mixture thereof, or a prodrug thereof, or a pharmaceutically acceptable salt thereof, or the pharmaceutical composition comprising the same according to the present invention, for use in the prevention and/or treatment of diseases, the disease is selected from the group consisting of ocular disease, skin disease, autoimmune disease, digestive system disease, cardiovascular disease, respiratory system disease, neurodegenerative disease, obesity, cancer, and aging-related disease; and the ocular disease is preferably noninfectious uveitis, allergic conjunctivitis and dry eye.

A method for preventing and/or treating a disease related to active carbonyl compound comprising administration of a preventively or therapeutically effective dose of the compound of formula (I) or a mesomer, racemate, enantiomer, diastereomer thereof, or mixture thereof, or a prodrug thereof, or a pharmaceutically acceptable salt thereof, or the pharmaceutical composition comprising the same according to the present invention to a patient in need thereof; the disease is selected from the group consisting of ocular disease, skin disease, autoimmune disease, digestive system disease, cardiovascular disease, respiratory system disease, neurodegenerative disease, obesity, cancer, and aging-related disease; and the ocular disease is preferably noninfectious uveitis, allergic conjunctivitis and dry eye.

The prodrug of the present invention is a derivatives of the compound of formula (I), which may have weak or even no activity *per se,* but can be converted to the corresponding biologically active forms under physiological conditions (for example by metabolism, solvolysis or other ways) upon administration.

The pharmaceutical composition containing the active ingredient can be in a form suitable for oral administration, for example, a tablet, troche, lozenge, aqueous or oily suspension, dispersible powder or granule, emulsion, hard or soft capsule, syrup or elixir. An oral composition can be prepared according to any known method in the art for the preparation of pharmaceutical composition. Such composition can contain one or more ingredients selected from the group consisting of sweeteners, flavoring agents, colorants and preservatives, in order to provide a pleasing and palatable pharmaceutical formulation. The tablet contains the active ingredient in admixture with nontoxic, pharmaceutically acceptable excipients suitable for the preparation of tablets. These excipients may be inert excipients, such as calcium carbonate, sodium carbonate, lactose, calcium phosphate or sodium phosphate; granulating and disintegrating agents, such as microcrystalline cellulose, cross-linked sodium carboxylmethyl cellulose, corn starch or alginic acid; binders, such as starch, gelatin, polyvinylpyrrolidone or acacia; and lubricants, such as magnesium stearate, stearic acid or talc. The tablet may be uncoated or coated by means of known techniques, which can mask drug taste or delay the disintegration and absorption of the active ingredient in the gastrointestinal tract, thereby providing sustained release over an extended period. For example, a water-soluble taste masking material can be used, such as hydroxypropyl methylcellulose or hydroxypropyl cellulose, or an extended-release material can be used, such as ethyl cellulose, cellulose acetate butyrate.

An oral formulation can also be provided as hard gelatin capsules in which the active ingredient is mixed with an inert solid diluent, such as calcium carbonate, calcium phosphate or kaolin, or as soft gelatin capsules in which the active ingredient is mixed with a water-soluble carrier such as polyethylene glycol or an oil medium such as peanut oil, liquid paraffin or olive oil.

An aqueous suspension contains the active ingredient in admixture with an excipient suitable for the preparation of aqueous suspension. Such excipient is a suspending agent, such as sodium carboxylmethylcellulose, methylcellulose, hydroxypropylmethylcellulose, sodium alginate, polyvinylpyrrolidone and acacia; a dispersant or humectant, which can be a naturally occurring phosphatide such as lecithin, or a condensation product of an alkylene oxide with fatty acid such as polyoxyethylene stearate, or a condensation product of ethylene oxide with a long chain aliphatic alcohol such as heptadecaethyleneoxy cetanol, or a condensation product of ethylene oxide with part esters derived from fatty acids and hexitols such as polyoxyethylene sorbitol monooleate, or a condensation product of ethylene oxide with partial esters derived from fatty acids and hexitol anhydrides such as polyoxyethylene sorbitan monooleate. The aqueous suspension can also contain one or more preservatives, such as ethylparaben or *n*-propylparaben, one or more colorants, one or more flavoring agents, and one or more sweeteners such as sucrose, saccharin or aspartame.

An oil suspension can be formulated by suspending the active ingredient in a vegetable oil such as peanut oil, olive oil, sesame oil or coconut oil, or in a mineral oil such as liquid paraffin. The oil suspension can contain a thickener, such as beeswax, hard paraffin or cetyl alcohol. The above sweetener and flavoring agent can be added to provide a palatable formulation. These compositions can be preserved by adding an antioxidant, such as butylated hydroxyanisole or *α*-tocopherol.

A dispersible powder or granule suitable for the preparation of an aqueous suspension can provide active ingredient by adding water and dispersants or wetting agents, suspending agent or one or more preservatives. Suitable dispersants, wetting agents and suspending agents are as described above. Additional excipients, such as sweetening agents, flavoring agents and coloring agents, can also be added. These compositions are preserved by adding an antioxidant such as ascorbic acid.

The pharmaceutical composition of the present invention can also be in the form of an oil-in-water emulsion. The oil phase can be a vegetable oil such as olive oil or peanut oil, or a mineral oil such as liquid paraffin or a mixture thereof. Suitable emulsifying agent can be naturally occurring phosphatides, such as soy bean lecithin, and esters or partial esters derived from fatty acids and hexitol anhydrides such as sorbitan monooleate, and condensation products of said partial esters with ethylene oxide such as polyoxyethylene sorbitol monooleate. The emulsion can also contain a sweetener, flavoring agent, preservative and antioxidant. Syrup and elixir can be formulated with a sweetener, such as glycerol, propylene glycol, sorbitol or sucrose. Such formulations can also contain a moderator, a preservative, a colorant and an antioxidant.

The pharmaceutical composition of the present invention can be in the form of a sterile injectable aqueous solution. The acceptable medium or solvent that can be used include water, Ringer's solution and isotonic sodium chloride solution. The sterile injectable formulation can be a sterile injectable oil-in-water microemulsion in which the active ingredient is dissolved in the oil phase. For example, the active ingredient can be firstly dissolved in a mixture of soybean oil and lecithin, the oil solution is then introduced into a mixture of water and glycerol and processed to form a microemulsion. The injectable solution or microemulsion can be introduced into a patient's bloodstream by local bolus injection. Alternatively, it may be advantageous to administer the solution or microemulsion in such a way as to maintain a constant circulation concentration of the compound of the present invention. In order to maintain such a constant concentration, a continuous intravenous delivery device can be utilized.

The pharmaceutical composition of the present invention can be in the form of a sterile injectable aqueous or oily suspension for intramuscular and subcutaneous administration. Such a suspension can be formulated with suitable dispersants or wetting agents and suspending agents as described above according to known techniques. The sterile injectable formulation can also be a sterile injectable solution or suspension prepared in a nontoxic parenterally acceptable diluent or solvent, such as a solution prepared in 1,3-butanediol. Moreover, sterile fixed oils can easily be used as a solvent or suspending medium. For this purpose, any blending fixed oils including synthetic mono- or di-glyceride can be employed. Moreover, fatty acids such as oleic acid can also be employed in the preparation of an injection.

The pharmaceutical composition of the present invention can be in the form for topical administration, for example, a cream, suspension, emulsion, ointment, gel, drop, oil, lotion, film, patch, tape, inhalant, spray. The form for intraocular administration can be a subconjunctival capsule, subfascial capsule, retrobulbar or intravitreal injection, depot injection or implant. The compound administered by these routes can be in the form of solution or suspension. The compound administered by depot injection can contain a pharmaceutically acceptable carrier or excipient. These pharmaceutically acceptable carriers or excipients can be natural or synthetic, biodegradable or non-biodegradable, and facilitate drug release in a controlled manner. The implant for controlled release of compound can be constructed of natural or synthetic, biodegradable or non-biodegradable materials. The carrier is acceptable, since it is compatible with the other components of the composition and is not detrimental to the patient. Some examples of carrier include sugar, such as lactose, dextrose and sucrose; starch, such as corn starch and potato starch; cellulose; and cyclodextrins.

It is well known to those skilled in the art that the dosage of a drug depends on a variety of factors including, but not limited to the following factors: activity of a specific compound, age of the patient, body weight of the patient, general health of the patient, behavior of the patient, diet of the patient, administration time, administration route, excretion rate, drug combination and the like. In addition, the optimal treatment, such as treatment mode, daily dose of the compound of the present invention or the type of pharmaceutically acceptable salt thereof can be verified according to the traditional therapeutic regimens.

The present invention may contain a composition comprising the compound of formula (I) or the pharmaceutically acceptable salt, hydrate or solvate as an active ingredient, and a pharmaceutically acceptable carrier or excipient, which is formulated into a clinically acceptable formulation. The derivatives of the present invention can be used in combination with other active ingredients as long as they do not cause other adverse effects such as allergic reactions and the like. The compound of the present invention can be used as the sole active ingredient, and can also be used in combination with other therapeutic agents. A combination therapy is achieved by administering the individual therapeutic components simultaneously, separately or sequentially.

### Definitions

Unless otherwise stated, the terms used in the specification and claims have the meanings described below.

The carbon, hydrogen, oxygen, sulfur, nitrogen and halogen involved in the groups and compounds of the present invention all include their isotopes. That is to say, the carbon, hydrogen, oxygen, sulfur, nitrogen and halogen involved in the groups and compounds of the present invention are optionally further substituted by one or more of their corresponding isotopes. The isotopes of carbon include ¹²C, ¹³C and ¹⁴C; the isotope of hydrogen include protium (H), deuterium (D, also known as heavy hydrogen), tritium (T, also known as superheavy hydrogen); the isotope of oxygen include ¹⁶O, ¹⁷O and ¹⁸O; the isotope of sulfur include ³²S, ³³S, ³⁴S and ³⁶S; the isotopes of nitrogen include ¹⁴N and ¹⁵N; the isotope of fluorine include ¹⁹F; the isotope of chlorine include ³⁵Cl and ³⁷Cl; and the isotope of bromine include ⁷⁹Br and ⁸¹Br.

The term "alkyl" refers to a saturated aliphatic hydrocarbon group, which is a straight or branched chain group comprising 1 to 20 carbon atoms, preferably an alkyl having 1 to 12 carbon atoms, and more preferably an alkyl having 1 to 6 carbon atoms. Non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof. More preferably, the alkyl group is a lower alkyl having 1 to 6 carbon atoms, and non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl group can be substituted or unsubstituted. When substituted, the substituent group(s) can be substituted at any available connection point. The substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxyl and ester group.

The term "alkylene" refers to a linear or branched divalent saturated hydrocarbon group, including -(CH₂)ᵥ- (v is an integer from 1 to 10, and preferably an integer from 1 to 6). Examples of alkylene include, but are not limited to, methylene, ethylene, propylene, butylene and the like. The alkylene group can be substituted or unsubstituted. When substituted, the substituent group(s) can be substituted at any available connection point. The substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxyl and ester group. When the number of substituents in an alkylene is 2 or more, the substituents can be fused together to form a cyclic structure.

The term "alkenyl" refers to an alkyl group as defined above consisting of at least two carbon atoms and at least one carbon-carbon double bond, such as ethenyl, 1-propenyl, 2-propenyl, 1-, 2- or 3-butenyl and the like. The alkenyl group can be substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio and heterocyclylthio.

The term "alkenylene" refers to a straight or branched chain divalent alkenyl group, wherein the alkenyl is as defined above.

The term "alkynyl" refers to an alkyl group as defined above consisting of at least two carbon atoms and at least one carbon-carbon triple bond, such as ethynyl, propynyl, butynyl and the like. The alkynyl group can be substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio and heterocyclylthio.

The term "alkynylene" refers to a straight or branched chain divalent alkynyl group, wherein the alkynyl is as defined above.

The term "cycloalkyl" refers to a saturated or partially unsaturated monocyclic or polycyclic hydrocarbon substituent group having 3 to 20 carbon atoms, preferably 3 to 10 carbon atoms, and more preferably 3 to 7 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes a cycloalkyl having a spiro ring, fused ring or bridged ring.

The term "spiro cycloalkyl" refers to a 5 to 20 membered polycyclic group with individual rings connected through one commom carbon atom (called a spiro atom), wherein one or more rings can contain one or more double bonds, but none of the rings has a completely conjugated π-electron system. The spiro cycloalkyl is preferably a 5 to 12 membered spiro cycloalkyl, and more preferably a 7 to 10 membered spiro cycloalkyl. According to the number of the spiro atoms shared between the rings, the spiro cycloalkyl can be divided into a mono-spiro cycloalkyl, a di-spiro cycloalkyl, or a poly-spiro cycloalkyl, and the spiro cycloalkyl is preferably a mono-spiro cycloalkyl or di-spiro cycloalkyl, and more preferably a 4-membered/4-membered, 4-membered/5-membered, 4-membered/6-membered, 5-membered/5-membered, or 5-membered/6-membered mono-spiro cycloalkyl. Non-limiting examples of spiro cycloalkyl include:

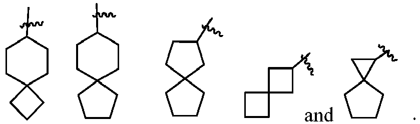

The term "fused cycloalkyl" refers to a 5 to 20 membered all-carbon polycyclic group, wherein each ring in the system shares an adjacent pair of carbon atoms with another ring, one or more rings can contain one or more double bonds, but none of the rings has a completely conjugated π-electron system. The fused cycloalkyl is preferably a 6 to 14 membered fused cycloalkyl, and more preferably a 7 to 10 membered fused cycloalkyl. According to the number of membered rings, the fused cycloalkyl can be divided into a bicyclic, tricyclic, tetracyclic or polycyclic fused cycloalkyl, and the fused cycloalkyl is preferably a bicyclic or tricyclic fused cycloalkyl, and more preferably a 5-membered/5-membered, or 5-membered/6-membered bicyclic fused cycloalkyl. Non-limiting examples of fused cycloalkyl include:

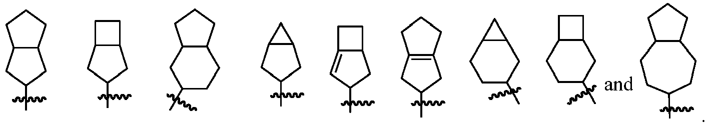

The term "bridged cycloalkyl" refers to a 5 to 20 membered all-carbon polycyclic group, wherein any two rings in the system share two disconnected carbon atoms, one or more rings can have one or more double bonds, but none of the rings has a completely conjugated π-electron system. The bridged cycloalkyl is preferably a 6 to 12 membered bridged cycloalkyl, and more preferably a 7 to 10 membered bridged cycloalkyl. According to the number of membered rings, the bridged cycloalkyl can be divided into a bicyclic, tricyclic, tetracyclic or polycyclic bridged cycloalkyl, and the bridged cycloalkyl is preferably a bicyclic, tricyclic or tetracyclic bridged cycloalkyl, and more preferably a bicyclic or tricyclic bridged cycloalkyl. Non-limiting examples of bridged cycloalkyl include:

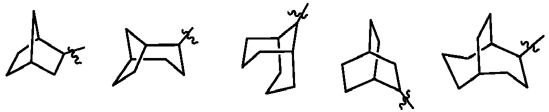

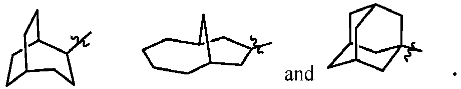

The cycloalkyl ring can be fused to the ring of aryl, heteroaryl or heterocyclyl, wherein the ring linking to the parent structure is cycloalkyl. Non-limiting examples include indanyl, tetrahydronaphthyl, benzocycloheptyl and the like. The cycloalkyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxyl and ester group.

The term "heterocyclyl" or "heterocycle" refers to a 3 to 20 membered saturated or partially unsaturated monocyclic or polycyclic hydrocarbon group, wherein one or more ring atoms are heteroatoms selected from the group consisting of N, O and S(O)ₘ (wherein m is an integer of 0 to 2), but excluding -O-O-, -O-S- or -S-S- in the ring, with the remaining ring atoms being carbon atoms. Preferably, the heterocyclyl has 3 to 10 ring atoms wherein 1 to 4 atoms are heteroatoms; more preferably, 3 to 8 ring atoms wherein 1 to 3 atoms are heteroatoms; and most preferably 5 to 7 ring atoms wherein 1 to 2 or 1 to 3 atoms are heteroatoms. Non-limiting examples of monocyclic heterocyclyl include pyrrolidinyl, imidazolyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl and the like, and preferably, 1,2,5-oxadiazolyl, pyranyl or morpholinyl. Polycyclic heterocyclyl includes a heterocyclyl having a spiro ring, fused ring or bridged ring.

The term "spiro heterocyclyl" refers to a 5 to 20 membered polycyclic heterocyclyl group with individual rings connected through one common atom (called a spiro atom), wherein one or more ring atoms are heteroatoms selected from the group consisting of N, O and S(O)ₘ (wherein m is an integer of 0 to 2), with the remaining ring atoms being carbon atoms, and the rings can contain one or more double bonds, but none of the rings has a completely conjugated π-electron system. The spiro heterocyclyl is preferably a 5 to 12 membered spiro heterocyclyl, and more preferably a 7 to 10 membered spiro heterocyclyl. According to the number of the spiro atoms shared between the rings, the spiro heterocyclyl can be divided into a mono-spiro heterocyclyl, di-spiro heterocyclyl, or poly-spiro heterocyclyl, and the spiro heterocyclyl is preferably a mono-spiro heterocyclyl or di-spiro heterocyclyl, and more preferably a 4-membered/4-membered, 4-membered/5-membered, 4-membered/6-membered, 5-membered/5-membered, or 5-membered/6-membered mono-spiro heterocyclyl. Non-limiting examples of spiro heterocyclyl include:

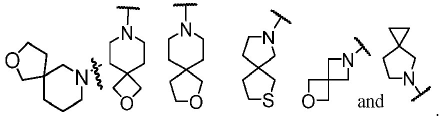

The term "fused heterocyclyl" refers to a 5 to 20 membered polycyclic heterocyclyl group, wherein each ring in the system shares an adjacent pair of atoms with another ring, one or more rings can contain one or more double bonds, but none of the rings has a completely conjugated π-electron system, and one or more ring atoms are heteroatoms selected from the group consisting of N, O and S(O)ₘ (wherein m is an integer of 0 to 2), with the remaining ring atoms being carbon atoms. The fused heterocyclyl is preferably a 6 to 14 membered fused heterocyclyl, and more preferably a 7 to 10 membered fused heterocyclyl. According to the number of membered rings, the fused heterocyclyl can be divided into a bicyclic, tricyclic, tetracyclic or polycyclic fused heterocyclyl, and preferably a bicyclic or tricyclic fused heterocyclyl, and more preferably a 5-membered/5-membered or 5-membered/6-membered bicyclic fused heterocyclyl. Non-limiting examples of fused heterocyclyl include:

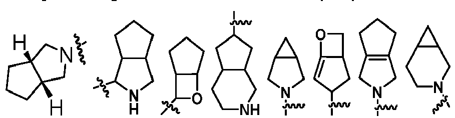

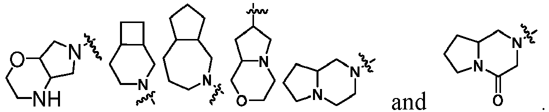

The term "bridged heterocyclyl" refers to a 5 to 14 membered polycyclic heterocyclyl group, wherein any two rings in the system share two disconnected atoms, wherein one or more rings can have one or more double bond(s), but none of the rings has a completely conjugated π-electron system, and one or more ring atoms are heteroatoms selected from the group consisting of N, O and S(O)ₘ (wherein m is an integer of 0 to 2), with the remaining ring atoms being carbon atoms. The bridged heterocyclyl is preferably a 5 to 12 membered bridged heterocyclyl, and more preferably a 7 to 10 membered bridged heterocyclyl. According to the number of membered rings, the bridged heterocyclyl can be divided into a bicyclic, tricyclic, tetracyclic or polycyclic bridged heterocyclyl, and the bridged heterocyclyl is preferably a bicyclic, tricyclic or tetracyclic bridged heterocyclyl, and more preferably a bicyclic or tricyclic bridged heterocyclyl. Non-limiting examples of bridged heterocyclyl include:

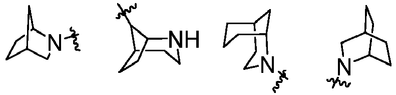

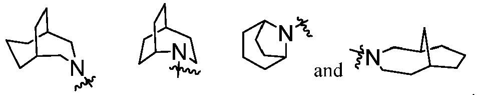

The heterocyclyl ring can be fused to the ring of aryl, heteroaryl or cycloalkyl, wherein the ring bound to the parent structure is heterocyclyl. Non-limiting examples thereof include: 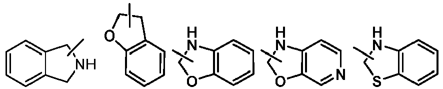 and the like.

The heterocyclyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxyl and ester group.

The term "aryl" or "aromatic ring" refers to a 6 to 14 membered all-carbon monocyclic ring or polycyclic fused ring (*i.e.* each ring in the system shares an adjacent pair of carbon atoms with another ring in the system) having a conjugated π-electron system, preferably a 6 to 10 membered aryl, for example, phenyl and naphthyl. The aryl is more preferably phenyl. The aryl ring can be fused to the ring of heteroaryl, heterocyclyl or cycloalkyl, wherein the ring bound to the parent structure is aryl ring. Non-limiting examples thereof include:

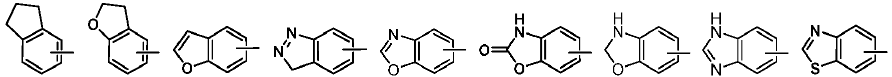

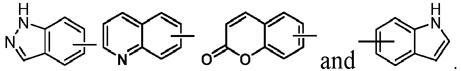

The aryl can be substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxyl and ester group.

The term "heteroaryl" or "heteroaromatic ring" refers to a 5 to 14 membered heteroaromatic system having 1 to 4 heteroatoms selected from the group consisting of O, S and N. The heteroaryl is preferably a 5 to 10 membered heteroaryl having 1 to 3 heteroatom(s), and more preferably a 5 or 6 membered heteroaryl having 1 to 2 heteroatom(s), for example imidazolyl, furyl, thienyl, thiazolyl, pyrazolyl, oxazolyl, pyrrolyl, tetrazolyl, pyridyl, pyrimidinyl, thiadiazolyl, pyrazinyl and the like, preferably imidazolyl, thiazolyl, pyrazolyl, pyrimidinyl or thiazolyl, and more preferably pyrazolyl or thiazolyl. The heteroaryl ring can be fused to the ring of aryl, heterocyclyl or cycloalkyl, wherein the ring bound to the parent structure is heteroaryl ring. Non-limiting examples thereof include:

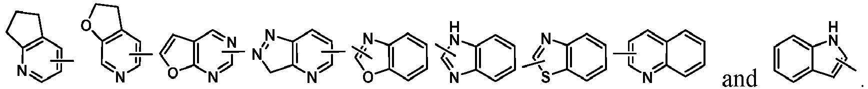

The heteroaryl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxyl and ester group.

The term "alkoxy" refers to an -O-(alkyl) or an -O-(cycloalkyl) group, wherein the alkyl and cycloalkyl are as defined above. Non-limiting examples of alkoxy include methoxy, ethoxy, propoxy, butoxy, cyclopropyloxy, cyclobutyloxy, cyclopentyloxy, cyclohexyloxy. The alkoxy can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxyl and ester group.

The term "haloalkyl" refers to an alkyl group substituted by one or more halogen(s), wherein the alkyl is as defined above.

The term "haloalkoxy" refers to an alkoxy group substituted by one or more halogen(s), wherein the alkoxy is as defined above.

The term "hydroxy" refers to an -OH group.

The term "halogen" refers to fluorine, chlorine, bromine or iodine.

The term "amino" refers to a -NH₂ group.

The term "cyano" refers to a -CN group.

The term "nitro" refers to a -NO₂ group.

The term "oxo" refers to a =O group.

The term "carboxy" refers to a -C(O)OH group.

The term "thiol" refers to a -SH group.

The term "ester group" refers to a -C(O)O(alkyl) or a -C(O)O(cycloalkyl) group, wherein the alkyl and cycloalkyl are as defined above.

The term "acyl" refers to a compound comprising a -C(O)R group, where R is an alkyl, cycloalkyl, heterocyclyl, aryl or heteroaryl.

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl group can be, but need not be, present, and such a description includes the situation of the heterocyclyl being substituted by an alkyl and the situation of the heterocyclyl being not substituted by an alkyl.

"Substituted" refers to one or more hydrogen atoms in a group, preferably up to 5, and more preferably 1 to 3 hydrogen atoms in a group, are independently substituted by a corresponding number of substituents. It goes without saying that the substituents only exist in their possible chemical position. The person skilled in the art is able to determine whether the substitution is possible or impossible by experiments or theory without excessive efforts. For example, the combination of amino or hydroxy having free hydrogen and carbon atoms having unsaturated bonds (such as olefinic) may be unstable.

A "pharmaceutical composition" refers to a mixture of one or more of the compounds according to the present invention or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to exert biological activity.

A "pharmaceutically acceptable salt" refers to a salt of the compound of the present invention, which is safe and effective in mammals and has the desired biological activity.

A "carrier" refers to a carrier or diluent that is not appreciably irritating to the organism and that does not reduce the biological activity and properties of the compound.

A "prodrug" refers to a compound that can be converted under physiological conditions or by solvolysis to a compound of the present invention having biological activity. The prodrug of the present invention is prepared by modifying functional groups of the compound of the present invention, and such modification can be removed by conventional process or *in vivo* to give the parent compound.

### DESCRIPTION OF THE DRAWINGS

Figure 1 is a time-varying diagram of the capture reaction of the compounds of the examples of the present invention to nonenal.
Figure 2 is a graph showing the results of the treatment score of the compound of Example 4 of the present invention in the C48/80-induced Wistar rat allergic conjunctivitis animal model.

### DETAILED DESCRIPTION OF THE INVENTION

The compounds of the present invention and their preparation are further illustrated with reference to the following examples. These examples illustrate some methods for preparing or using said compounds. However, it should be understood that these examples are not intended to limit the scope of the present invention. Variations of the present invention now known or to be further developed are considered to fall within the scope of the present invention described and claimed herein.

The compound of the present invention is prepared by using convenient starting materials and general preparation procedures. The present invention provides typical or preferential reaction conditions, such as reaction temperature, duration, solvent, pressure and molar ratio of reactants. However, unless otherwise specified, other reaction conditions can also be adopted. Optimal conditions may vary with the use of specific reactants or solvents, but under normal circumstances, reaction optimization steps and conditions can be determined.

In addition, some protecting groups may be used in the present invention to protect certain functional groups from unnecessary reactions. The protecting groups suitable for various functional groups and their protection or deprotection conditions are well known to those skilled in the art. For example, "Protective Groups in Organic Synthesis" by T. W. Greene and G. M. Wuts (3rd edition, Wiley, New York, 1999 and citations in the book) describes in detail the protection or deprotection of a large number of protective groups.

The separation and purification of compounds and intermediates are carried out by appropriate methods and steps according to specific needs, such as filtration, extraction, distillation, crystallization, column chromatography, preparative thin-layer chromatography, preparative high performance liquid chromatography or a combination of the above methods. The examples described in the present invention can be referred for the specific method of use. Of course, other similar separation and purification methods can also be used. They can be characterized using conventional methods (including physical constants and spectral data).

The structures of the compounds are identified by nuclear magnetic resonance (NMR) and/or mass spectrometry (MS). NMR shifts (δ) are given in 10⁻⁶ (ppm). NMR is determined by a Brukerdps300 machine. The solvents for determination are deuterated-dimethyl sulfoxide (DMSO-*d*6), deuterated-chloroform (CDCl₃) and deuterated-methanol (CD₃OD), and the internal standard is tetramethylsilane (TMS).

MS is determined by a LC(Waters 2695)/MS(Quattro Premier xE) mass spectrograph (manufacturer: Waters) (Photodiode Array Detector).

Preparative liquid chromatography is conducted on a lc6000 high performance liquid chromatograph (manufacturer: Beijing Chuangxintongheng science and Technology Co., Ltd.).

Qingdao Haiyang Chemical GF254 silica gel plate is used for the thin-layer silica gel chromatography (TLC). The dimension of the silica gel plate used in TLC is 0.20 mm to 0.25 mm, and the dimension of the silica gel plate used in product purification (Prep-TLC) is 0.5 mm.

Qingdao Haiyang Chemical 100 to 200 mesh, 200 to 300 mesh and 300 to 400 mesh silica gel is generally used as a carrier for column chromatography.

The known starting materials of the present invention can be prepared by the known methods in the art, or can be purchased from Wanghua Mall, Beijing Ouhe Technology, Sigma, J&K Scientific, Yishiming, Shanghai Shuya Chemical, Innochem Science & Technology, Energy Chemical, Shanghai Bide Pharmatech and the like.

Unless otherwise stated, the reactions are carried out under a nitrogen atmosphere.

Argon atmosphere or nitrogen atmosphere means that a reaction flask is equipped with an argon or nitrogen balloon (about 1 L).

The reaction solvent, organic solvent or inert solvent are each expressed as the solvent used that does not participate in the reaction under the described reaction conditions, which includes for example, benzene, toluene, acetonitrile, tetrahydrofuran (THF), dimethylformamide (DMF), chloroform, dichloromethane, ether, methanol, N-methylpyrrolidone (NMP), pyridine and the like. Unless otherwise specified in the examples, a solution means an aqueous solution.

The chemical reaction described in the present invention is generally carried out under normal pressure. The reaction temperature is between -78°C and 200°C. The reaction duration and condition are, for example, between -78°C and 200°C under one atmospheric pressure, and completed within about 1 to 24 hours. If the reaction is conducted overnight, then the reaction duration is generally 16 hours. Unless otherwise specified in the examples, the reaction temperature is room temperature, which is 20°C to 30°C.

The progress of the reaction in the examples is monitored by thin layer chromatography (TLC). The developing systems used in the reactions include: A: dichloromethane and methanol system, B: petroleum ether and ethyl acetate system, C: acetone. The volume ratio of the solvent is adjusted depending on the polarity of the compound.

The eluent systems of column chromatography and the developing systems of TLC for the purification of the compound include: A: dichloromethane and methanol system, B: petroleum ether and ethyl acetate system. The volume ratio of the solvent is adjusted depending on the polarity of the compound, and a small amount of an alkaline or acidic reagent such as triethylamine or trifluoroacetic acid may be added for adjustment.

Unless otherwise defined, all professional and scientific terms used herein have the same meaning as those familiar to those skilled in the art. In addition, any methods and materials similar or equivalent to the content described herein can be applied to the method of the present invention.

### Abbreviations

NMR = nuclear magnetic resonance
Boc = *tert*-butoxycarbonyl
BPD = benzenephosphorusdichloride
DCM = dichloromethane
DIPEA = diisopropylethylamine
DMA = N,N-dimethylaniline
DMAP = 4-dimethylaminopyridine
DMF = N,N-dimethylformamide
DMSO = dimethyl sulfoxide
EA = ethyl acetate
HPLC = high performance liquid chromatography
LC-MS = liquid chromatography-mass spectrometry
NBS = n-bromosuccinimide
PE = petroleum ether
tol. = toluene
TBSCI = *tert*-butyldimethylsilyl chloride
TEA = triethylamine
THF = tetrahydrofuran
TMEDA = N,N,N',N'-tetramethylethylenediamine.

### Example 1: Preparation of 2-(3-aminobenzofuro[2,3-b]pyridin-2-yl)propan-2-ol (1)

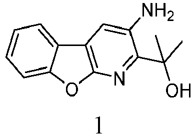

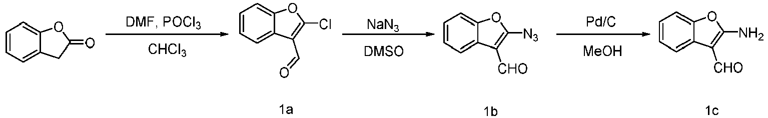

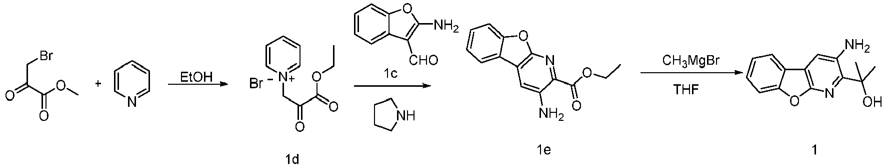

### Step 1: Preparation of 2-chlorobenzofuran-3-carbaldehyde (1a)

DMF (10.0 g, 123 mmol) was dissolved in chloroform (40 mL) at room temperature. The solution was cooled to 0°C, followed by the slow addition of phosphorus oxychloride (15.5 g, 101 mmol), and stirred for 10 minutes at 0-5°C under a nitrogen atmosphere. A solution of benzofuran-2(3H)-ketone (5.50 g, 41.0 mmol) in chloroform (40 mL) was slowly added dropwise at 0°C, and then the reaction system was refluxed for 18 hours. The reaction solution was concentrated under reduced pressure, followed by addition of water. The pH value was adjusted to 5 with potassium acetate, and then the pH value was adjusted to 7 with aqueous sodium hydroxide solution (2N). The reaction solution was extracted with dichloromethane, and the organic phase was dried over anhydrous sodium sulfate and filtered. The filtrate was concentrated under reduced pressure, and the residues were purified by silica gel column chromatography (mobile phase: PE/EA = 1:1) to obtain 3.20 g of the title compound as a white solid, yield: 43.3%.

LC-MS: m/z 181.0 [M + H]⁺.

¹H NMR (400 MHz, CDCl₃) *δ* 10.20 (s, 1H), 8.18-8.14 (m, 1H), 7.53-7.49 (m, 1H), 7.44-7.39 (m, 2H).

### Step 2: Preparation of 2-azidobenzofuran-3-carbaldehyde (1b)

2-Chlorobenzofuran-3-carbaldehyde (1a) (1.50 g, 8.33 mmol) was dissolved in DMSO (25 mL), followed by addition of sodium azide (1.05 g, 16.7 mmol). The reaction solution was stirred at 20°C for 1 hour. After the reaction was completed by LCMS monitoring, water (30 mL) was added, and the reaction solution was extracted with ethyl acetate (50 mL), dried over anhydrous sodium sulfate, and filtered. The filtrate was concentrated under reduced pressure to obtain 1.40 g of the title compound as a brownish-yellow solid, yield: 89.9%.

LC-MS: m/z 188.0 [M + H]⁺.

### Step 3: Preparation of 2-aminobenzofuran-3-carbaldehyde (1c)

2-Azidobenzofuran-3-carbaldehyde (1b) (500 mg, 2.67 mmol) and palladium on carbon (150 mg, 30% wt) were dissolved in methanol (20 mL) at room temperature. The reaction system was purged with hydrogen three times, and then stirred under a hydrogen atmosphere for 6 hours. After the reaction was completed by LCMS monitoring, the reaction solution was concentrated under reduced pressure to obtain 460 mg of the crude title compound as a brownish-yellow solid, which was directly used in the next step without purification.

LC-MS: m/z 162.1 [M + H]⁺.

### Step 4: Preparation of 1-(3-ethoxy-2,3-dioxopropyl)pyridin-1-ium bromide (1d)

Pyridine (246 mg, 3.11 mmol) was dissolved in anhydrous ethanol (8 mL) at room temperature, followed by the slowly dropwise addition of a solution of ethyl 3-bromo-2-oxopropionate (574 mg, 2.96 mmol) in anhydrous ethanol (8 mL). The reaction solution was then heated to 65°C and stirred for 1 hour, and directly used for the next step.

### Step 5: Preparation of ethyl 3-aminobenzofuro[2,3-b]pyridine-2-carboxylate (1e)

The reaction solution of step 4 was cooled to room temperature, followed by addition of 2-aminobenzofuran-3-carbaldehyde (1c) (417 mg, 2.59 mmol), pyridine (492 mg, 6.22 mmol) and anhydrous ethanol (10 mL). The reaction solution was heated to 85°C and stirred for 5 hours. Pyrrolidine (461 mg, 6.48 mmol) was then added, and the reaction solution was stirred for 3 hours at 85°C. After the reaction was completed by LCMS monitoring, the reaction solution was cooled and concentrated under reduced pressure. The residues were purified by silica gel column chromatography (mobile phase: PE/EA = 1:1) to obtain 110 mg of the title compound as a reddish-brown solid, yield: 16.6%.

LC-MS: m/z 257.1 [M + H]⁺.

### Step 6: Preparation of 2-(3-aminobenzofuro[2,3-b]pyridin-2-yl)propan-2-ol (1)

Methylmagnesium bromide (1.3 mL, 3 M in Et₂O, 3.91 mmol) was dissolved in tetrahydrofuran (10 mL) at room temperature. The solution was cooled to -5°C, followed by the slowly dropwise addition of a solution of ethyl 3-aminobenzofuro[2,3-*b*]pyridine-2-carboxylate (100 mg, 0.391 mmol) in tetrahydrofuran (10 mL), and stirred at -5°C for 2 hours. After the reaction was completed by LCMS monitoring, saturated aqueous solution of ammonium chloride (20 mL) was added, and the reaction solution was extracted with ethyl acetate (50 mL). The organic phase was dried over anhydrous sodium sulfate and filtered, and the filtrate was concentrated under reduced pressure. The residues were separated and purified by silica gel column chromatography (mobile phase: DCM/EA = 3:1) to obtain 7.4 mg of the title compound as a white solid, yield: 7.83%.

LC-MS: m/z 224.9[M-OH]⁺.

¹H NMR (400 MHz, CD₃OD) *δ* 7.93-7.91 (m, 1H), 7.70 (s, 1H), 7.55-7.53 (m, 1H), 7.50-7.45 (m, 1H), 7.35-7.30 (m, 1H), 1.69 (s, 6H).

### Example 2: Preparation of 2-(3-amino-9-methyl-9H-pyrido[2,3-b]indol-2-yl)propan-2-ol (2)

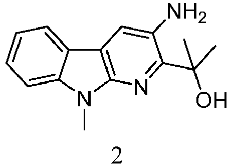

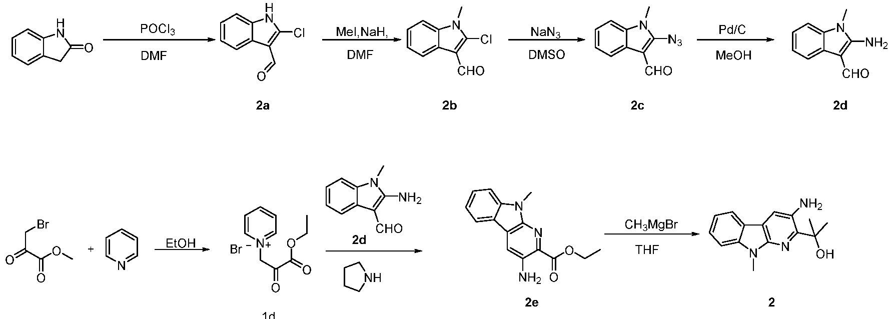

### Step 1: Preparation of 2-chloro-1H-indole-3-carbaldehyde (2a)

Indolin-2-one (12.0 g, 90.2 mmol) was dissolved in DMF (60 mL), followed by the slowly dropwise addition of phosphorus oxychloride (60 mL) at 0°C. The reaction solution was stirred for 24 hours at room temperature under a nitrogen atmosphere, then poured into 1.2 L of ice water and stirred for 24 hours and filtered. The filter cake was washed with purified water, and dried to obtain 12.8 g of the crude title compound as a yellow solid, which was directly used in the next step without purification.

LC-MS: m/z 180.0 [M + H]⁺.

### Step 2: Preparation of 2-chloro-1-methyl-1H-indole-3-carbaldehyde (2b)

2-Chloro-1*H*-indole-3-carbaldehyde (2a) (5.00 g, 27.9 mmol) and methyl iodide (4.76 g, 33.5 mmol) were dissolved in DMF (10 mL) at 0°C, followed by addition of sodium hydrogen (1.23 g, 30.7 mmol, in mineral oil), and the reaction solution was stirred at 20°C for 4 hours. After the reaction was completed, water (30 mL) was added, and the reaction solution was extracted with ethyl acetate (50 mL), dried over anhydrous sodium sulfate and filtered. The filtrate was concentrated under reduced pressure, and the residues were purified by silica gel column chromatography (mobile phase: PE/EA = 1:1) to obtain 2.60 g of the title compound as a yellow solid, yield: 48.2%.

LC-MS: m/z 194.1 [M + H]⁺.

### Step 3: Preparation of 2-azido-1-methyl-1H-indole-3-carbaldehyde (2c)

2-Chloro-1-methyl-1*H*-indole-3-carbaldehyde (2b) (4.10 g, 21.2 mmol) and sodium azide (2.68 g, 42.4 mmol) were dissolved in DMSO (40 mL) at room temperature, and the solution was stirred for 24 hours. After the reaction was completed, water (30 mL) was added, and the reaction solution was extracted with ethyl acetate (50 mL), dried over anhydrous sodium sulfate and filtered. The filtrate was concentrated under reduced pressure, and the residues were purified by silica gel column chromatography (mobile phase: PE/EA = 1:1) to obtain 2.90 g of the title compound as a yellow solid, yield: 68.2%.

LC-MS: m/z 201.1 [M + H]⁺.

### Step 4: Preparation of 2-amino-1-methyl-1H-indole-3-carbaldehyde (2d)

2-Azido-1-methyl-1*H*-indole-3-carbaldehyde (2c) (1.00 g, 4.99 mmol) and palladium on carbon (150 mg, 15.0% wt) were dissolved in methanol (30 mL) at room temperature. The reaction system was purged with hydrogen three times, and then stirred under a hydrogen atmosphere for 5 hours. After the reaction was completed by LCMS monitoring, the reaction solution was concentrated under reduced pressure to obtain 700 mg of the crude title compound as a brownish-yellow solid, which was directly used in the next step without purification.

LC-MS: m/z 175.2 [M + H]⁺.

### Step 5: Preparation of 1-(3-ethoxy-2,3-dioxopropyl)pyridin-1-ium bromide (1d)

Pyridine (245 mg, 3.10 mmol) was dissolved in anhydrous ethanol (8 mL) at room temperature, followed by the slowly dropwise addition of a solution of ethyl 3-bromo-2-oxopropionate (530 mg, 2.73 mmol) in anhydrous ethanol (10 mL). The reaction solution was then heated to 65°C and stirred for 1 hour, and directly used for the next step.

### Step 6: Preparation of ethyl 3-amino-9-methyl-9H-pyrido[2,3-b]indole-2-carboxylate (2e)

The reaction solution of step 5 was cooled to room temperature, followed by addition of 2-amino-1-methyl-1*H*-indole-3-carbaldehyde (2d) (450 mg, 2.59 mmol), pyridine (490 mg, 6.21 mmol) and anhydrous ethanol (10 mL), and heated to 85°C for 5 hours. Pyrrolidine (460 mg, 6.47 mmol) was then added, and the reaction solution was stirred for 3 hours at 85°C. After the reaction was completed by LCMS monitoring, the reaction solution was cooled and concentrated under reduced pressure. The residues were purified by silica gel column chromatography (mobile phase: PE/EA = 1:1) to obtain 300 mg of the title compound as a reddish-brown solid, yield: 43.1%.

LC-MS: m/z 270.1 [M + H]⁺.

### Step 7: Preparation of 2-(3-amino-9-methyl-9H-pyrido[2,3-b]indol-2-yl)propan-2-ol (2)

Methylmagnesium bromide (2.61 mL, 3 M in THF, 7.84 mmol) was dissolved in tetrahydrofuran (10 mL) at room temperature. The solution was cooled to -5°C, followed by the slowly dropwise addition of a solution of ethyl 3-amino-9-methyl-9*H*-pyrido[2,3-*b*]indole-2-carboxylate (2e) (300 mg, 1.12 mmol) in tetrahydrofuran (10 mL), stirred at -5°C for 15 minutes, and then stirred at room temperature for 5 hours. After the reaction was completed by LCMS monitoring, saturated aqueous solution of ammonium chloride (20 mL) was added, and the reaction solution was extracted with ethyl acetate (50 mL). The organic phase was dried over anhydrous sodium sulfate and filtered, and the filtrate was concentrated under reduced pressure. The residues were purified by silica gel column chromatography (mobile phase: PE/EA = 1:1) to obtain 36.6 mg of the title compound as a white solid, yield: 12.9%.

LC-MS: m/z 256.1 [M + H]⁺.

¹H NMR (400 MHz, DMSO-*d*₆) δ 7.98 (d, J = 7.6 Hz, 1H), 7.75 (s, 1H), 7.49 (d, J = 8.0 Hz, 1H), 7.43 (t, J = 7.6 Hz, 1H), 7.15 (t, J = 7.6 Hz, 1H), 3.79 (s, 3H), 1.63 (s, 6H).

### Example 3: Preparation of 2-(3-amino-9-methyl-9H-carbazol-2-yl)propan-2-ol (3)

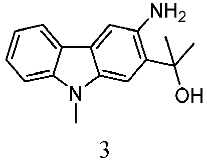

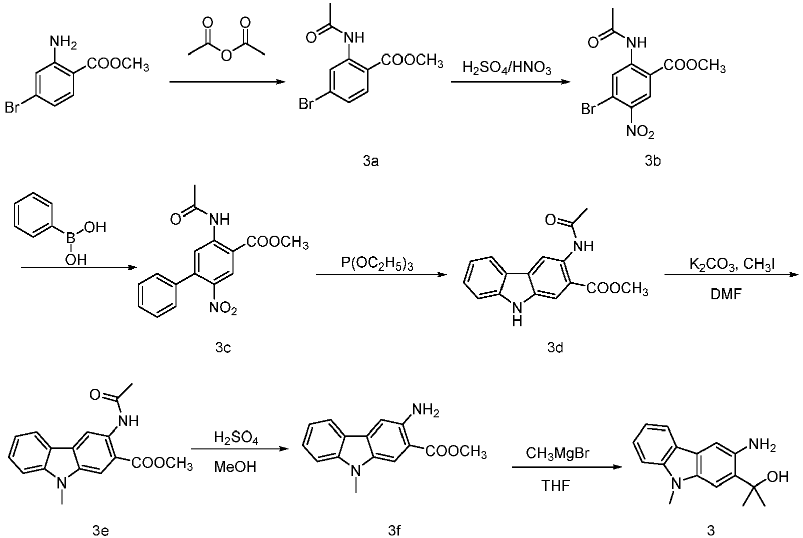

### Step 1: Preparation of methyl 2-acetamido-4-bromobenzoate (3a)

Methyl 2-amino-4-bromobenzoate (10.0 g, 44.0 mmol) was dissolved in 1,4-dioxane (100 mL) at room temperature, followed by the slow addition of acetic anhydride (60 mL). The reaction solution was heated to 50°C, and continuously stirred for 8 hours. The reaction solution was cooled to room temperature and concentrated under reduced pressure, and the residues were purified by silica gel column chromatography (mobile phase: PE/EA = 2:1) to obtain 9.10 g of the title compound as a gray-white solid, yield: 76.4%.

LC-MS: m/z 271.8 [M + H]⁺.

### Step 2: Preparation of methyl 2-acetamido-4-bromo-5-nitrobenzoate (3b)

Methyl 2-acetamido-4-bromobenzoate (3a) (9.00 g, 33.2 mmol) was dissolved in concentrated sulfuric acid (30 mL) at room temperature, followed by the slowly dropwise addition of concentrated nitric acid (6 mL) in an ice bath. The reaction solution was naturally raised to room temperature and continuously stirred for 2 hours. After the reaction was complete by LCMS monitoring, the reaction solution was slowly poured into ice water (50 mL), and then extracted three times with ethyl acetate (20 mL), dried over anhydrous sodium sulfate, and filtered. The filtrate was concentrated under reduced pressure, and the residues were purified by silica gel column chromatography (mobile phase: PE/EA = 2:1) to obtain 3.60 g of the title compound as a yellow solid, yield: 34.6%.

LC-MS: m/z 317.0 [M + H]⁺.

### Step 3: Preparation of methyl 5-acetamido-2-nitro-[1,1'-biphenyl]-4-carboxylate (3c)

Methyl 2-acetamido-4-bromo-5-nitrobenzoate (3b) (3.50 g, 11.1 mmol), phenylboronic acid (4.03 g, 33.0 mmol), potassium carbonate (3.04 g, 22.0 mmol) and tetrakis(triphenylphosphine)palladium (636 mg, 0.550 mmol) were dissolved in 1,4-dioxane (100 mL) and water (5 mL) at room temperature. The reaction system was purged with nitrogen three times, and then heated to 100°C, and stirred for 8 hours under a nitrogen atmosphere. After the reaction was completed by LCMS monitoring, the reaction solution was cooled to room temperature, extracted with ethyl acetate, dried over anhydrous sodium sulfate, and filtered. The filtrate was concentrated under reduced pressure, and the residues were purified by silica gel column chromatography (mobile phase: PE/EA = 5:1) to obtain 1.20 g of the title compound as a yellow solid, yield: 34.8%.

LC-MS: m/z 315.1 [M + H]⁺.

### Step 4: Preparation of methyl 3-acetamido-9H-carbazole-2-carboxylate (3d)

Methyl 5-acetamido-2-nitro-[1,1'-biphenyl]-4-carboxylate (3c) (1.00 g, 3.20 mmol) was dissolved in triethyl phosphite (15 mL) at room temperature, and then the solution was heated to 140°C and stirred for 8 hours. The reaction solution was cooled to room temperature, followed by addition of water (20 mL) and ethyl acetate (20 mL) to extract three times, dried over anhydrous sodium sulfate, and filtered. The filtrate was concentrated under reduced pressure, and the residues were purified by silica gel column chromatography (mobile phase: PE/EA = 5:1) to obtain 700 mg of the title compound as a light yellow solid, yield: 77.6%.

### Step 5: Preparation of methyl 3-acetamido-9-methyl-9H-carbazole-2-carboxylate (3e)

Methyl 3-acetamido-9*H*-carbazole-2-carboxylate (3d) (500 mg, 1.80 mmol), potassium carbonate (497 mg, 3.60 mmol) and methyl iodide (310 mg, 2.20 mmol) were dissolved in acetone (10 mL) at room temperature. The reaction system was heated to 40°C and stirred for 4 hours. After the reaction was complete by LCMS monitoring, the reaction solution was cooled to room temperature and filtered. The filtrate was concentrated under reduced pressure, and the residues were purified by silica gel column chromatography (mobile phase: PE/EA = 5:1) to obtain 300 mg the title compound as a yellow solid, yield: 57.1%.

LC-MS: m/z 296.9 [M + H]⁺.

### Step 6: Preparation of methyl 3-amino-9-methyl-9H-carbazole-2-carboxylate (3f)

Methyl 3-acetamido-9-methyl-9*H*-carbazole-2-carboxylate (300 mg, 1.01 mmol) was dissolved in anhydrous methanol (10 mL) at room temperature, followed by the slowly dropwise addition of concentrated sulfuric acid (1 mL). The reaction solution was stirred for 4 hours at room temperature. After the reaction was completed by LCMS monitoring, the reaction solution was concentrated to remove methanol, followed by addition of sodium hydroxide solution (1 N) to adjust the pH to 8-10, and then extracted with ethyl acetate, dried over anhydrous sodium sulfate, and filtered. The filtrate was concentrated under reduced pressure, and the residues were purified by silica gel column chromatography (mobile phase: PE/EA = 2:1) to obtain 160 mg of the title compound as a light yellow solid, yield: 62.9%.

LC-MS: m/z 254.9 [M + H]⁺.

### Step 7: Preparation of 2-(3-amino-9-methyl-9H-carbazol-2-yl)propan-2-ol (3)

Methylmagnesium bromide (0.8 mL, 3 M in Et₂O, 2.40 mmol) was dissolved in tetrahydrofuran (10 mL) at room temperature. The solution was cooled to 0°C, followed by the slowly dropwise addition of a solution of methyl 3-amino-9-methyl-9*H*-carbazole-2-carboxylate (3f) (100 mg, 0.390 mmol) in tetrahydrofuran (5 mL), and then stirred at 0°C for 2 hours. After the reaction was completed by LCMS monitoring, saturated aqueous solution of ammonium chloride (20 mL) was added, and the reaction solution was extracted with ethyl acetate (50 mL). The organic phase was dried over anhydrous sodium sulfate and filtered, and the filtrate was concentrated under reduced pressure. The residues were purified by silica gel column chromatography (mobile phase: DCM/EA = 1:1), and then purified by preparative liquid chromatography (column model: Xbridge 150 x 19 mm, C18, 5µm, 100A, mobile phase: acetonitrile/water, gradient: 20%-60%) to obtain 7.3 mg of the title compound as an off-white solid, yield: 7.31%.

LC-MS: m/z 237.0 [(M-H₂O) + H]⁺.

¹H NMR (400 MHz, CD₃OD) *δ* 7.96 (d, J = 8.0 Hz, 1H), 7.50 (s, 1H), 7.39 (d, J = 4.4 Hz, 2H), 7.33 (s, 1H), 7.13-7.09 (m, 1H), 3.82 (s, 3H). 1.79 (s, 6H).

### Example 4: Preparation of 2-(3-aminodibenzo[b,e][1,4]dioxin-2-yl)propan-2-ol (4)

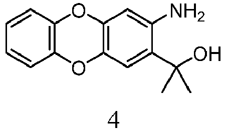

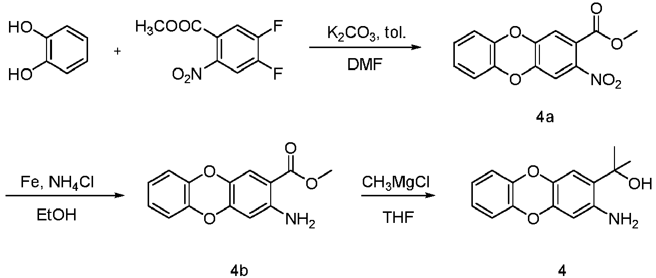

### Step 1: Preparation of methyl 3-nitrodibenzo[b,e][1,4]dioxine-2-carboxylate (4a)

Catechol (1.00 g, 4.60 mmol), methyl 4,5-difluoro-2-nitrobenzoate (510 mg, 4.60 mmol) and potassium carbonate (1.27 g, 9.20 mmol) were dissolved in toluene (100 mL) and DMF (50 mL) at room temperature. The reaction solution was heated to 130°C, and stirred under reflux for 18 hours. The organic phase was extracted with 100 mL of ethyl acetate and 100 mL of water, and the aqueous phase was extracted again with 20 mL of ethyl acetate. The organic phases were combined, washed again with 100 mL of water, and concentrated under reduced pressure. The residues were purified by silica gel column chromatography (mobile phase: PE/EA=10:1-7:1) to obtain 500 mg of the title compound as a yellow solid, yield: 37.8%.

LC-MS: m/z 287.04 [M + H]⁺.

### Step 2: Preparation of methyl 3-aminodibenzo[b,e][1,4]dioxine-2-carboxylate (4b)

Iron powder (480 mg, 8.60 mmol) and ammonium chloride (59 mg, 1.10 mmol) were dissolved in a mixed solvent of ethanol (10 mL) and water (1 mL) (ethanol: water = 10:1) at room temperature, followed by addition of methyl 3-nitrodibenzo[*b*,*e*][1,4]dioxine-2-carboxylate (4a) (450 mg, 1.57 mmol). The reaction solution was heated to 79°C and stirred under reflux for 18 hours. The reaction solution was directly concentrated under reduced pressure, and the residues were purified by silica gel column chromatography (mobile phase: PE/EA=10:1-4:1) to obtain 280 mg of the title compound as a gray-white solid, yield: 69.4%.

LC-MS: m/z 257.07 [M + H]⁺.

### Step 3: Preparation of 2-(3-aminodibenzo[b,e][1,4]dioxin-2-yl)propan-2-ol (4)

Methylmagnesium chloride (2.43 mL, 7.35 mmol) was dissolved in tetrahydrofuran (5 mL) at 0°C, followed by the slowly dropwise addition of a solution of methyl 3-aminodibenzo[*b*,*e*][1,4]dioxine-2-carboxylate (4b) in tetrahydrofuran (5 mL) under a nitrogen atmosphere. The reaction solution was stirred for 18 hours at room temperature and quenched with saturated solution of ammonium chloride (5 mL). After the reaction was completed, the reaction solution was extracted with water (100 mL) and ethyl acetate (50 mL), and the aqueous phase was extracted again with 50 mL of ethyl acetate. The organic phases were combined and concentrated under reduced pressure. The residues were purified by preparative liquid chromatography (column model: Daisogei 30 mm x 250 mm, C18, 10 µm, 100 Å, mobile phase: acetonitrile/water, gradient: 2-22-29 min, 20-60-95%, target time: 24.6 min) to obtain 120 mg of the title compound as a white solid, yield: 44.5%.

LC-MS: m/z 258.5 [M + H]⁺.

¹H NMR (300 MHz, DMSO-*d*₆) *δ* 6.90-6.86 (s, 4H), 6.60 (s, 1H), 6.23 (s, 1H), 5.31 (s, 2H), 5.20 (s, 1H), 1.43 (s, 6H).

### Example 5: Preparation of 2-(2-amino-10-methyl-10H-phenoxazin-3-yl)propan-2-ol (5)

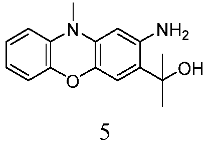

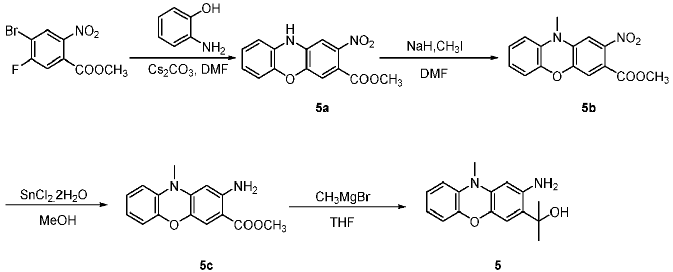

### Step 1: Preparation of methyl 2-nitro-10H-phenoxazine-3-carboxylate (5a)

Methyl 4-bromo-5-fluoro-2-nitrobenzoate (1.00 g, 3.61 mmol) was dissolved in N,N-dimethylformamide (10 mL) at room temperature, followed by addition of 2-aminophenol (393 mg, 3.61 mmol) and cesium carbonate (2.35 g, 7.22 mmol). The reaction solution was heated to 120°C and stirred for 24 hours under a nitrogen atmosphere. The reaction solution was cooled to room temperature, and concentrated under reduced pressure. The residues were purified by silica gel column chromatography (mobile phase: DCM) to obtain 530 mg of the title compound as a red solid, yield: 51.3%.

LC-MS: m/z 287.1 [M + H]⁺.

### Step 2: Preparation of methyl 10-methyl-2-nitro-10H-phenoxazine-3-carboxylate (5b)

Methyl 2-nitro-10*H*-phenoxazine-3-carboxylate (5a) (400 mg, 1.40 mmol) was dissolved in anhydrous N,N-dimethylformamide (5 mL) at 0°C, followed by addition of sodium hydride (67.0 mg, 2.79 mmol). The reaction solution was stirred for 30 minutes at 0°C, followed by addition of methyl iodide (992 mg, 6.99 mmol), and stirred at 0°C for 2 hours. The reaction soultion was quenched with water (3 mL), then extracted with dichloromethane, and concentrated under reduced pressure. The residues were purified by silica gel column chromatography (mobile phase: DCM) to obtain 380 mg of the title compound as a red solid, yield: 90.6%.

LC-MS: m/z 300.8 [M + H]⁺.

### Step 3: Preparation of methyl 2-amino-10-methyl-10H-phenoxazine-3-carboxylate (5c)

Stannous chloride (208 mg, 0.925 mmol) was dissolved in ethanol (5 mL) at room temperature, followed by the slow addition of methyl 10-methyl-2-nitro-10*H*-phenoxazine-3-carboxylate (5b) (50.0 mg, 0.185 mmol). The reaction solution was heated to 70°C and stirred for 5 hours under a nitrogen atmosphere. The reaction solution was cooled to room temperature, and concentrated under reduced pressure. The residues were purified by Prep-TLC (developing agent: DCM) to obtain 20.0 mg of the title compound as a green solid, yield: 40.0%.

### Step 4: Preparation of 2-(2-amino-10-methyl-10H-phenoxazin-3-yl)propan-2-ol (5)

Methyl 2-amino-10-methyl-10*H*-phenoxazine-3-carboxylate (5c) (100 mg, 0.369 mmol) was dissolved in anhydrous tetrahydrofuran (5 mL) at 0°C, followed by the slow addition of methylmagnesium bromide (7.38 mL, 7.38 mmol) at 0°C. The reaction system was raised to room temperature and stirred for 2 hours. The reaction solution was quenched with saturated solution of ammonium chloride, and then extracted with ethyl acetate, concentrated under reduced pressure. The residues were purified by Prep-TLC (developing agent: EA/PE = 1:2) to obtain 11.0 mg of the title compound as a gray solid, yield: 13.5%.

LC-MS: m/z 270.9 [M + H]⁺.

¹H NMR (300 MHz, DMSO-*d*₆) *δ* 6.82-6.80 (m, 1H), 6.65-6.61 (m, 3H), 6.36 (s, 1H), 6.13 (s, 1H), 5.15 (s, 3H), 2.96 (s, 3H), 1.47 (s, 6H).

### Example 6: Preparation of 2-(3-amino-10-methyl-10H-phenoxazin-2-yl)propan-2-ol (6)

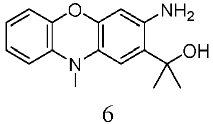

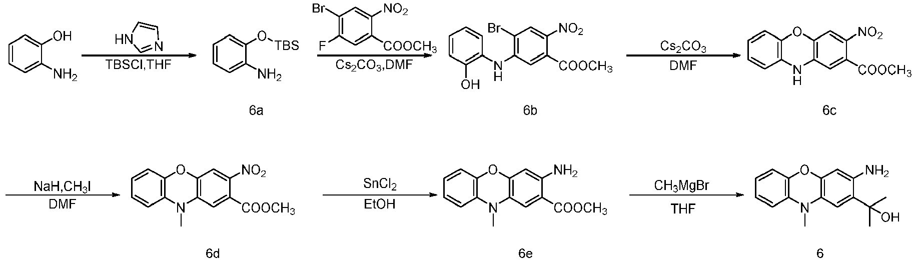

### Step 1: Preparation of 2-((tert-butyldimethylsilyl)oxy)aniline (6a)

2-Aminophenol (1.00 g, 9.16 mmol) and imidazole (0.624 g, 18.3 mmol) were added to tetrahydrofuran (10 mL) at room temperature. The mixture was cooled to 0°C, and *tert*-butyldimethylchlorosilane (1.38 mg, 9.61 mmol) was added thereto. The reaction solution was stirred for 16 hours under a nitrogen atmosphere. The reaction solution was washed with NaOH. The organic phase was extracted with ethyl ether, and concentrated under reduced pressure to obtain 1.74 g of the title compound as a red liquid, yield: 85.0%.

### Step 2: Preparation of methyl 4-bromo-5-((2-hydroxyphenyl)amino)-2-nitrobenzoate (6b)

2-((*tert*-Butyldimethylsilyl)oxy)aniline (6a) (200 mg, 0.896 mmol) was added to *N,N-*dimethylformamide (10 mL) at room temperature, followed by addition of methyl 4-bromo-5-fluoro-2-nitrobenzoate (249 mg, 0.896 mmol) and cesium carbonate (584 mg, 1.79 mmol). The reaction solution was heated to 120°C and stirred for 16 hours under a nitrogen atmosphere. The reaction solution was cooled to room temperature and washed with water, and then the organic phase was extracted with ethyl acetate. The organic phase was dried over anhydrous sodium sulfate and filtered. The filtrate was concentrated under reduced pressure, and the residues were purified by silica gel column chromatography (mobile phase: EA/PE = 1:1) to obtain 142 mg of the title compound as a red solid, yield: 43.3%.

### Step 3: Preparation of methyl 3-nitro-10H-phenoxazine-2-carboxylate (6c)

Methyl 4-bromo-5-((2-hydroxyphenyl)amino)-2-nitrobenzoate (6b) (50.0 mg, 0.136 mmol) was added to *N,N*-dimethylformamide (10 mL), followed by addition of cesium carbonate (89.0 mg, 0.272 mmol) at room temperature. The reaction solution was heated to 140°C and stirred for 16 hours under a nitrogen atmosphere. The reaction solution was cooled to room temperature and washed with water, and then the organic phase was extracted with ethyl acetate. The organic phase was dried over anhydrous sodium sulfate and filtered. The filtrate was concentrated under reduced pressure, and the residues were purified by silica gel column chromatography (mobile phase: EA/PE = 1:3) to obtain 30.0 mg of the title compound as a red solid, yield: 77.1%.

### Step 4: Preparation of methyl 10-methyl-3-nitro-10H-phenoxazine-2-carboxylate (6d)

Methyl 3-nitro-10*H*-phenoxazine-2-carboxylate (7c) (120 mg, 0.105 mmol) was added to anhydrous *N,N-*dimethylformamide (5 mL), followed by addition of sodium hydride (20.0 mg, 0.210 mmol, 60% in mineral oil) at 0°C. The reaction solution was stirred for 30 minutes, followed by addition of methyl iodide (180 mg, 0.315 mmol). The reaction system was stirred at 0°C for 2 hours, and quenched with water (3 mL). The organic phase was extracted with dichloromethane, and concentrated under reduced pressure. The residues were purified by silica gel chromatography (mobile phase: EA/PE = 1:3) to obtain 100 mg of the title compound as a red solid, yield: 79.5%.

### Step 5: Preparation of methyl 3-amino-10-methyl-10H-phenoxazine-2-carboxylate (6e)

Stannous chloride (316 mg, 1.67 mmol) was added to ethanol (5 mL) at room temperature, followed by the slow addition of methyl 10-methyl-3-nitro-10*H*-phenoxazine-2-carboxylate (6d) (100 mg, 0.330 mmol). The reaction solution was heated to 70°C and stirred for 5 hours under a nitrogen atmosphere. The reaction solution was cooled to room temperature, and concentrated under reduced pressure. The residues were purified by silica gel chromatography (mobile phase: MeOH/DCM = 1:20) to obtain 82.0 mg of the title compound as a green solid, yield: 91.9%.

### Step 6: Preparation of 2-(3-amino-10-methyl-10H-phenoxazin-2-yl)propan-2-ol (6)

Methyl 3-amino-10-methyl-10*H*-phenoxazine-2-carboxylate (6e) (50.0 mg, 0.111 mmol) was added to a three-neck bottle at 0°C. The reaction system was purged with nitrogen, followed by the slow addition of anhydrous tetrahydrofuran (5 mL). Methylmagnesium bromide (66.0 mg, 0.333 mmol) was slowly added at 0°C under a nitrogen atmosphere, then the reaction system was stirred for 3 hours at room temperature. The reaction solution was quenched with an aqueous ammonium chloride solution, and then extracted with dichloromethane, and concentrated under reduced pressure. The residues were purified with Prep-TLC (developing agent: EA/PE = 1:5) to obtain the title compound as an off-white solid 33.0 mg, yield: 66.0%.

LC-MS: m/z 271.1 [M + H]⁺.

¹H NMR (300 MHz, CDCl₃) *δ* 6.83-6.81 (m, 1H), 6.67 (s, 2H), 6.50-6.47 (m, 2H), 6.31 (s, 1H), 6.14 (s, 1H), 3.50 (br, 3H), 3.01 (s, 3H), 1.64 (s, 6H).

### Example 7: Preparation of 3-(2-amino-10-methyl-10H-phenoxazin-3-yl)pentan-3-ol (7)

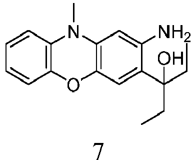

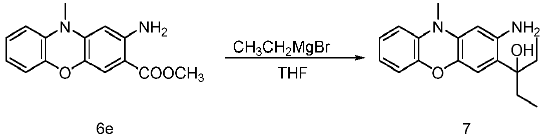

Methyl 2-amino-10-methyl-10*H*-phenoxazine-3-carboxylate (6e) (500 mg, 1.85 mmol) was added to a three-neck bottle at 0°C. The reaction system was purged with nitrogen, followed by the slowly dropwise addition of anhydrous tetrahydrofuran (5 mL). Tetraisopropyl titanate (370 mg, 1.30 mmol) was slowly added at 0°C under a nitrogen atmosphere, then the reaction system was stirred for 2 hours at room temperature. Ethylmagnesium bromide (740 mg, 5.55 mmol) was slowly added at 0°C, and then the reaction system was stirred for 3 hours at room temperature. The reaction solution was quenched with water, then extracted with ethyl acetate, and concentrated under reduced pressure. The residues were purified by silica gel column chromatography (mobile phase: PE/EA=1:5) to obtain 66.8 mg of the title compound as a light yellow solid, yield: 12.1%.

LC-MS: m/z 299.9 [M + H]⁺.

¹H NMR (400 MHz, DMSO-*d*₆) *δ* 6.85-6.81 (m, 1H), 6.66-6.62 (m, 3H), 6.26 (s, 1H), 6.09 (s, 1H), 5.07 (s, 2H), 4.76 (s, 1H), 2.96 (s, 3H), 1.79-1.73 (m, 4H), 0.76-0.72 (m, 6H).

### Example 8: Preparation of 2-(2-amino-dibenzo[b,d]furan-3-yl)propan-2-ol (8)

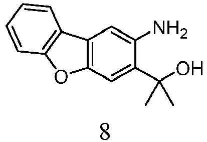

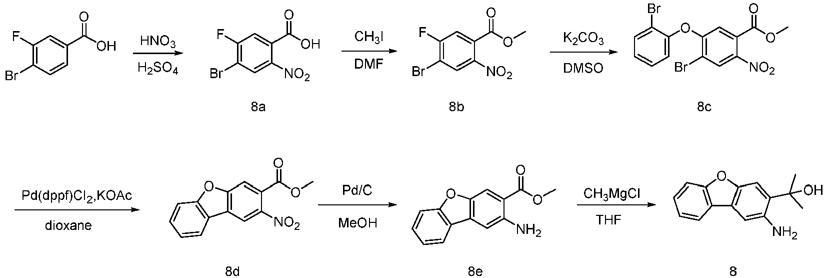

### Step 1: Preparation of 4-bromo-5-fluoro-2-nitrobenzoic acid (8a)

4-Bromo-3-fluorobenzoic acid (3.00 g, 11.3 mmol) was dissolved in concentrated sulfuric acid (20 mL) at 0°C, followed by the slowly dropwise addition of concentrated nitric acid (4.20 mL, 33.9 mmol). The reaction solution was stirred for 18 hours at room temperature, poured into ice water (100 mL) and filtered. The filter cake was washed with 50 mL of water and dried under reduced pressure to obtain the title compound as a light yellow solid 2.5 g, yield: 71.6%.

### Step 2: Preparation of methyl 4-bromo-5-fluoro-2-nitrobenzoate (8b)

4-Bromo-5-fluoro-2-nitrobenzoic acid (8a) (2.00 g, 7.60 mmol) and potassium carbonate (2.10 g, 15.2 mmol) were dissolved in DMF (30 mL) at room temperature, followed by addition of methyl iodide (3.24 g, 22.8 mmol). The reaction solution was stirred for 18 hours at room temperature, and extracted with ethyl acetate (80 mL) and water (80 mL). The organic phase was concentrated under reduced pressure, and the residues were purified by silica gel column chromatography (mobile phase: PE/EA=50:1-8:1) to obtain the title compound as a dark yellow solid 2.00 g, yield: 94.9%.

### Step 3: Preparation of methyl 4-bromo-5-(2-bromophenoxy)-2-nitrobenzoate (8c)

Methyl 4-bromo-5-fluoro-2-nitrobenzoate (8b) (1.00 g, 3.61 mmol), o-bromophenol (749 mg, 4.33 mmol) and potassium carbonate (996 mg, 7.22 mmol) were dissolved in DMSO (20 mL) at room temperature. The reaction solution was stirred for 16 hours at 80°C, and extracted with ethyl acetate (80 mL) and water (80 mL). The organic phase was concentrated under reduced pressure, and the residues were purified by silica gel column chromatography (mobile phase: PE/EA=5:1) to obtain 1.35 g of the title compound as a yellow solid, yield: 87.2%.

### Step 4: Preparation of methyl 2-nitro-dibenzo[b,d]furan-3-carboxylate (8d)

Methyl 4-bromo-5-(2-bromophenoxy)-2-nitrobenzoate (8c) (600 mg, 1.40 mmol), potassium acetate (548 mg, 5.60 mmol), BPD (355 mg, 1.40 mmol) and Pd(dppf)Cl₂ (102 mg, 0.140 mmol) were added to dioxane (20 mL) at room temperature. The reaction solution was stirred at 100°C for 16 hours under a nitrogen atmosphere. The reaction solution was concentrated under reduced pressure, and the residues were purified by silica gel column chromatography (mobile phase: PE/EA=5:1) to obtain 150 mg of the title compound as a white solid, yield: 39.4%.

### Step 5: Preparation of methyl 2-amino-dibenzo[b,d]furan-3-carboxylate (8e)

Methyl 2-nitro-dibenzo[*b*,*d*]furan-3-carboxylate (8d) (300 mg, 1.11 mmol) and Pd/C (45.0 mg) were added to methanol (15 mL) at room temperature. The reaction solution was stirred for 16 hours at room temperature under a hydrogen atmosphere. The reaction system was filtered, and the filtrate was concentrated under reduced pressure. The residues were purified by silica gel column chromatography (mobile phase: PE/EA=5:1) to obtain 120 mg of the title compound as a light yellow solid, yield: 45.0%.

### Step 6: Preparation of 2-(2-amino-dibenzo[b,d]furan-3-yl)propan-2-ol (8)

Methylmagnesium chloride (3 N, 0.72 mL, 2.17 mmol) was added dropwise to THF (5 mL) at 0°C under a nitrogen atmosphere, followed by addition of methyl 2-amino-dibenzo[*b*,*d*]furan-3-carboxylate (8e) dissolved in THF (4 mL) (40.0 mg, 0.166 mmol). The reaction solution was heated to 6°C, followed by the dropwise addition of methylmagnesium chloride (3 N, 1.08 mL, 3.27 mmol). The reaction solution was stirred at 60°C for 3 hours, quenched with 5 mL of water, followed by addition of 15 mL of water. The reaction solution was extracted with 20 mL of ethyl acetate, and the aqueous phase was extracted twice with 15 mL of ethyl acetate. The organic phases were combined, dried over anhydrous sodium sulfate, and filtered. The filtrate was concentrated under reduced pressure, and the residues were purified by preparative liquid chromatography (column model: Daisogei 30 mm x 250 mm, C18, 10 µm, 100A, mobile phase: acetonitrile/water, gradient: 10%-60%) to obtain 40.00 mg of the title compound as a light yellow solid, yield: 50.0%.

LC-MS: m/z 242.23 [M+H]⁺.

¹H NMR (300 MHz, DMSO-*d*₆) *δ* 7.93(dd, J = 7.6 Hz, 1H), 7.56 (d, J = 7.6 Hz, 1H), 7.47-7.27 (m, 3H), 7.23(s, 1H), 5.45 (s, 2H), 5.39 (s, 1H), 1.59 (s, 6H).

### Example 9: Preparation of 2-(3-aminobenzo[5,6][1,4]dioxino[2,3-b]pyridin-2-yl)propan-2-ol (9)

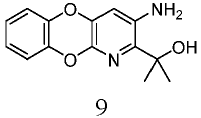

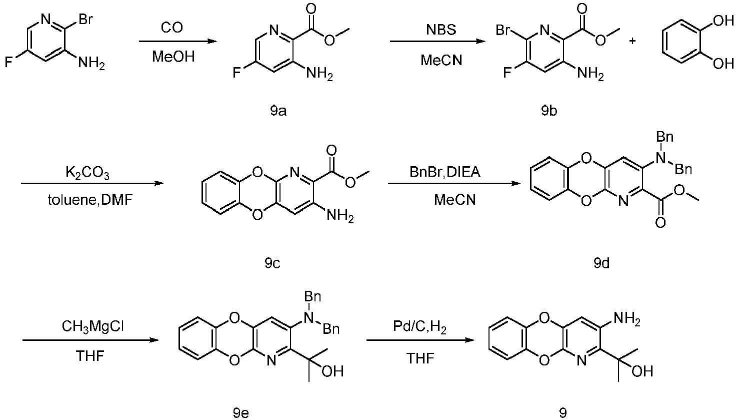

### Step 1: Preparation of methyl 3-amino-5-fluoropicolinate (9a)

2-Bromo-5-fluoropyridin-3-amine (5.00 g, 26.2 mmol), TEA (4.23 g, 41.9 mmol) and Pd(dppf)Cl₂ (1.92 g, 2.62 mmol) were dissolved in MeOH (50 mL) at room temperature, and the reaction solution was stirred overnight at 90°C under a carbon monoxide atmosphere. The reaction solution was concentrated under reduced pressure, and the residues were purified by silica gel column chromatography (mobile phase: petroleum ether/ethyl acetate = 1:1) to obtain 3.00 g of the title compound as a yellow solid, yield: 67.4%.

### Step 2: Preparation of methyl 3-amino-6-bromo-5-fluoropicolinate (9b)

Methyl 3-amino-5-fluoropicolinate (9a) (2.00 g, 11.8 mmol) was dissolved in MeCN (100 mL) at room temperature, followed by addition of NBS (2.09 g, 11.7 mmol) at 0°C. The reaction solution was stirred overnight at room temperature, followed by addition of 200 mL of water. The reaction solution was extracted with 200 mL of ethyl acetate, and the aqueous phase was extracted again with 200 mL of ethyl acetate. The organic phases were combined, dried over anhydrous sodium sulfate, and filtered. The filtrate was concentrated under reduced pressure, and the residues were purified by silica gel column chromatography (mobile phase: petroleum ether/ethyl acetate = 5:1) to obtain 2.00 g of the title compound as a yellow solid, yield: 68.5%.

### Step 3: Preparation of methyl 3-aminobenzo[5,6][1,4]dioxino[2,3-b]pyridine-2-carboxylate (9c)

Methyl 3-amino-6-bromo-5- fluoropicolinate (9b) (2.00 g, 8.06 mmol), catechol (887 mg, 8.06 mmol) and potassium carbonate (2.23 g, 16.2 mmol) were dissolved in toluene (10 mL) and DMF (50 mL) at room temperature, and the reaction solution was stirred overnight at 130°C. After direct filtration, the filter cake was dried under reduced pressure to obtain 1.30 g of the crude title compound as yellow solid.

### Step 4: Preparation of methyl 3-(dibenzylamino)benzo[5,6][1,4]dioxino[2,3-b]pyridine-2-carboxylate (9d)

Methyl 3-aminobenzo[5,6][1,4]dioxin[2,3-*b*]pyridine-2-carboxylate (9c) (1.30 g, 5.04 mmol), benzyl bromide (2.58 g, 15.1 mmol) and DIPEA (1.95 g, 15.1 mmol) were dissolved in MeCN (100 mL) at room temperature, and the reaction solution was stirred overnight at 90°C under a nitrogen atmosphere. The reaction solution was concentrated under reduced pressure, and the residues were purified by silica gel column chromatography (mobile phase: petroleum ether/ethyl acetate = 5:1) to obtain 800 mg of the title compound as a yellow solid, yield: 36.2%.

### Step 5: Preparation of 2-(3-(dibenzylamino)benzo[5,6][1,4]dioxino[2,3-b]pyridin-2-yl)propan-2-ol (9e)

Methyl 3-(dibenzylamino)benzo[5,6][1,4]dioxin[2,3-*b*]pyridine-2-carboxylate (9d) (200 mg, 0.457 mmol) was dissolved in THF (10 mL) at room temperature, followed by the dropwise addition of CH₃Li (1.6 M, 1 mL) under a nitrogen atmosphere at 0°C. The reaction solution was stirred for 5 hours at room temperature, followed by addition of 2 mL of methanol. The reaction solution was extracted with 50 mL of ethyl acetate and 50 mL of water, and the aqueous phase was extracted again with 50 mL of ethyl acetate. The organic phases were combined, dried over anhydrous sodium sulfate, and filtered. The filtrate was concentrated under reduced pressure, and the residues were purified by silica gel column chromatography (mobile phase: petroleum ether/ethyl acetate = 5:1) to obtain the title compound as a yellow liquid 200 mg, yield: 50.0%.

### Step 6: Preparation of 2-(3-aminobenzo[5,6][1,4]dioxino[2,3-b]pyridin-2-yl)propan-2-ol (9)

2-(3-(Dibenzylamino)benzo[5,6][1,4]dioxino[2,3-*b*]pyridin-2-yl)propan-2-ol (9e) (100 mg, 0.228 mmol) was dissolved in THF (5 mL) at room temperature, followed by addition of Pd/C (20.0 mg), and the reaction solution was stirred under a hydrogen atmosphere for 6 hours. The reaction solution was filtered through Celite, and the filtrate was concentrated under reduced pressure. The residues were purified by preparative liquid chromatography (column model: Daisogei 30mm x 250mm, C18, 10 µm, 100A, mobile phase: acetonitrile/water, gradient: 25%-65%) to obtain 17.0 mg of the title compound as a white solid, yield: 28.9%.

LC-MS: m/z 259.19 [M + H]⁺.

¹H NMR (300 MHz, DMSO) *δ* 6.98-6.97 (m, 4H), 6.69 (s, 1H), 5.45 (s, 2H), 5.40 (s, 1H), 1.42 (s, 6H).

### Example 10: Preparation of 2-(3-aminobenzofuro[3,2-b]pyridin-2-yl)propan-2-ol (10)

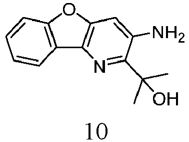

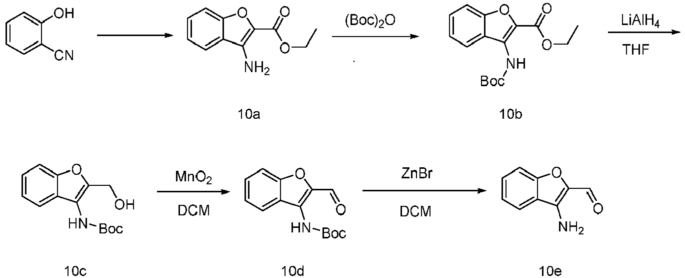

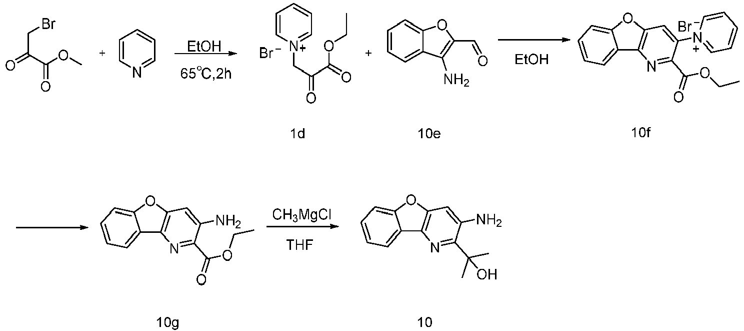

### Step 1: Preparation of ethyl 3-aminobenzofuran-2-carboxylate (10a)

2-Hydroxybenzonitrile (4.00 g, 33.6 mmol) and potassium carbonate (9.28 g, 67.2 mmol) were dissolved in DMF (60 mL) at room temperature, and the solution was stirred for 16 hours at 80°C, followed by addition of 100 mL of water. The reaction solution was extracted with 100 mL of ethyl acetate, and the aqueous phase was extracted twice with 100 mL of ethyl acetate. The organic phases were combined, dried over anhydrous sodium sulfate, and filtered. The filtration was concentrated under reduced pressure, and the residues were purified by silica gel column chromatography (mobile phase: petroleum ether: ethyl acetate = 8:1) to obtain 4.00 g of the title compound as a white solid, yield: 58.0%.

### Step 2: Preparation of ethyl 3-((tert-butoxycarbonyl)amino)benzofuran-2-carboxylate (10b)

Ethyl 3-aminobenzofuran-2-carboxylate (10a) (4.00 g, 19.5 mmol) and TEA (5.91 g, 58.5 mmol) were added to DCM (60 mL) at 0°C, followed by addition ofand then di-*tert*-butyl dicarbonate (6.38 g, 29.3 mmol) was added in batches. The reaction solution was stirred overnight at room temperature and poured into 100 mL of water. The organic phase was separated, and the aqueous phase was extracted twice with 100 mL of dichloromethane. The organic phases were combined, dried over anhydrous sodium sulfate, and filtered. The filtrate was concentrated under reduced pressure, and the residues were purified by silica gel column chromatography (mobile phase: petroleum ether: ethyl acetate = 4:1) to obtain 4.00 g of the title compound as a white solid, yield: 67.2%.

### Step 3: Preparation of tert-butyl (2-(hydroxymethyl)benzofuran-3-yl)carbamate (10c)

Ethyl 3-((*tert*-Butoxycarbonyl)amino)benzofuran-2-carboxylate (10b) (2.00 g, 6.56 mmol) was dissolved in THF (30 mL) at 0°C, followed by the dropwise addition of lithium tetrahydrogen (9.84mL, 9.84 mmol, 1M), and the reaction solution was stirred for 3 hours at room temperature. 10 mL of water was added dropwise to quench the reaction, followed by addition of 50 mL of water. The reaction solution was extracted with 50 mL of dichloromethane, and the aqueous phase was extracted twice with 50 mL of dichloromethane. The organic phases were combined, dried over anhydrous sodium sulfate, and filtered. The filtrate was concentrated under reduced pressure, and the residues were purified by silica gel column chromatography (mobile phase: ethyl acetate) to obtain 1.50 g of the title compound as a white solid, yield: 87.0%.

### Step 4: Preparation of tert-butyl (2-formylbenzofuran-3-yl)carbamate (10d)

*tert*-Butyl (2-(hydroxymethyl)benzofuran-3-yl)carbamate (10c) (3.80 g, 14.5 mmol) was dissolved in DCM (100 mL) at 0°C, followed by addition of manganese dioxide (25.1 g, 289 mmol), and the reaction solution was stirred for 24 hours at room temperature. The reaction solution was filtered through Celite, and the filtrate was concentrated under reduced pressure. The residues were purified by silica gel column chromatography (mobile phase: petroleum ether/ethyl acetate = 5:1) to obtain 2.5 g of the title compound as a yellow solid, yield: 66.3%.

### Step 5: Preparation of 3-aminobenzofuran-2-carbaldehyde (10e)

*tert*-Butyl (2-formylbenzofuran-3-yl)carbamate (10d) (500 mg, 1.92 mmol) was dissolved in DCM (30 mL) at 0°C, followed by addition of zinc bromide (862 mg, 3.83 mmol) at 0°C. The reaction solution was stirred for 2.5 hours at room temperature, followed by addition of 50 mL of water. The reaction solution was extracted with 50 mL of dichloromethane, and the aqueous phase was extracted twice with 50 mL of dichloromethane. The organic phases were combined, dried over anhydrous sodium sulfate, and filtered. The filtrate was concentrated under reduced pressure, and the residues were purified by silica gel column chromatography (mobile phase: petroleum ether/ethyl acetate = 2:1) to obtain 30 mg of the title compound as a yellow solid, yield: 9.72%.

### Step 6: Preparation of 1-(3-ethoxy-2,3-dioxopropyl)pyridin-1-ium bromide (1d)

Pyridine (108 mg, 1.36 mmol) was dissolved in EtOH (10 mL) at room temperature, followed by addition of ethyl 3-bromo-2-oxopropionate (242 mg, 1.24 mmol) under a nitrogen atmosphere. The reaction solution was stirred at 65°C for 2 hours, and directly used for the next step.

### Step 7: Preparation of 1-(2-(ethoxycarbonyl)benzofuro[3,2-b]pyridin-3-yl)pyridin-1-ium bromide (10f)

The reaction solution of step 6 was cooled to 18-22°C, followed by addition of 3-aminobenzofuran-2-carbaldehyde (10e) (180 mg, 1.12 mmol) and pyridine (225 mg, 2.85 mmol). The reaction solution was stirred overnight at 80°C under a nitrogen atmosphere, and directly used for the next step.

### Step 8: Preparation of ethyl 3-aminobenzofuro[3,2-b]pyridine-2-carboxylate (10g)

The reaction solution of step 7 was cooled to 70°C, followed by addition of morpholine (270 mg, 3.10 mmol). The reaction solution was heated to 80°C, and stirred at 80°C for 18 hours under a nitrogen atmosphere. followed by addition of50 mL of water was added. The reaction solution was extracted with 50 mL of dichloromethane, and the aqueous phase was extracted twice with 50 mL of dichloromethane. The organic phases were combined, dried over anhydrous sodium sulfate, and filtered. The filtrate was concentrated under reduced pressure, and the residues were purified by silica gel column chromatography (mobile phase: dichloromethane/methanol = 20:1) to obtain 140 mg of the title compound as a yellow solid, yield: 32.4%.

### Step 9: Preparation of 2-(3-aminobenzofuro[3,2-b]pyridin-2-yl)propan-2-ol (10)

Methylmagnesium chloride (3 N, 0.78 mL, 2.34 mmol) was added to THF (10 mL) at 0°C under a nitrogen atmosphere, followed by addition of ethyl 3-aminobenzofuro[3,2-*b*]pyridine-2-carboxylate (15 g) (50.0 mg, 0.195 mmol) dissolved in THF (10 mL). The reaction solution was stirred at room temperature for 24 hours, followed by addition of 50 mL of water. The reaction solution was extracted with 50 mL of dichloromethane, and the aqueous phase was extracted twice with 50 mL of dichloromethane. The organic phases were combined, dried over anhydrous sodium sulfate, and filtered. The filtrate was concentrated under reduced pressure, and the residues were purified by preparative liquid chromatography (column model: Daisogei 30mm x 250mm, C18, 10 µm, 220nm, 100A, mobile phase: acetonitrile/water, gradient: 57%) to obtain 13 mg of the title product as a yellow solid, yield: 27.5%.

LC-MS: m/z 242.9 [M + H]⁺.

1H NMR (300 MHz, CDCl₃) δ 8.03 (d, J = 8.1 Hz, 1 H), 7.97 (d, J = 8.1 Hz, 1H), 7.42-7.31(m, 2H), 7.26 (s, 1H), 7.05 (s, 1H), 4.73 (s, 2H), 3.25 (s, 1H), 1.77 (s, 6H).

### Example 11: Preparation of 2-(3-amino-5-methyl-5H-pyrido[3,2-b]indol-2-yl)propan-2-ol (11)

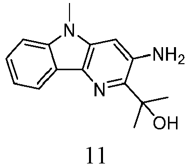

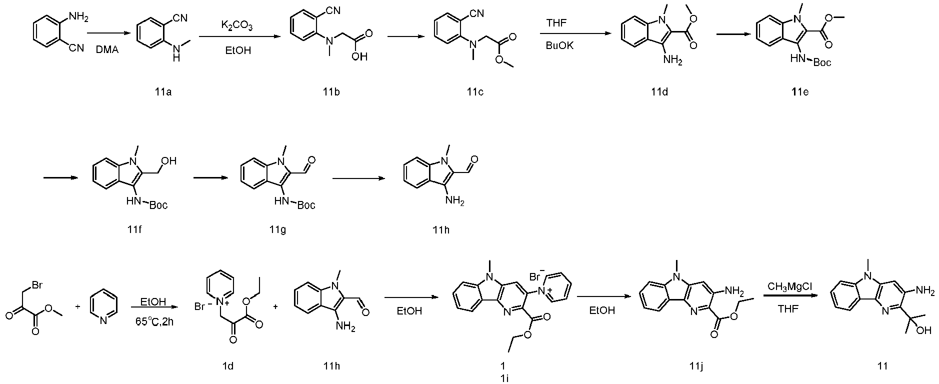

### Step 1: Preparation of 2-(methylamino)benzonitrile (11a)

2-Aminobenzonitrile (5.00 g, 42.3 mmol), dimethyl oxalate (7.50 g, 63.6 mmol) and potassium *tert*-butoxide (5.93 g, 53.0 mmol) were dissolved in DMA (30 mL) at room temperature. The reaction solution was stirred at 130°C for 18 hours, followed by addition of 100 mL of water. The reaction solution was extracted with 100 mL of ethyl acetate, and the aqueous phase was extracted twice with 100 mL of ethyl acetate. The organic phases were combined, dried over anhydrous sodium sulfate, and filtered. The filtrate was concentrated under reduced pressure, and the residues were purified by silica gel column chromatography (mobile phase: petroleum ether: ethyl acetate = 5:1) to obtain 4.00 g of the title product as a white solid, yield: 71.5%.

### Step 2: Preparation of N-(2-cyanophenyl)-N-methylglycine (11b)

2-(Methylamino)benzonitrile (11a) (3.50 g, 26.5 mmol), potassium carbonate (3.84 g, 27.8 mmol) and ethyl bromoacetate (14.9 g, 89.6 mmol) were added to ethanol (35 mL) and water (35 mL) at room temperature. The reaction solution was stirred at 78°C for 48 hours, poured into 100 mL of water, followed by addition of 100 mL of dichloromethane. The organic phase was separated, and the aqueous phase was extracted twice with 100 mL of dichloromethane. The organic phases were combined, dried over anhydrous sodium sulfate, and filtered. The filtrate was concentrated under reduced pressure, and the residues were purified by silica gel column chromatography (mobile phase: ethyl acetate) to obtain 2.90 g of the title product as a yellow liquid, yield: 57.6%.

### Step 3: Preparation of methyl N-(2-cyanophenyl)-N-methylglycinate (11c)

N-(2-cyanophenyl)-*N*-methylglycine (11b) (1.00 g, 5.26 mmol), potassium carbonate (1.45 g, 10.5 mmol) and methyl iodide (2.24 g, 15.8 mmol) were dissolved in DMF (30 mL) at 0°C. The reaction solution was stirred at room temperature for 3 hours, followed by addition of 50 mL of water. The reaction solution was extracted with 50 mL of dichloromethane, and the aqueous phase was extracted twice with 50 mL of dichloromethane. The organic phases were combined, dried over anhydrous sodium sulfate, and filtered. The filtrate was concentrated under reduced pressure, and the residues were purified by silica gel column chromatography (mobile phase: petroleum ether: ethyl acetate = 4:1) to obtain the title product as a white solid 0.90 g, yield: 83.8%.

### Step 4: Preparation of methyl 3-amino-1-methyl-1H-indole-2-carboxylate (11d)

Methyl *N*-(2-cyanophenyl)-*N*-methylglycinate (11c) (5.00 g, 22.9 mmol) was dissolved in THF (100 mL) at 0°C, followed by addition of potassium *tert*-butoxide (2.82 g, 25.2 mmol). The reaction solution was stirred for 24 hours at room temperature, and filtered through Celite. The filtrate was concentrated under reduced pressure, and the residues were purified by silica gel column chromatography (mobile phase: petroleum ether/ethyl acetate = 2/1) to obtain 2.5 g of the title product as a yellow solid, yield: 50.0%.

### Step 5: Preparation of methyl 3-((tert-butoxycarbonyl)amino)-1-methyl-1H-indole-2-carboxylate (11e)

Methyl 3-amino-1-methyl-1*H*-indole-2-carboxylate (11d) (3.00 g, 13.7 mmol), TEA (4.15 g, 41.1 mmol) and DMAP (1.67 g, 13.7 mmol) were dissolved in DCM (100 mL) at 0°C, followed by addition of Boc anhydride (4.48 g, 20.5 mmol) at 0°C. The reaction solution was stirred at 40°C for 16 hours, followed by addition of 50 mL of water. The reaction solution was extracted with 50 mL of dichloromethane, and the aqueous phase was extracted twice with 50 mL of dichloromethane. The organic phases were combined, dried over anhydrous sodium sulfate, and filtered. The filtrate was concentrated under reduced pressure, and the residues were purified by silica gel column chromatography (mobile phase: petroleum ether/ethyl acetate = 2/1) to obtain 1.50 g of the title product as a yellow solid, yield: 33.5%.

### Step 6: Preparation of tert-butyl (2-(hydroxymethyl)-1-methyl-1H-indol-3-yl)carbamate (11f)

Methyl 3-((tert-butoxycarbonyl)amino)-1-methyl-1*H*-indole-2-carboxylate (11e) (500 mg, 1.64 mmol) was dissolved in THF (100 mL) at 0°C, and lithium tetrahydrogen aluminum (93.8 mg, 2.47 mmol) was added at 0°C. The reaction solution was stirred at room temperature for 2.5 hours, followed by addition of 50 mL of water. The reaction solution was extracted with 50 mL of dichloromethane, and the aqueous phase was extracted twice with 50 mL of dichloromethane. The organic phases were combined, dried over anhydrous sodium sulfate, and filtered. The filtrate was concentrated under reduced pressure, and the residues were purified by silica gel column chromatography (mobile phase: petroleum ether/ethyl acetate = 1/1) to obtain 250 mg of the title product as a yellow solid, yield: 55.1%.

### Step 7: Preparation of tert-butyl (2-formyl-1-methyl-1H-indol-3-yl)carbamate (11g)

*tert*-Butyl (2-(hydroxymethyl)-1-methyl-1*H*-indol-3-yl)carbamate (11f) (200 g, 0.760 mmol) was dissolved in DCM (100 mL) at 0°C, followed by addition of manganese dioxide (2.51 g, 28.9 mmol). The reaction solution was stirred for 24 hours at room temperature, and filtered through Celite. The filtrate was concentrated under reduced pressure, and the residues were purified by silica gel column chromatography (mobile phase: petroleum ether/ethyl acetate = 5:1) to obtain 180 mg of the title compound as a yellow solid, yield: 66.3%.

### Step 8: Preparation of 3-amino-1-methyl-1H-indole-2-carbaldehyde (11h)

*tert-Butyl* (2-formyl-1-methyl-1*H*-indol-3-yl)carbamate (11g) (500 mg, 1.81 mmol) was dissolved in DCM (30 mL) at 0°C, and zinc bromide (815 mg, 3.62 mmol) was added at 0°C. The reaction solution was stirred for 6 hours at room temperature, followed by addition of 50 mL of water. The reaction solution was extracted with 50 mL of dichloromethane, and the aqueous phase was extracted twice with 50 mL of dichloromethane. The organic phases were combined, dried over anhydrous sodium sulfate, and filtered. The filtrate was concentrated under reduced pressure, and the residues were purified by silica gel column chromatography (mobile phase: petroleum ether/ethyl acetate = 2/1) to obtain 80 mg of the title product as a yellow solid, yield: 16.1%.

### Step 9: Preparation of 1-(3-ethoxy-2,3-dioxopropyl)pyridin-1-ium bromide (1d)

Pyridine (108 mg, 1.36 mmol) was dissolved in EtOH (10 mL) at room temperature, and ethyl 3-bromo-2-oxopropionate (242 mg, 1.24 mmol) was added under a nitrogen atmosphere. The reaction solution was stirred at 65°C for 2 hours, and directly used for the next step.

### Step 10: Preparation of 1-(2-(ethoxycarbonyl)-5-methyl-5H-pyrido[3,2-b]indol-3-yl)pyridin-1-ium bromide (11i)

The reaction solution of step 9 was cooled to 18-22°C, followed by addition of 3-amino-1-methyl-1*H*-indole-2-carbaldehyde (11h) (180 mg, 1.12 mmol) and pyridine (225 mg, 2.85 mmol). The reaction solution was stirred overnight at 80°C under a nitrogen atmosphere, and directly used for the next step.

### Step 11: Preparation of ethyl 3-amino-5-methyl-5H-pyrido[3,2-b]indole-2-carboxylate (11i)

The reaction solution of step 10 was cooled to 70°C, followed by addition of morpholine (270 mg, 3.10 mmol). The reaction solution was heated to 80°C, and stirred at 80°C for 18 hours under a nitrogen atmosphere. 50 mL of water was added. The reaction solution was extracted with 50 mL of dichloromethane, and the aqueous phase was extracted twice with 50 mL of dichloromethane. The organic phases were combined, dried over anhydrous sodium sulfate, and filtered. The filtrate was concentrated under reduced pressure, and the residues were purified by silica gel column chromatography (mobile phase: dichloromethane/methanol = 20:1) to obtain 140 mg of the title compound as a yellow solid, yield: 32.4%.

### Step 12: Preparation of 2-(3-amino-5-methyl-5H-pyrido[3,2-b]indol-2-yl)propan-2-ol (11)

Methylmagnesium chloride (3 N, 0.78 mL, 2.34 mmol) was added to THF (10 mL) at 0°C under a nitrogen atmosphere, and ethyl 3-amino-5-methyl-SH-pyrido[3,2-b]indole-2-carboxylate (11i) (50.0 mg, 0.186 mmol) dissolved in THF (10 mL) was added. The reaction solution was stirred at room temperature for 4 hours, followed by addition of 50 mL of water. The reaction solution was extracted with 50 mL of dichloromethane, and the aqueous phase was extracted twice with 50 mL of dichloromethane. The organic phases were combined, dried over anhydrous sodium sulfate, and filtered. The filtrate was concentrated under reduced pressure, and the residues were purified by preparative liquid chromatography (column model: Daisogei 30mm x 250mm, C18, 10 µm, 220nm, 100A, mobile phase: acetonitrile/water, gradient: 57%) to obtain 17 mg of the title product as a yellow solid, yield: 35.9%.

LC-MS: m/z 256.10 [M+H]⁺.

¹H NMR (400 MHz, DMSO) *δ* 7.92 (d, J = 8.0 Hz,1 H), 7.50 (d, J = 8.0 Hz, 1H), 7.34(t, J = 6.4 Hz,1 H), 7. 14(t, J = 6.4 Hz,1 H), 7.03 (s, 1H), 5.80 (s, 1H), 5.51(s, 2H), 3.73(s, 3H), 1.63 (s, 6H).

### Example 12: Preparation of 2-(3-amino-7-chloro-9-methyl-9H-pyrido[2,3-b]indol-2-yl)propan-2-ol (12)

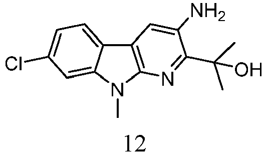

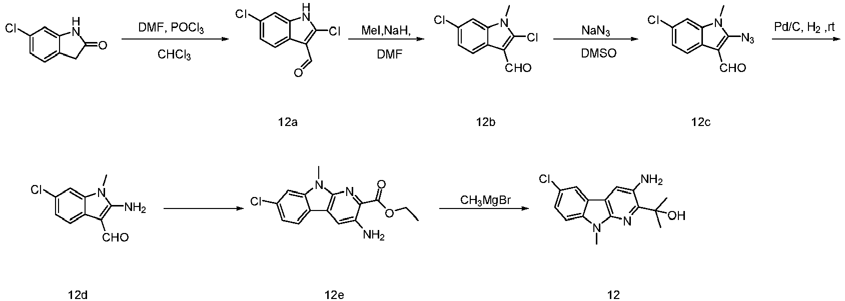

### Step 1: Preparation of 2,6-dichloro-1H-indole-3-carbaldehyde (12a)

*N,N-*Dimethylformamide (13.1 g, 180 mmol) was dissolved in chloroform (100 mL) at room temperature. The solution was cooled to 0°C, and phosphorus oxychloride (22.6 g, 147 mmol) was added dropwise. The reaction was stirred at 0-5°C for 10 minutes under a nitrogen atmosphere. A suspension of 6-chloroindolin-2-one (10.0 g, 59.9 mmol) in chloroform (100 mL) was slowly added dropwise at 0°C, and then the reaction system was stirred under refulx for 1 hours. The reaction solution was concentrated under reduced pressure, followed by addition of water. The pH value was adjusted to 5 with potassium acetate, and then the pH value was adjusted to 7 with aqueous sodium hydroxide solution (2N). The reaction solution was extracted with dichloromethane, and the organic phase was dried over anhydrous sodium sulfate and filtered. The filtrate was concentrated under reduced pressure, and the residues were purified by silica gel column chromatography (mobile phase: PE/EA = 1: 1) to obtain 4.60 g of the title compound as a white solid, yield: 36.1%.

LCMS: m/z 213.8 [M + H]⁺.

### Step 2: Preparation of 2,6-dichloro-1-methyl-1H-indole-3-carbaldehyde (12b)

2,6-Dichloro-1*H*-indole-3-carbaldehyde (12a) (4.60 g, 21.6 mmol) and methyl iodide (3.68 g, 25.9 mmol) were added to N,N-dimethylformamide (60 mL) at room temperature. The reaction system was cooled to 0°C, and sodium hydride (950 mg, 23.8 mmol) was added in batches. The reaction solution was heated to room temperature, stirred for 4 hours, and quenched with water. The reaction solution was extracted with ethyl acetate. The organic phase was washed three times with saturated brine, dried over anhydrous sodium sulfate, and filtered. The filtrate was concentrated under reduced pressure, and the result residues were purified by silica gel column chromatography (mobile phase: EA/PE = 1: 1) to obtain 4.40 g of the title compound as a brown solid, yield: 87.8%.

LCMS: m/z 227.8 [M + H]⁺.

### Step 3: Preparation of 2-azido-6-chloro-1-methyl-1H-indole-3-carbaldehyde (12c)

2,6-Dichloro-1-methyl-1*H*-indole-3-carbaldehyde (12b) (4.40 g, 19.4 mmol) was added to dimethyl sulfoxide (45 mL) at room temperature, followed by addition of sodium azide (2.44 g, 38.8 mmol). The reaction solution was stirred at 20°C for 7 hours. Water (50 mL) was added, and the mixture was extracted with ethyl acetate (50 mL). The organic phase was dried over anhydrous sodium sulfate and filtered. The filtrate was concentrated under reduced pressure to obtain 3.80 g of the crude title compound as a brown solid, which was used directly for the next step.

LCMS: m/z 234.9 [M + H]⁺.

### Step 4: Preparation of 2-amino-6-chloro-1-methyl-1H-indole-3-carbaldehyde (12d)

2-Azido-6-chloro-1-methyl-1*H*-indole-3-carbaldehyde (12c) (3.80 g, 16.0 mmol), palladium on carbon (570 mg, 30% wt) and methanol (80 mL) were mixed at room temperature. The reaction system was purged with hydrogen three times, and then stirred under a hydrogen atmosphere for 18 hours. After the reaction was completed by LCMS monitoring, the reaction solution was filtered, and the filtrate was concentrated under reduced pressure. The residues were purified by silica gel column chromatography (mobile phase: EA/PE = 1:1) to obtain 1.70 g of the title compound as a brownish-yellow solid, yield: 50.3%.

LCMS: m/z 208.9 [M + H]⁺.

### Step 5: Preparation of ethyl 3-amino-7-chloro-9-methyl-9H-pyrido[2,3-b]indole-2-carboxylate (12e)

Pyridine (500 mg, 6.33 mmol) was added to anhydrous ethanol (10 mL) at room temperature, and a solution of ethyl 3-bromo-2-carbonylpropanoate (1.05 g, 5.39 mmol) in anhydrous ethanol (10 mL) was added dropwise. The reaction solution was heated to 65°C and stirred for 1 hour. The reaction solution was cooled to room temperature, followed by addition of compound 2-amino-6-chloro-1-methyl-1H-indole-3-carbaldehyde (12d) (940 mg, 4.52 mmol), pyridine (928 mg, 11.7 mmol) and anhydrous ethanol (10 mL). The reaction solution was heated to 85°C and stirred for 18 hours. Pyrrolidine (835 mg, 11.75 mmol) was added, and the reaction solution was stirred for 3 hours at 85°C. After the reaction was completed by LCMS monitoring, the reaction solution was cooled and concentrated under reduced pressure. The residues were purified by silica gel column chromatography (mobile phase: PE/EA = 2:1) to obtain 620 mg of the title compound as a reddish-brown solid, yield: 45.3%.

LCMS: m/z 303.9 [M + H]⁺.

### Step 6: Preparation of 2-(3-amino-7-chloro-9-methyl-9H-pyrido[2,3-b]indol-2-yl)propan-2-ol (12)

Methylmagnesium bromide (3.33 mL, 3 M in Et₂O, 10.0 mmol) was dissolved in tetrahydrofuran (30 mL) at room temperature. The solution was cooled to -5°C, and a solution of ethyl 3-amino-7-chloro-9-methyl-9*H*-pyrido[2,3-*b*]indole-2-carboxylate (12e) (303 mg, 1.00 mmol) in tetrahydrofuran (30 mL) was added dropwise. The reaction was stirred at -5°C for 2 hours. After the reaction was completed by LCMS monitoring, saturated aqueous solution of ammonium chloride (20 mL) was added, and the reaction solution was extracted with ethyl acetate (50 mL). The organic phase was dried over anhydrous sodium sulfate and filtered, and the filtrate was concentrated under reduced pressure. The residues were purified by silica gel column chromatography (mobile phase: EA/PE = 1:1) to obtain 147 mg of the title compound as a white solid, yield: 48.8%.

LCMS: m/z 289.9 [M + H]⁺.

¹H NMR (400 MHz, CD₃OD) *δ* 7.92 (d, *J* = 8.0 Hz, 1H), 7.74 (s, 1H), 7.46 (s, 1H), 7.13 (dd, *J* = 8.3 Hz, 1.8 Hz, 1H), 3.83 (s, 3H), 1.73 (s, 6H).

### Example 13: Preparation of 2-(3-amino-6-chloro-9-methyl-9H-pyrido[2,3-b]indol-2-yl)propan-2-ol (13)

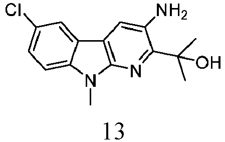

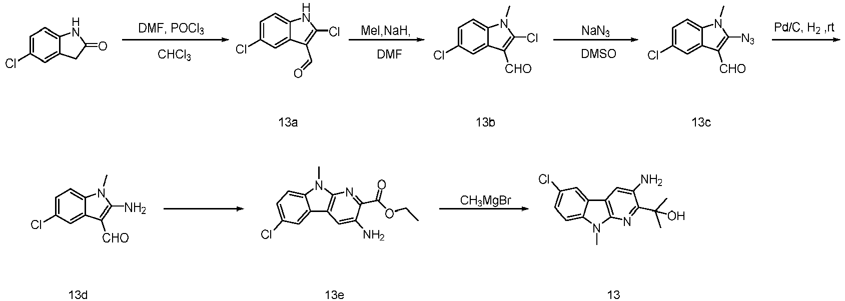

### Step 1: Preparation of 2,5-dichloro-1H-indole-3-carbaldehyde (13a)

*N*,*N-*Dimethylformamide (13.1 g, 180 mmol) was dissolved in chloroform (100 mL) at room temperature. The solution was cooled to 0°C,and phosphorus oxychloride (22.6 g, 147 mmol) was added slowly. The reaction was stirred for 10 minutes at 0-5°C under a nitrogen atmosphere. A suspension of 5-chloroindolin-2-one (10.0 g, 59.9 mmol) in chloroform (100 mL) was slowly added dropwise at 0°C, and then the reaction system was refluxed for 1 hours. The reaction solution was concentrated under reduced pressure, followed by addition of water. The pH value was adjusted to 7 with potassium carbonate. The reaction solution was extracted with ethyl acetate, and the organic phase was dried over anhydrous sodium sulfate and filtered. The filtrate was concentrated under reduced pressure, and the residues were purified by silica gel column chromatography (mobile phase: EA/PE = 2:1) to obtain 4.10 g of the title compound as a white solid, yield: 32.1%.

LCMS: m/z 213.7 [M + H]⁺.

### Step 2: Preparation of 2,5-dichloro-1-methyl-1H-indole-3-carbaldehyde (13b)

2,5-Dichloro-1*H*-indole-3-carbaldehyde (2.00 g, 8.41 mmol) and anhydrous potassium carbonate (1.95 g, 14.1 mmol) were dissolved in *N,N-*dimethylformamide (20 mL) at room temperature, and methane iodide (1.45 g, 10.3 mmol) was added slowly. The reaction solution was stirred at room temperature for 18 hours, and quenched with water. The reaction solution was extracted with ethyl acetate. The organic phase was washed three times with saturated brine, dried over anhydrous sodium sulfate, and filtered. The filtrate was concentrated under reduced pressure, and the result residues were purified by silica gel column chromatography (mobile phase: EA/PE = 1:5) to obtain 1.70 g of the title compound as a yellow solid, yield: 79.8%.

LCMS: m/z 227.8 [M + H]⁺.

### Step 3: Preparation of 2-azido-5-chloro-1-methyl-1H-indole-3-carbaldehyde (13c)

2,5-Dichloro-1-methyl-1*H*-indole-3-carbaldehyde (13b) (1.50 g, 6.61 mmol) and dimethyl sulfoxide (20 mL) were mixed at room temperature, followed by addition of sodium azide (1.05 g, 16.7 mmol). The reaction solution was stirred at 20°C for 7 hours, followed by addition of water (50 mL), and extracted with ethyl acetate (50 mL). The organic phase was dried over anhydrous sodium sulfate and filtered. The filtrate was concentrated under reduced pressure to obtain 1.30 g of the crude title compound as a light yellow solid (yield: 83.8%), which was used directly for the next step.

LCMS: m/z 234.8 [M + H]⁺.

### Step 4: Preparation of 2-amino-5-chloro-1-methyl-1H-indole-3-carbaldehyde (13d)

2-Azido-5-chloro-1-methyl-1*H*-indole-3-carbaldehyde (13c) (1.30 g, 5.56 mmol) and palladium on carbon (400 mg, 30% wt) were added to methanol (15 mL) at room temperature. The reaction system was purged with hydrogen three times, and then stirred under a hydrogen atmosphere for 4 hours. After the reaction was completed by LCMS monitoring, the reaction solution was filtered, and the filtrate was concentrated under reduced pressure. The residues were purified by silica gel column chromatography (mobile phase: EA/PE = 1:2) to obtain the title compound as a brownish-yellow solid 900 mg, yield: 77.8%.

LCMS: m/z 208.9 [M + H]⁺.

### Step 5: Preparation of ethyl 3-amino-6-chloro-9-methyl-9H-pyrido[2,3-b]indole-2-carboxylate (13e)

Pyridine (500 mg, 6.33 mmol) was added to anhydrous ethanol (10 mL) at room temperature, and a solution of ethyl 3-bromo-2-carbonylpropanoate (1.05 g, 5.39 mmol) in anhydrous ethanol (10 mL) was added dropwise. The reaction solution was heated to 65°C and stirred for 1 hour. The reaction solution was cooled to room temperature, followed by addition of compound 2-amino-5-chloro-1-methyl-1*H*-indole-3-carbaldehyde (13d) (940 mg, 4.52 mmol), pyridine (928 mg, 11.7 mmol) and anhydrous ethanol (10 mL). The reaction solution was heated to 85°C and stirred for 18 hours. Pyrrolidine (835 mg, 11.75 mmol) was added, and the reaction solution was stirred for 3 hours at 85°C. After the reaction was completed by LCMS monitoring, the reaction solution was cooled and concentrated under reduced pressure. The residues were purified by silica gel column chromatography (mobile phase: EA/PE = 2:1) to obtain 150 mg of the title compound as a brownish-yellow solid, yield: 20.6%.

LCMS: m/z 303.9 [M + H]⁺.

### Step 6: Preparation of 2-(3-amino-6-chloro-9-methyl-9H-pyrido[2,3-b]indol-2-yl)propan-2-o (13)

Methylmagnesium bromide (0.7 mL, 3 M in Et₂O, 2.1 mmol) was dissolved in tetrahydrofuran (10 mL) at 0°C. The solution was cooled to -5°C, and a solution of ethyl 3-amino-6-chloro-9-methyl-9*H*-pyrido[2,3-*b*]indole-2-carboxylate (13e) (303 mg, 1.00 mmol) in tetrahydrofuran (30 mL) was added dropwise. The reaction was stirred at -5°C for 3 hours. Saturated aqueous ammonium chloride solution (20 mL) was added, and the reaction solution was extracted with ethyl acetate (50 mL). The organic phase was dried over anhydrous sodium sulfate and filtered, and the filtrate was concentrated under reduced pressure. The residues were purified by silica gel column chromatography (mobile phase: EA/PE = 1:1) to obtain 11.1 mg of the title compound as a white solid, yield: 19.2%.

LCMS: m/z 289.9 [M + H]⁺.

¹H NMR (400 MHz, DMSO-d₆) *δ* 8.08 (d, *J* = 2.0Hz, 1H), 7.71 (s, 1H), 7.43 (s, 1H), 7.13 (dd, *J* = 8.8 Hz, 2.0 Hz, 1H), 5.56 (s, 1H), 5.35 (s, 1H), 3.80 (s, 3H), 1.63 (s, 6H).

### Example 14: Preparation of 2-(2-amino-9,9-dimethyl-9H-fluoren-3-yl)propan-2-ol (14)

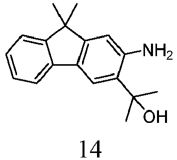

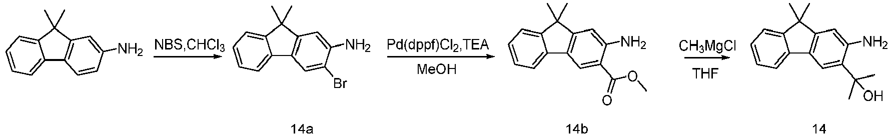

### Step 1: Preparation of 3-bromo-9,9-dimethyl-9H-fluoren-2-amine (14a)

9,9-Dimethyl-9*H*-fluoren-2-amine (3.00 g, 14.4 mmol) was dissolved in chloroform (30 mL) at room temperature, followed by addition of NBS (2.68 g, 15.1 mmol). The reaction solution was stirred for 16 hours at room temperature under a nitrogen atmosphere, and 50 mL of water was added. The reaction solution was extracted with 100 mL of ethyl acetate, and the aqueous phase was extracted twice with 30 mL of ethyl acetate. The organic phases were combined, dried over anhydrous sodium sulfate, and filtered. The filtrate was concentrated under reduced pressure, and the residues were purified by silica gel column chromatography (mobile phase: PE/EA=4: 1) to obtain 1.50 g of the title product as an orange solid, yield: 36.3%.

### Step 2: Preparation of methyl 2-amino-9,9-dimethyl-9H-fluorene-3-carboxylate (14b)

3-Bromo-9,9-dimethyl-9*H*-fluoren-2-amine (14a) (900mg, 3.14 mmol) was dissolved in methanol (17 mL) at room temperature, followed by addition of TEA (507 mg, 5.03 mmol) and Pd(dppf)Cl₂ (230 mg, 0.314 mmol). The reaction solution was stirred at 90°C for 16 hours under a CO atmosphere. The reaction solution was extracted with 30 mL of ethyl acetate, and the aqueous phase was extracted twice with 20 mL of ethyl acetate. The organic phases were combined, dried over anhydrous sodium sulfate, and filtered. The filtrate was concentrated under reduced pressure, and the residues were purified by silica gel column chromatography (mobile phase: PE/EA=5:1) to obtain 110 mg of the title product as a yellow oil, yield: 13.1%.

### Step 3: Preparation of 2-(2-amino-9,9-dimethyl-9H-fluoren-3-yl)propan-2-ol (14)

Methylmagnesium chloride (3 N, 0.62 mL, 1.87 mmol) was added dropwise to THF (10 mL) at 0°C under a nitrogen atmosphere, andmethyl 2-amino-9,9-dimethyl-9*H*-fluorene-3-carboxylate (14b) (50.0 mg, 0.187 mmol) dissolved in THF (10 mL) was added. The reaction solution was stirred at room temperature for 4 hours, and quenched with 25 mL of water. The reaction solution was extracted with 20 mL of ethyl acetate, and the aqueous phase was extracted again with 10 mL of ethyl acetate. The organic phases were combined, dried over anhydrous sodium sulfate, and filtered. The filtrate was concentrated under reduced pressure, and the residues were purified by preparative liquid chromatography (column model: Daisogei 30mm*250mm, C18, 10 µm, 100A, mobile phase: acetonitrile/water, gradient: 10%-50%) to obtain 16.0 mg of the title product as a white solid, yield: 32.1%.

LC-MS: m/z 267.7 [M+H]⁺

¹H NMR (300 MHz, DMSO-d6) *δ* 7.56~7.54(m,1 H), 7.42~7.37 (m,1 H), 7.35 (s,1 H), 7.21~7.16 (m,1 H), 7.10-7.05 (m,1 H), 6.69 (s,1H), 5.58 (s,2H), 5.22 (s,1H), 1.54 (s, 6H), 1.31 (s, 6H).

### Example 15: Preparation of 2-(8-aminobenzo[5,6][1,4]dioxino[2,3-b]pyridin-7-yl)propan-2-ol (15)

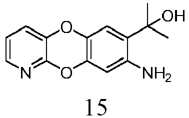

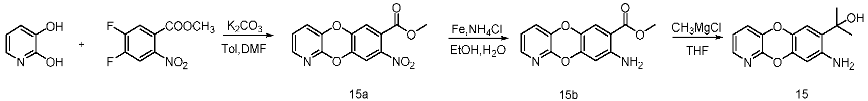

### Step 1: Preparation of methyl 8-nitrobenzo[5,6][1,4]dioxino[2,3-b]pyridine-7-carboxylate (15a)

Pyridine-2,3-diol (1.00 g, 9.01 mmol) was dissolved in toluene (4 mL) and DMF (20 mL) at room temperature, followed by addition of methyl 4,5-difluoro-2-nitrobenzoate (1.95 g, 9.01 mmol) and potassium carbonate (2.49 g, 18.0 mmol). The reaction solution was stirred at 120°C for 4 hours. The reaction solution was extracted with 40 mL of ethyl acetate, and the aqueous phase was extracted twice with 20 mL of ethyl acetate. The organic phases were combined, dried over anhydrous sodium sulfate, and filtered. The filtrate was concentrated under reduced pressure, and the residues were purified by silica gel column chromatography (mobile phase: DCM/EA=3: 1) to obtain 1.10 g of the title product as a yellow solid, yield: 42.4%.

### Step 2: Preparation of methyl 8-aminobenzo[5,6][1,4]dioxino[2,3-b]pyridine-7-carboxylate (15b)

Methyl 8-nitro-benzo[5,6][1,4]dioxino[2,3-*b*]pyridine-7-carboxylate (15a) (500 mg, 1.74 mmol) was dissolved in ethanol (20 mL) and water (2 mL) at room temperature, followed by addition of iron powder (535 mg, 9.55 mmol) and ammonium chloride (65.2 mg, 1.22 mmol). The reaction solution was stirred at 78°C for 16 hours. The reaction solution was extracted with 30 mL of ethyl acetate, and the aqueous phase was extracted twice with 20 mL of ethyl acetate. The organic phases were combined, dried over anhydrous sodium sulfate, and filtered. The filtrate was concentrated under reduced pressure, and the residues were purified by silica gel column chromatography (mobile phase: PE/EA= 1: 1) to obtain 250 mg of the title product as a light yellow solid, yield: 55.7%.

### Step 3: Preparation of 2-(8-aminobenzo[5,6][1,4]dioxino[2,3-b]pyridin-7-yl)propan-2-ol (15)

Methylmagnesium chloride (3 N, 1.74 mL, 5.23 mmol) was added dropwise to THF (10 mL) at 0°C under a nitrogen atmosphere, and methyl 8-aminobenzo[5,6][1,4]dioxino[2,3-*b*]pyridine-7-carboxylate (15b) (135 mg, 0.523 mmol) dissolved in THF (10 mL) was added. The reaction solution was stirred at room temperature for 3 hours, and quenched with 25 mL of water. The reaction solution was extracted with 20 mL of ethyl acetate, and the aqueous phase was extracted again with 10 mL of ethyl acetate. The organic phases were combined, dried over anhydrous sodium sulfate, and filtered. The filtrate was concentrated under reduced pressure, and the residues were purified by preparative liquid chromatography (column model: Daisogei 30mm*250mm, C18, 10 µm, 100A, mobile phase: acetonitrile 15%, 230/297 nm, 60 ml/min) to obtain 37.0 mg of the title product as a white solid, yield: 27.4%.

LC-MS: m/z259.19 [M+H]⁺.

¹H NMR (300 MHz, DMSO-d₆) *δ* 7.80~7.77(m,l H), 7.40~7.37 (d,1 H), 7.04~7.00 (t,1 H), 6.71 (m,1 H), 6.28 (m,1 H), 5.41 (m,2H), 5.26 (m,1H), 1.46 (s, 6H).

### Example 16: Preparation of 2-(3-aminodibenzo[b,d]furan-2-yl)propan-2-ol (16)

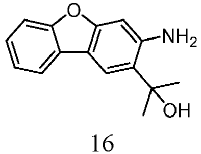

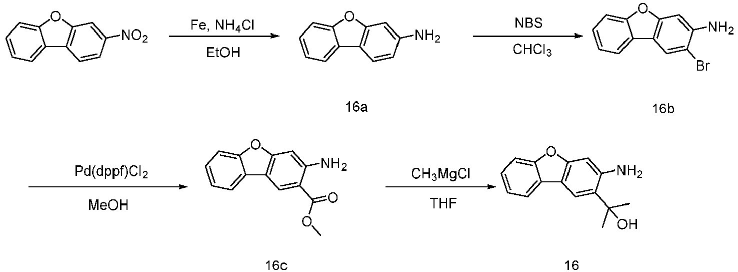

### Step 1: Preparation of dibenzo[b,d]furan-3-amine (16a)

3-Nitrodibenzo[*b*,*d*]furan (1.00 g, 4.69 mmol), Fe powder (2.63 g, 47.0 mmol), NH₄Cl (251 mg, 4.69 mmol) were dissolved in EtOH (20 mL) and H₂O (2 mL) at room temperature. The reaction solution was stirred overnight at 78°C. The reaction solution was filtered, and concentrated under reduced pressure. The residues were purified by silica gel column chromatography (mobile phase: petroleum ether/ethyl acetate = 5: 1) to obtain 800 mg of the title product as a yellow solid, yield: 93.1%.

### Step 2: Preparation of 2-bromo-dibenzo[b,d]furan-3-amine (16b)

Dibenzo[*b*,*d*]furan-3-amine (16a) (400 mg, 2.19 mmol) was dissolved in CHCl₃ (20 mL) at room temperature, followed by addition of NBS (389 mg, 2.19 mmol) at 0°C. The reaction solution was then stirred overnight at room temperature. The reaction solution was concentrated under reduced pressure, and the residues were purified by silica gel column chromatography (mobile phase: petroleum ether/ethyl acetate = 10:1) to obtain 400 mg of the title product as a yellow solid, yield: 69.8%.

### Step 3: Preparation of methyl 3-amino-dibenzo[b,d]furan-2-carboxylate (16c)

2-Bromo-dibenzo[*b*,*d*]furan-3-amine (16b) (300 mg, 1.15 mmol), TEA (185 mg, 1.83 mmol) and Pd(dppf)Cl₂ (83.8 g, 0.114 mmol) were dissolved in MeOH (10 mL) at room temperature. The reaction solution was stirred overnight at 90°C under a CO atmosphere. The reaction solution was concentrated under reduced pressure, and the residues were purified by silica gel column chromatography (mobile phase: petroleum ether/ethyl acetate = 10:1) to obtain 100 mg of the crude title product as a yellow solid, yield: 27.2%.

### Step 4: Preparation of 2-(3-aminodibenzo[b,d]furan-2-yl)propan-2-ol (16)

Methyl 3-aminodibenzo[*b*,*d*]furan-2-carboxylate (16c) (100 mg, 0.415 mmol) was dissolved in MeCN (100 mL) at room temperature, and CH₃MgCl was added dropwise (3 *N,* 0.91 mL) at 0°C. The reaction solution was then stirred overnight at room temperature, and 50 mL of water was added. The reaction solution was extracted with 50 mL of dichloromethane, and the aqueous phase was extracted again with 50 mL of dichloromethane. The organic phases were combined, dried over anhydrous sodium sulfate, and filtered. The filtrate was concentrated under reduced pressure, and the residues were purified by preparative liquid chromatography (column model: Daisogei 30mm*250mm, C18, 10 µm, 100A, mobile phase: acetonitrile/water, gradient: 25%-65%) to obtain 39.0 mg of the title product as a white solid, yield: 39.0%.

LC-MS: m/z 224.10 [M-OH]⁺.

¹H NMR (300 MHz, DMSO) *δ* 7.88-7.85 (m, 1H), 7.71 (s, 1H), 7.49-7.46 (m, 1H), 7.28-7.22 (m, 2H), 6.81 (s, 1H), 5.91 (s, 2H), 5.35 (s, 1H), 1.59 (s, 6H).

### Example 17: Preparation of 2-(6-aminobenzofuro[2,3-b]pyridin-7-yl)propan-2-ol (17)

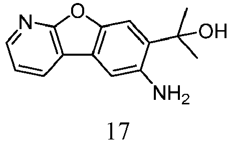

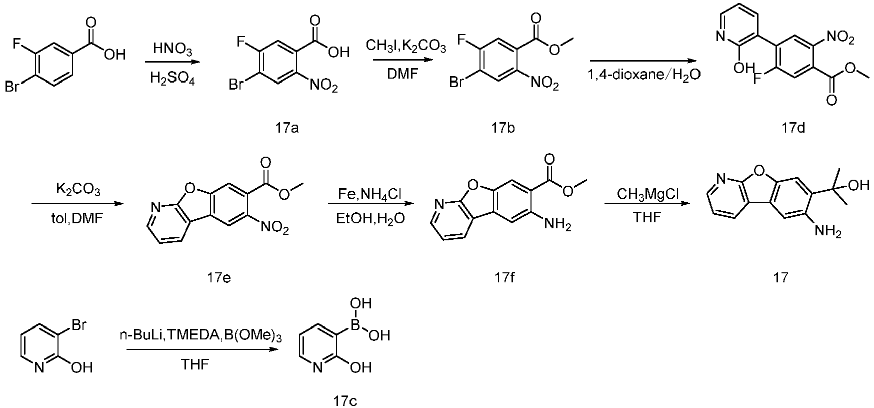

### Step 1: Preparation of 4-bromo-5-fluoro-2-nitrobenzoic acid (17a)

4-Bromo-3-fluorobenzoic acid (3.00 g, 13.7 mmol) was dissolved in concentrated sulfuric acid (20 mL) at room temperature, and a concentrated nitric acid (2.7 mL, 41.1 mmol) was added dropwise into the reaction flask in an ice bath. After the addition was completed, the reaction solution was stirred for 16 hours at room temperature. The reaction solution was poured into ice water to precipitate a solid which was filtered and dried to obtain 3.00 g of the title compound as a white solid, yield: 94.4%.

### Step 2: Preparation of methyl 4-bromo-5-fluoro-2-nitrobenzoate (17b)

4-Bromo-5-fluoro-2-nitrobenzoic acid (17a) (3.00 g, 12.9 mmol) was dissolved in DMF (20 mL) at room temperature, and methyl iodide (5.51 g, 38.8 mmol) and potassium carbonate (3.56 g, 25.8 mmol) were into the reaction flask. The reaction solution was stirred for 16 hours at room temperature. The reaction solution was extracted with 50 mL of dichloromethane and 50 mL of water, and the aqueous phase was extracted again with 20 mL of dichloromethane. The organic phases were combined, dried over anhydrous sodium sulfate, and filtered. The filtrate was concentrated under reduced pressureto obtain 2.60 g of the title compound as a yellow oil, yield: 72.8%.

### Step 3: Preparation of (2-hydroxypyridin-3-yl)boronic acid (17c)

3-Bromopyridine-2-ol (1.00 g, 5.75 mmol) was dissolved in THF (15 mL) at room temperature. Then the solution was cooled to -76°C under a nitrogen atmosphere, followed by addition of TMEDA (1.97 g, 17.0 mmol). The reaction solution was stirred for 15 minutes, followed by addition of n-BuLi (1.6 M in THF, 11 mL, 17.5 mmol) . The reaction solution was stirred for 15 minutes, and then raised to 0°C. Trimethyl borate (1.23 g, 11.8 mmol) was added, and the reaction was stirred for 15 minutes. After that, the reaction was stirred for 16 hours at room temperature. The reaction solution was cooled to 0°C, and a small amount of ice and 2 M hydrochloric acid (36 mL) were added. The mixture was concentrated under reduced pressure, and washed twice with 30 mL of dichloromethane. The aqueous phase was adjusted to pH = 5 with saturated aqueous NaOH solution to precipitate a solid. The reaction solution was cooled to 0°C, stirred for 10 minutes, and filtered. The filter cake was dried to obtain 460 mg of the title compound as a white solid, yield: 57.6%.

### Step 4: Preparation of methyl 5-fluoro-4-(2-hydroxypyridin-3-yl)-2-nitrobenzoate (17d)

Methyl 4-bromo-5-fluoro-2-nitrobenzoate (17b) (450 mg, 1.62 mmol), (2-hydroxypyridin-3-yl)boronic acid (17c) (338 mg, 2.43 mmol), sodium carbonate (344 mg, 3.24 mmol) and Pd(dppf)Cl₂ (59.3 mg, 0.0810 mmol) were dissolved in dioxane (25 mL) and water (1 mL) at room temperature. The reaction solution was stirred at 100°C for 16 hours under a nitrogen atmosphere. The organic phase was extracted with 30 mL of ethyl acetate and 20 mL of water, and the aqueous phase was extracted again with 20 mL of ethyl acetate. The organic phases were combined, dried over anhydrous sodium sulfate, and filtered. The filtrate was concentrated under reduced pressure, and the residues were purified by silica gel column chromatography (mobile phase: EA) to obtain 500 mg of the title compound as a white solid, yield: 70.5%.

### Step 5: Preparation of methyl 6-nitrobenzofuro[2,3-b]pyridine-7-carboxylate (17e)

Methyl 5-fluoro-4-(2-hydroxypyridin-3-yl)-2-nitrobenzoate (17d) (500 mg, 1.71 mmol) was dissolved in DMSO (15 mL) at room temperature, and a concentrated nitric acid (2.7 mL, 41.1 mmol) was added dropwise into the reaction flask in an ice bath. After the addition was completed, the reaction solution was stirred at 90°C for 16 hours. The organic phase was extracted with 40 mL of ethyl acetate and 30 mL of water, and the aqueous phase was extracted again with 40 mL of ethyl acetate. The organic phases were combined, dried over anhydrous sodium sulfate, and filtered. The filtrate was concentrated under reduced pressure, and the residues were purified by silica gel column chromatography (mobile phase: PE/EA = 1:1) to obtain 140 mg of the title compound as a light yellow solid, yield: 30.1%.

### Step 6: Preparation of methyl 6-aminobenzofuro[2,3-b]pyridine-7-carboxylate (17f)

Iron powder (136 mg, 2.43 mmol) and ammonium chloride (16.5 mg, 0.309 mmol) were added to a mixed solvent of ethanol (15 ml) and water (1.5 mL) at room temperature, followed by addition of methyl 6-nitrobenzofuro[2,3-b]pyridine-7-carboxylate (17e) (120 mg, 0.441 mmol). The reaction solution was heated to 78°C and stirred under reflux for 16 hours. The organic phase was extracted with 30 mL of ethyl acetate and 20 mL of water, and the aqueous phase was extracted again with 20 mL of ethyl acetate. The organic phases were combined, dried over anhydrous sodium sulfate, and filtered. The filtrate was concentrated under reduced pressure, and the residues were purified by silica gel column chromatography (mobile phase: PE/EA = 10:1-1:1) to obtain 100 mg of the title compound as a light yellow solid, yield: 93.7%.

### Step 7: Preparation of 2-(6-aminobenzofuro[2,3-b]pyridin-7-yl)propan-2-ol (17)

Methylmagnesium chloride (0.88 mL, 2.65 mmol) was dissolved in tetrahydrofuran (5 mL) at 0°C, and a solution of methyl 6-aminobenzofuro[2,3-b]pyridine-7-carboxylate (24f) (80 mg, 0.331 mmol) in tetrahydrofuran (10 mL) was added dropwise undera nitrogen atmosphere. After the addition was completed, the reaction solution was stirred for 3 hours at room temperature. After the reaction was completed, the reaction solution was quenched with water (5 mL), extracted with water (20 mL) and ethyl acetate (30 mL), and the aqueous phase was extracted again with 20 mL of ethyl acetate. The organic phases were combined and concentrated under reduced pressure. The residues were purified by preparative liquid chromatography (column model 30 mm * 250 mm, filler type: Daisogei C18, 10 µm, 100 Å, mobile phase: acetonitrile/water, gradient: 2-22min A15-65%, 60 mL/min) to obtain 23 mg of the title compound as a white solid, yield: 28.7%.

LC-MS: m/z 225.00 [M+H-18]⁺.

¹H NMR (300 MHz, DMSO-*d*₆) *δ* 8.40-8.31 (t, 2H), 7.40-7.34 (s, 2H), 7.24 (s, 1H), 5.52-5.43 (d, 3H), 1.58 (s, 6H).

### Example 18: Preparation of 2-(3-amino-9,9-dimethyl-9H-fluoren-2-yl)propan-2-ol (18)

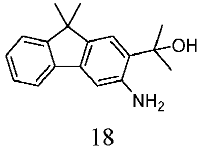

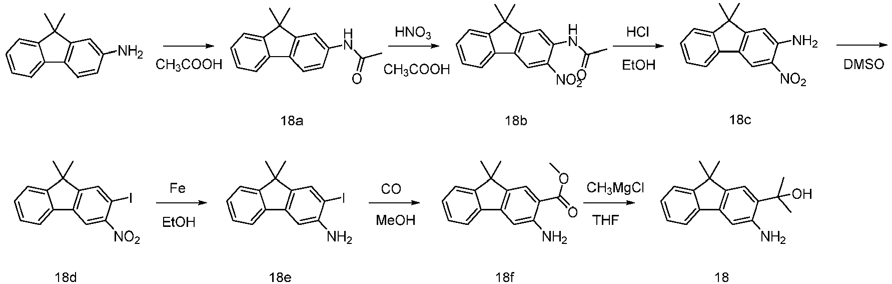

### Step 1: Preparation of N-(9,9-dimethyl-9H-fluoren-2-yl)acetamide (18a)

9,9-Dimethyl-9*H*-fluoren-2-amine (6.00 g, 28.7 mmol) was dissolved in acetic acid (30 mL) at room temperature, followed by the slow addition of acetic anhydride (5.86 g, 57.4 mmol). The reaction solution was stirred for 18 hours at room temperature, and then concentrated under reduced pressure. Ice water (100 mL) was added, and the mixture was filtered to obtain 4.50 g of the title compound as a white solid, yield: 74.9%.

### Step 2: Preparation of N-(9,9-dimethyl-3-nitro-9H-fluoren-2-yl)acetamide (18b)

*N*-(9,9-Dimethyl-9*H*-fluoren-2-yl)acetamide (18a) (1.00 g, 3.98 mmol) and concentrated sulfuric acid (0.2 mL) were added to acetic acid (15 mL) at room temperature, followed by addition of concentrated nitric acid (0.6 mL). The reaction solution was stirred for 2 hours at room temperature, cooled to room temperature, poured into 15 mL of ice water. The mixture was filtered to obtain 0.40g of the title compound as a yellow solid, yield: 34.7%.

### Step 3: Preparation of 9,9-dimethyl-3-nitro-9H-fluoren-2-amine (18c)

*N*-(9,9-Dimethyl-3-nitro-9*H*-fluoren-2-yl)acetamide (18b) (3.00 g, 10.3 mmol) and concentrated hydrochloric acid (10.2 mL) were dissolved in ethanol (50 mL) at room temperature. The reaction solution was stirred at 115°C for 3 hours, and cooled to room temperature. Water (100 mL) was added, and the mixture was extracted with ethyl acetate (100 mL). The organic phase was dried over anhydrous sodium sulfate and filtered, and the filtrate was concentrated under reduced pressure. The residues were purified by silica gel column chromatography (mobile phase: EA/PE = 5:1) to obtain 2.50 g of the title compound as a yellow solid, yield: 97.1%.

### Step 4: Preparation of 2-iodo-9,9-dimethyl-3-nitro-9H-fluorene (18d)

9,9-Dimethyl-3-nitro-9*H*-fluoren-2-amine (18c) (1.00 g, 3.94 mmol) and concentrated hydrochloric acid (0.5 mL) were dissolved in DMSO (10 mL) at room temperature, and sodium nitrite (285 mg, 4.13 mmol) was added at 0°C. The reaction solution was stirred for 2.5 hours, and aqueous potassium iodide (1.96 g, 11.8 mmol) solution was added. The reaction solution was stirred for 18 hours, followed by addition of water (100 mL), and extracted with ethyl acetate (100 mL). The organic phase was dried over anhydrous sodium sulfate and filtered, and the filtrate was concentrated under reduced pressure. The residues were purified by silica gel column chromatography (mobile phase: EA/PE = 5:1) to obtain 0.50 g of the title compound as a yellow solid, yield: 34.8%.

### Step 5: Preparation of 2-iodo-9,9-dimethyl-9H-fluoren-3-amine (18e)

2-Iodo-9,9-dimethyl-3-nitro-9*H*-fluorene (18d) (1.00 g, 2.74 mmol), iron powder (843 mg, 15.1 mmol) and ammonium chloride (104 mg, 1.92 mmol) were dissolved in ethanol (50 mL) and water (5 mL) at room temperature. The reaction solution was stirred at 78°C for 18 hours, cooled to room temperature, followed by addition of water (50 mL). The mixture was extracted with ethyl acetate (50 mL). The organic phase was dried over anhydrous sodium sulfate and filtered, and the filtrate was concentrated under reduced pressure. The residues were purified by silica gel column chromatography (mobile phase: EA/PE = 3:1) to obtain 0.60 g of the title compound as a yellow solid, yield: 65.3%.

### Step 6: Preparation of methyl 3-amino-9,9-dimethyl-9H-fluorene-2-carboxylate (18f)

2-Iodo-9,9-dimethyl-9*H*-fluoren-3-amine (18e) (300 mg, 0.895 mmol), TEA (181 mg, 1.79 mmol) and Pd(dppf)Cl₂ (83.8 g, 0.114 mmol) were dissolved in MeOH (10 mL) at room temperature. The reaction solution was stirred overnight at 90°C under a CO atmosphere. The reaction solution was concentrated under reduced pressure, and the residues were purified by silica gel column chromatography (mobile phase: petroleum ether/ethyl acetate = 4:1) to obtain 100 mg of the crude title product as a yellow solid, yield: 41.8%.

### Step 7: Preparation of 2-(3-amino-9,9-dimethyl-9H-fluoren-2-yl)propan-2-ol (18)

Methylmagnesium bromide (3.33 mL, 3 M in Et₂O, 10.0 mmol) was dissolved in tetrahydrofuran (30 mL) at room temperature. The solution was cooled to -5°C, and a solution methyl 3-amino-9,9-dimethyl-9*H*-fluorene-2-carboxylate (18f) (303 mg, 1.16 mmol) in tetrahydrofuran (30 mL) was added dropwise. The reaction was stirred at -5°C for 2 hours. After the reaction was completed by TLC monitoring, saturated aqueous ammonium chloride solution (20 mL) was added, and the reaction solution was extracted with ethyl acetate (50 mL). The organic phase was dried over anhydrous sodium sulfate, filtered, and the filtrate was concentrated under reduced pressure. The residues were purified by preparative liquid chromatography (column model: Daisogei 30mm*250mm, C18, 10 µm, 100A, mobile phase: acetonitrile/water, gradient: 10%-50%) to obatin 50.0 mg of the title product as a white solid, yield: 16.5%.

LCMS: m/z 250.10 [M + H]⁺.

¹H NMR (300 MHz, DMSO-*d₆*) 7.58~7.55(m, 1H), 7.46~7.43(m, 1H), 7.14~7.26(m, 2H), 7.15 (s, 1H), 6.98 (s, 1H), 5.43 (s, 2H), 5.25 (s, 1H), 1.54 (s, 6H), 1.35 (s, 6H).

### Example 19: Preparation of 2-(7-aminobenzo[5,6][1,4]dioxino[2,3-b]pyridin-8-yl)propan-2-ol (19)

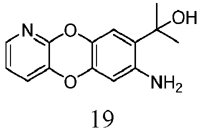

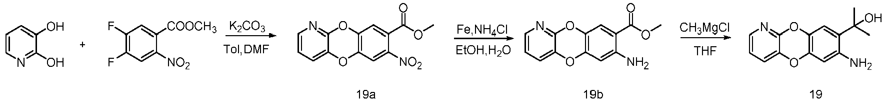

### Step 1: Preparation of methyl 7-nitrobenzo[5,6][1,4]dioxino[2,3-b]pyridine-8-carboxylate (19a)

Pyridine-2,3-diol (1.00 g, 9.01 mmol) was dissolved in toluene (4 mL) and DMF (20 mL) at room temperature, followed by addition of methyl 4,5-difluoro-2-nitrobenzoate (1.95 g, 9.01 mmol) and potassium carbonate (2.49 g, 18.0 mmol). Then the reaction solution was stirred at 120°C for 4 hours. The reaction solution was extracted with 40 mL of ethyl acetate, and the aqueous phase was extracted twice with 20 mL of ethyl acetate. The organic phases were combined, dried over anhydrous sodium sulfate, and filtered. The filtrate was concentrated under reduced pressure, and the residues were purified by silica gel column chromatography (mobile phase: DCM/EA=3:1) to obtain 1.10 g of the title product as a yellow solid, yield: 42.4%.

### Step 2: Preparation of methyl 7-aminobenzo[5,6][1,4]dioxino[2,3-b]pyridine-8-carboxylate (19b)

Methyl 7-nitrobenzo[5,6][1,4]dioxino[2,3-*b*]pyridine-8-carboxylate (19a) (500 mg, 1.74 mmol) was dissolved in ethanol (20 mL) and water (2 mL) at room temperature, and iron powder (535 mg, 9.55 mmol) and ammonium chloride (65.2 mg, 1.22 mmol) were added. The reaction solution was stirred at 78°C for 16 hours. The reaction solution was extracted with 30 mL of ethyl acetate, and the aqueous phase was extracted twice with 20 mL of ethyl acetate. The organic phases were combined, dried over anhydrous sodium sulfate, and filtered. The filtrate was concentrated under reduced pressure, and the residues were purified by silica gel column chromatography (mobile phase: PE/EA= 1:1) to obtain 250 mg of the title product as a light yellow solid, yield: 55.7%.

### Step 3: Preparation of 2-(7-aminobenzo[5,6][1,4]dioxino[2,3-b]pyridin-8-yl)propan-2-ol (19)

Methylmagnesium chloride (3 N, 1.74 mL, 5.23 mmol) was added dropwise to THF (10 mL) at 0°C in a nitrogen atmosphere, and methyl 7-aminobenzo[5,6][1,4]dioxino[2,3-*b*]pyridine-8-carboxylate (19b) (135 mg, 0.523 mmol) dissolved in THF (10 mL) was added. The reaction solution was stirred at room temperature for 3 hours, and quenched with 10 mL of water. 15 mL of water was added. The reaction solution was extracted with 20 mL of ethyl acetate, and the aqueous phase was extracted again with 10 mL of ethyl acetate. The organic phases were combined, dried over anhydrous sodium sulfate, and filtered. The filtrate was concentrated under reduced pressure, and the residues were purified by preparative liquid chromatography (column model: Daisogei 30mm*250mm, C18, 10 µm, 100A, mobile phase: acetonitrile 15%, 230/297 nm, 60ml/min) to obtain 56.0 mg of the title product as a white solid, yield: 41.5%.

LC-MS: m/z259.25 [M+H]⁺.

¹H NMR (300 MHz, DMSO-*d₆*) *δ* 7.78~7.76(m, 1H), 7.35~7.32 (d, 1H), 7.06~7.02 (t, 1H), 6.66 (m, 1H), 6.33 (m, 1H), 5.40 (m, 2H), 5.26 (m, 1H), 1.45 (s, 6H).

### Example 20: Preparation of 2-(3-amino-7-fluorodibenzo[b,e][1,4]dioxin-2-yl)propanol (20)

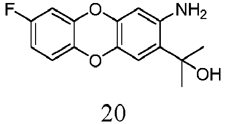

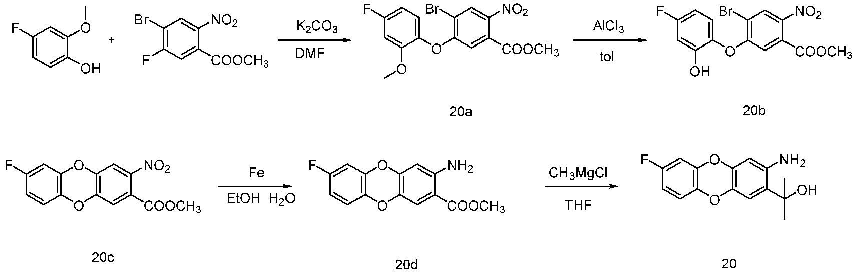

### Step 1: Preparation of methyl 4-bromo-5-(4-fluoro-2-methoxyphenoxy)-2-nitrobenzoate (20a)

Methyl 4-fluoro-2-methoxyphenol (2.05 g, 14.4 mmol) and 4-bromo-5-fluoro-2-nitrobenzoate and potassium carbonate (5.96 g, 43.2 mmol) were dissolved in DMF (40 mL) at room temperature. Then the reaction solution was stirred at 80°C for 16 hours. The reaction solution was extracted twice with 100 mL of ethyl acetate, and the aqueous phase was extracted again with 50 mL of ethyl acetate. The organic phases were combined, dried over anhydrous sodium sulfate, and filtered. The filtrate was concentrated under reduced pressure, and the residues were purified by silica gel column chromatography (mobile phase: PE/EA = 10:1) to obtain 1.90 g of the title compound as a yellow solid, yield: 92.6%.

### Step 2: Preparation of methyl 4-bromo-5-(4-fluoro-2-hydroxyphenoxy)-2-nitrobenzoate (20b)

Methyl 4-bromo-5-(4-fluoro-2-methoxyphenoxy)-2-nitrobenzoate (1.35 g, 3.38 mmol) and aluminum chloride (1.04 g, 7.83 mmol) were dissolved in toluene (80 mL) at room temperature. The reaction solution was stirred at 90°C for 16 hours under a nitrogen atmosphere. The reaction solution was extracted twice with 100 mL of ethyl acetate, and the aqueous phase was extracted again with 50 mL of ethyl acetate. The organic phases were combined, dried over anhydrous sodium sulfate, and filtered. The filtrate was concentrated under reduced pressure, and the residues were purified by silica gel column chromatography (mobile phase: PE/EA = 10:1) to obtain 400 mg of the title compound as a yellow solid, yield: 29.6%.

### Step 3: Preparation of methyl 7-fluoro-3-nitrodibenzo[b,e][1,4]dioxine-2-carboxylate (20c)

Methyl 4-bromo-5-(4-fluoro-2-hydroxyphenoxy)-2-nitrobenzoate (430 mg, 1.12 mmol) and potassium carbonate (310 mg, 2.23 mmol) were dissolved in DMF (40 mL) at room temperature, and a small amount of toluene was added dropwise. The reaction solution was stirred at 90°C for 16 hours under a nitrogen atmosphere. The reaction solution was extracted twice with 50 mL of ethyl acetate, and the aqueous phase was extracted again with 50 mL of ethyl acetate. The organic phases were combined, dried over anhydrous sodium sulfate, and filtered. The filtrate was concentrated under reduced pressure, and the residues were purified by silica gel column chromatography (mobile phase: PE/EA = 10:1) to obtain 220 mg of the title product as a yellow solid, yield: 51.2%.

### Step 4: Preparation of methyl 3-amino-7-fluorodibenzo[b,e][1,4]dioxine-2-carboxylate (20d)

Methyl 7-fluoro-3-nitrodibenzo[*b*,*e*][1,4]dioxine-2-carboxylate (250 mg, 0.820 mmol) and ammonium chloride (69.0 mg, 1.31 mmol) and iron powder (460 mg, 8.20 mmol) were dissolved in ethanol:water = 5:1 (30 mL) at room temperature. The reaction solution was stirred at 80°C for 6 hours. The reaction solution was extracted twice with 50 mL of ethyl acetate, and the aqueous phase was extracted again with 50 mL of ethyl acetate. The organic phases were combined, dried over anhydrous sodium sulfate, and filtered. The filtrate was concentrated under reduced pressure, and the residues were purified by silica gel column chromatography (mobile phase: PE/EA = 10:1-4:1) to obtain 180 mg of the title product as a light yellow solid, yield: 72%

### Step 5: Preparation of 2-(3-amino-7-fluorodibenzo[b,e][1,4]dioxin-2-yl)propanol (20)

Methylmagnesium chloride (1.50 mL, 3 mol/L) was dissolved in tetrahydrofuran (5 mL) at room temperature, followed by the slowly dropwise addition of a solution of methyl 3-amino-7-fluorodibenzo[*b*,*e*][1,4]dioxine-2-carboxylate (180 mg, 0.655 mmol) in tetrahydrofuran (5 mL) at 0°C. The reaction solution was stirred for 3 hours, extracted twice with 50 mL of ethyl acetate, and the aqueous phase was extracted again with 50 mL of ethyl acetate. The organic phases were combined, dried over anhydrous sodium sulfate, and filtered. The filtrate was concentrated under reduced pressure, and the residues were purified by silica gel column chromatography (mobile phase: PE/EA = 10:1-4:1) to obtain 160 mg of the title product as a light yellow solid, yield: 88.9%.

LC-MS: m/z 258 [M-17]⁺.

¹H NMR (400 MHz, DMSO-*d₆*) *δ* 6.92(d, *J*=8.9 Hz, 2H), 6.82 6.75 (m, 1H), 6.63(s, 1H), 6.26 (s, 1H), 5.30(d, *J*=52.9 Hz, 3H), 1.45 (s, 7H).

### Example 21: Preparation of 2-(3-amino-8-fluorodibenzo[b,e][1,4]dioxin-2-yl)propanol (21)

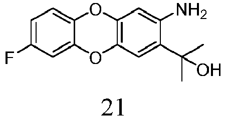

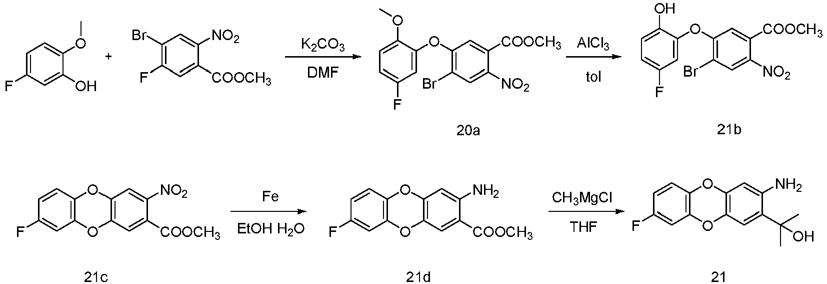

The synthesis steps were referred to Example 20, except that 4-fluoro-2-methoxyphenol was replaced with 5-fluoro-2-methoxyphenol, to obtain 10 mg of the title product as a light yellow solid, yield: 22.4%.

LC-MS: m/z 258 [M-17]⁺.

¹H NMR (400MHz, DMSO-*d*₆*) δ* 6.91 (s, 2H), 6.82-6.75 (m, 1 H), 6.63 (s, 1 H), 6.26 (s, 1 H), 5.36 (s, 2 H), 5.25 (s, 1 H), 1.45 (s, 6 H).

### Example 22: Preparation of 2-(2-amino-9,9-difluoro-9H-fluoren-3-yl)propanol (22)

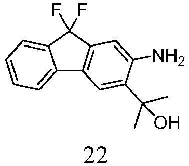

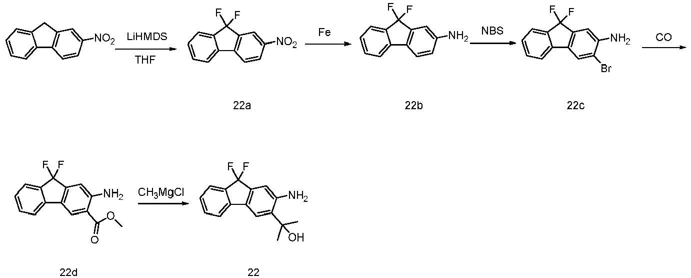

### Step 1: Preparation of 9,9-difluoro-2-nitro-9H-fluorene (22a)

2-Nitro-9*H*-fluorene (8.10 g, 384 mmol) and N-fluorobisbenzenesulfonamide (36.3 g, 115 mmol) were dissolved in DMF (200 mL) at room temperature. The solution was cooled to -78°C under a nitrogen atmosphere, and a LiHMDS solution (114 mL, 116 mmol) was added dropwise. The reaction solution was raised to room temperature and stirred for 5 hours. The reaction was quenched with ice water. The reaction solution was extracted twice with 500 mL of ethyl acetate, and the aqueous phase was extracted again with 200 mL of ethyl acetate. The organic phases were combined, dried over anhydrous sodium sulfate, and filtered. The filtrate was concentrated under reduced pressure, and the residues were purified by silica gel column chromatography (mobile phase: PE/EA = 10:1) to obtain 5.90 g of the title product as a light yellow solid, yield: 72.8%.

### Step 2: Preparation of 9,9-difluoro-9H-fluoren-2-amine (22b)

9,9-Difluoro-2-nitro-9*H*-fluorene (5.90 g, 23.9 mmol), iron powder (13.4 g, 239 mmol) and ammonium chloride (2.03 g, 38.4 mmol) were dissolved in a solution of ethanol:water = 60 ml: 12 ml at room temperature. The reaction solution was stirred overnight at 80°C, and filtered to remove the iron powder. The collected liquid was extracted twice with 300 mL ethyl acetate, and the aqueous phase was extracted again with 100 mL of ethyl acetate. The organic phases were combined, dried over anhydrous sodium sulfate, and filtered. The filtrate was concentrated under reduced pressure, and the residues were purified by silica gel column chromatography (mobile phase: PE/EA = 10:1) to obtain 4.50 g of the title compound as a yellow solid, yield: 76.2%.

### Step 3: Preparation of 3-bromo-9,9-difluoro-9H-fluoren-2-amine (22c)

9,9-Difluoro-9*H*-fluoren-2-amine (1.00 g, 4.60 mmol) and NBS (0.830 g, 4.60 mmol) were dissolved in chloroform (10 mL) at room temperature. The reaction solution was stirred for 16 hours, extracted twice with 200 mL of ethyl acetate, and the aqueous phase was extracted again with 100 mL of ethyl acetate. The organic phases were combined, dried over anhydrous sodium sulfate, and filtered. The filtrate was concentrated under reduced pressure, and the residues were purified by silica gel column chromatography (mobile phase: PE/EA = 10:1) to obtain 600 mg of the title compound as a yellow solid, yield: 60%.

### Step 4: Preparation of methyl 2-amino-9,9-difluoro-9H-fluorene-3-carboxylate (22d)

3-Bromo-9,9-difluoro-9*H*-fluoren-2-amine (500 mg, 1.70 mmol), pd(dppf)Cl₂ (124 mg, 1.70 mmol) and triethylamine (308 mg, 3.05 mmol) were dissolved in methanol (8 mL) at room temperature. The reaction solution was stirred overnight at 90°C under a carbon monoxide atmosphere. After cooling to room temperature, the reaction solution was extracted twice with 100 mL of ethyl acetate, and the aqueous phase was extracted again with 50 mL of ethyl acetate. The organic phases were combined, dried over anhydrous sodium sulfate, and filtered. The filtrate was concentrated under reduced pressure, and the residues were purified by silica gel column chromatography (mobile phase: PE/EA = 10:1) to obtain 220 mg of the title product as a yellow solid, yield: 44%.

### Step 5: Preparation of 2-(2-amino-9,9-difluoro-9H-fluoren-3-yl)propanol (22)

Methylmagnesium chloride (1.86 ml, 0.560 mmol) was dissolved in THF 5 ml at room temperature. The solution was cooled to 0°C under a nitrogen atmosphere, and methyl 2-amino-9,9-difluoro-9*H*-fluorene-3-carboxylate (220 mg, 0.800 mmol) was added slowly. The reaction solution was stirred for 3 hours, extracted twice with 100 mL of ethyl acetate, and the aqueous phase was extracted again with 50 mL of ethyl acetate. The organic phases were combined, dried over anhydrous sodium sulfate, and filtered. The filtrate was concentrated under reduced pressure, and the residues were purified by silica gel column chromatography (mobile phase: PE/EA = 10:1-4:1) to obtain 88.0 mg of the title product as a light yellow solid, yield: 40%.

LC-MS: m/z 258 [M-17]⁺.

¹H NMR (400 MHz, DMSO-*d₆*) *δ*7.62(d, *J*=7.6 Hz 1H), 7.53(d, *J*=7.4 Hz, 1H) 7.49-7.41 (m, 2H), 7.20(td, *J*=7.5, 1.0 Hz, 1H), 5.97 (s, 2H), 5.40(s, 1H), 1.56 (s, 6H).

### Example 23: Preparation of 2-(8-aminobenzo[5,6][1,4]dioxino[2,3-b]pyrazin-7-yl)propan-2-ol (23)

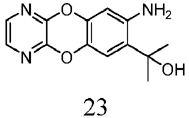

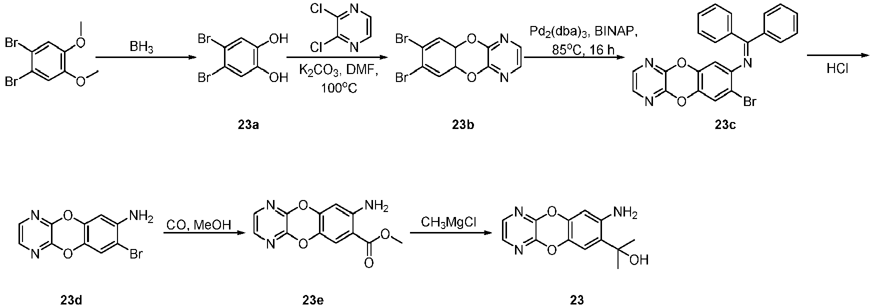

### Step 1: Preparation of 4,5-dibromobenzene-1,2-diol (23a)

Boron tribromide (100 mL, 100 mmol) was added to a solution of 1,2-dibromo-4,5-dimethoxybenzene (10.0 g, 34.0 mmol) in dichloromethane (240 mL) at room temperature. The reaction solution was stirred for 2 hours at room temperature, quenched with water (150 mL), and extracted with dichloromethane (150 mL×3). The organic phases were combined, dried, and concentrated under reduced pressure to obtain 10.0 g of the crude title compound as a brown oil.

### Step 2: Preparation of 7,8-dibromo-5a,9a-dihydrobenzo[5,6][1,4]dioxino[2,3-b]pyrazine (23b)

2,3-Dichloropyrazine (4.48 g, 30.1 mmol) was added to a solution of 4,5-dibromobenzene-1,2-diol (10.0 g, 37.6 mmol) and potassium carbonate (15.6 g, 173 mmol) in DMF (50 mL) at room temperature, and the reaction solution was stirred overnight at room temperature. The reaction solution was diluted with ethyl acetate (300 mL), and washed with water (200 mL×3). The organic phase was dried, and the residues were purified by silica gel column chromatography (mobile phase: petroleum ether/ethyl acetate = 4:1) to obtain 5.0 g of the title compound as a white solid, yield: 38.7%.

### Step 3: Preparation of N-(8-bromobenzo[5,6][1,4]dioxino[2,3-b]pyrazin-7-yl)-1,1-diphenylmethanimine (23c)

Tris(dibenzylideneacetone) dipalladium (399 mg, 0.436 mmol) and 2,2'-Bis(diphenylphosphino)-1,1'-binaphthyl (379 mg, 0.654 mmol) were added to a solution of 7,8-dibromo-5a,9a-dihydrobenzo[5,6][1,4]dioxino[2,3-*b*]pyrazine (1.5 g, 4.36 mmol), dibenzylamine (868 mg, 4.80 mmol) and sodium *tert*-butoxide (628 mg, 6.54 mmol) in toluene (50 mL) at room temperature under a nitrogen atmosphere. The reaction solution was stirred overnight at 85°C, cooled and concentrated under reduced pressure. The residues were purified by silica gel column chromatography (mobile phase: petroleum ether/ethyl acetate = 10:1) to obtain 1.20 g of the title compound as a yellow solid, yield: 62.2%.

### Step 4: Preparation of 8-bromobenzo[5,6][1,4]dioxino[2,3-b]pyrazin-7-amine (23d)

Diluted hydrochloric acid (10 mL, 1mol/L) was added to a solution of *N*-(8-bromobenzo[5,6][1,4]dioxino[2,3-*b*]pyrazin-7-yl)-1,1-diphenylmethanimine (1.20 g, 2.71 mmol) in tetrahydrofuran (20 mL) at room temperature. The reaction solution was stirred for 30 minutes at room temperature. The reaction solution was adjusted to pH to 8 with saturated aqueous sodium bicarbonate solution, and extracted with ethyl acetate (100 mL×2). The organic phase was combined, dried, and concentrated under reduced pressure. The residues were purified by silica gel column chromatography (mobile phase: petroleum ether/ethyl acetate = 10:l) to obtain 400 mg of the title product as a white solid, yield: 52.9%.

### Step 5: Preparation of methyl 8-aminobenzo[5,6][1,4]dioxino[2,3-b]pyrazine-7-carboxylate (23e)

[1,1'-Bis(diphenylphosphino)ferrocene]dichloropalladium(II) (105 mg, 0.143 mmol) was added to a solution of 8-bromobenzo[5,6][1,4]dioxino[2,3-*b*]pyrazin-7-amine (400 mg, 1.43 mmol) and triethylamine (290 mg, 2.87 mmol) in methanol (20 mL) at room temperature. The system was heated to 90°C and stirred for 16 hours under a carbon monoxide atmosphere. The reaction solution was cooled to room temperature, and filtered through Celite. The filtrate was concentrated under reduced pressure, and the residues were purified by silica gel column chromatography (mobile phase: petroleum ether/ethyl acetate = 5:l) to obtain 360 mg of the title compound as a yellow solid, yield: 97.0%.

LC-MS: m/z = 260 [M+H]⁺.

### Step 6: Preparation of 2-(8-aminobenzo[5,6][1,4]dioxino[2,3-b]pyrazin-7-yl)propan-2-ol (23)

Methyl 8-aminobenzo[5,6][1,4]dioxino[2,3-*b*]pyrazine-7-carboxylate (60 mg, 0.232 mmol) was added to a solution of methylmagnesium bromide (0.54 mL, 3 mol/L) in anhydrous tetrahydrofuran (10 mL) at room temperature under a nitrogen atmosphere. The reaction solution was stirred at 0°C for 3 hours under a nitrogen atmosphere. The reaction solution was quenched with saturated aqueous ammonium chloride solution (10 mL), and extracted with ethyl acetate (40 mL×2). The organic phases were combined, dried over anhydrous sodium sulfate, and filtered. The filtrate was concentrated under reduced pressure, and the residues were purified by preparative liquid chromatography (column model: Daisogei 30mm*250mm, C18, 10 µm, 100A, mobile phase: acetonitrile/water, gradient: 10%-50%) to obtain 28.0 mg of the title product as a white solid, yield: 20.0%.

LC-MS: m/z = 260 [M+H]⁺.

¹H NMR (400 MHz, DMSO-*d*₆) δ 7.89-7.76 (m, 2H), 6.76 (s, 1H), 6.36 (s, 1H), 5.48 (s, 2H), 5.29 (s, 1H), 1.47 (s, 6H).

### Example 24: Preparation of 2-(2-amino-9,9-dimethyl-9H-xanthen-3-yl)propan-2-ol (24)

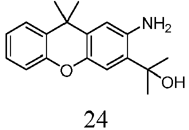

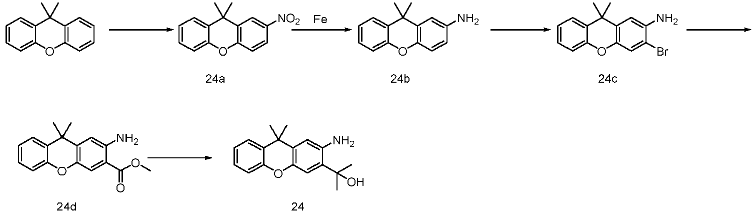

### Step 1: Preparation of 9,9-dimethyl-2-nitro-9H-xanthene (24a)

Concentrated nitric acid (5 mL) was added dropwise slowly to a solution of 9,9-dimethyl-9*H*-xanthene (10.0 g, 47.2 mmol) in acetic acid (100 mL) and concentrated sulfuric acid (10 mL) at 0°C, and the reaction solution was stirred overnight at room temperature. The reaction solution was adjusted the pH value to 8 with saturated aqueous sodium bicarbonate solution, and extracted with ethyl acetate (300 mL×3). The organic phase was combined and dried, and the residues were purified by silica gel column chromatography (mobile phase: petroleum ether/ethyl acetate = 100:l) to obtain 11.0 g of the title compound as a white solid, yield: 91.7%.

### Step 2: Preparation of 9,9-dimethyl-9H-xanthen-2-amine (24b)

Reduced iron powder (22 g, 392 mmol) was added to a solution of 9,9-dimethyl-2-nitro-9*H*-xanthene (10.0 g, 39.2 mmol) and ammonium chloride (3.33 g, 62.7 mmol) in ethanol (200 mL) and water (40 mL) at room temperature, and the reaction solution was stirred at 80°C overnight. The reaction solution was concentrated and filtered. The filtrate was diluted with ethyl acetate (300 mL), and washed with water (200 mL×2). The organic phase was dried, and the residues were purified by silica gel column chromatography (mobile phase: petroleum ether/ethyl acetate = 1:l) to obtain 6.00 g of the title compound as a yellow solid, yield: 69.1%.

LC-MS: m/z = 226 [M+H]⁺.

### Step 3: Preparation of 3-bromo-9,9-dimethyl-9H-xanthen-2-amine (24c)

NBS (2.40 g, 13.3 mmol) was added to a solution of 9,9-dimethyl-9*H*-xanthen-2-amine (3.00 g, 13.3 mmol) in chloroform (20 mL) at room temperature, and the reaction solution was stirred at room temperature overnight. The reaction solution was diluted with ethyl acetate (50 mL), and washed with water (50 mL×2). The organic phase was dried, and the residues were purified by silica gel column chromatography (mobile phase: petroleum ether/ethyl acetate = 5:l) to obtain 1.60 g of the title compound as a yellow solid, yield: 40.1%.

LC-MS: m/z = 304 [M+H]⁺.

### Step 4: Preparation of methyl 2-amino-9,9-dimethyl-9H-xanthene-3-carboxylate (24d)

[1,1'-Bis(diphenylphosphino)ferrocene]dichloropalladium(II) (145 mg, 0.198 mmol) was added to a solution of 3-bromo-9,9-dimethyl-9*H*-xanthen-2-amine (600 mg, 1.98 mmol) and triethylamine (360 mg, 3.56 mmol) in methanol (10 mL) at room temperature. The system was heated to 90°C and stirred for 18 hours under a carbon monoxide atmosphere. The reaction solution was cooled to room temperature, and filtered through Celite. The filtrate was concentrated under reduced pressure, and the residues were purified by silica gel column chromatography (mobile phase: petroleum ether/ethyl acetate = 4:l) to obtain 280 mg of the title compound as a yellow solid, yield: 50.0%.

LC-MS: m/z = 284 [M+H]⁺.

### Step 5: Preparation of 2-(2-amino-9,9-dimethyl-9H-xanthen-3-yl)propan-2-ol (24)

Methyl 2-amino-9,9-dimethyl-9*H*-xanthene-3-carboxylate (180 mg, 0.636 mmol) was added to a solution of methylmagnesium bromide (1.48 mL, 4.45 mmol) in anhydrous tetrahydrofuran (10 mL) at room temperature under a nitrogen atmosphere. The reaction solution was stirred at 0°C for 3 hours under a nitrogen atmosphere. The reaction solution was quenched with saturated aqueous ammonium chloride solution (10 mL), and extracted with ethyl acetate (40 mL×2). The organic phases were combined, dried over anhydrous sodium sulfate, and filtered. The filtrate was concentrated under reduced pressure, and the residues were purified by preparative liquid chromatography (column model: Gemini-C18 150*21.2 mm, 5 µm, mobile phase: acetonitrile/water 0.05% formic acid, gradient: 2 min-30% ~ 22 min-70%) to obtain 55.0 mg of the title compound as a white solid, yield: 30.6%.

LC-MS: m/z = 284 [M+H]⁺.

¹H NMR (400 MHz, DMSO-d₆) δ 7.53-7.46 (m, 1H), 7.21-7.14 (m, 1H), 7.09-7.01 (m, 1H), 6.99-6.92 (m, 1H), 6.73 (d, *J* = 8.1 Hz, 2H), 5.22 (s, 1H), 5.15 (s, 2H), 1.52 (d, *J* = 10.8 Hz, 12H).

### Example 25: Preparation of 2-(3-amino-7-chlorodibenzo[b,e][1,4]dioxin-2-yl)propanol (25)

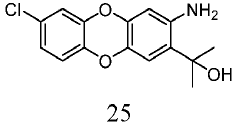

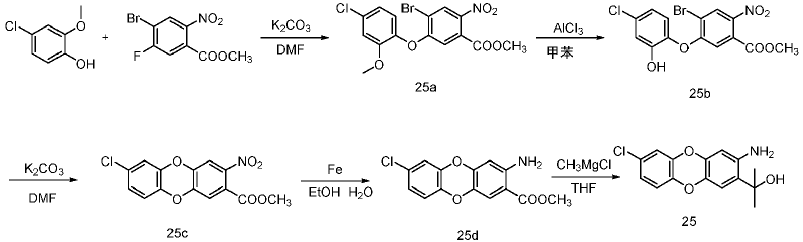

The synthesis steps were referred to Example 20, except that 4-fluoro-2-methoxyphenol was replaced with 4-chloro-2-methoxyphenol to obtain 27 mg of the title product as a light yellow solid, yield: 28.9%.

LC-MS: m/z 273.9 [M-17]⁺.

¹H NMR (400 MHz, CHCl₃-*d*) δ 6.87-6.80 (m, 2H), 6.72 (d, *J* = 9.2 Hz, 2H), 6.65 (s, 1H), 6.17 (s, 1H), 1.63 (s, 6H).

### Example 26: Preparation of 2-(3-amino-8-chlorodibenzo[b,e][1,4]dioxin-2-yl)propanol (26)

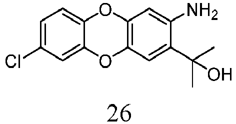

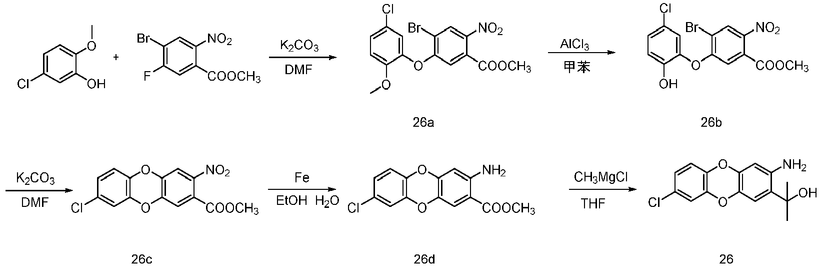

The synthesis steps were referred to Example 20, except that 4-fluoro-2-methoxyphenol was replaced with 5-chloro-2-methoxyphenol to obtain 8.00 mg of the title product as a light yellow solid, yield: 12.9%.

LC-MS: m/z 273.9 [M-17]⁺.

¹H NMR (400 MHz, Chlorofom-*d*) *δ* 6.84-6.83 (m, 2H), 6.73 (d, *J* = 9.2 Hz, 2H), 6.66 (s, 1H), 6.19 (s, 1H), 1.63 (s, 6H).

### Example 27: Preparation of 2-(2-amino-10-benzyl-10H-phenoxazin-3-yl)propan-2-ol (27)

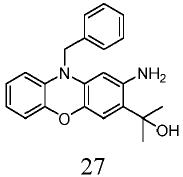

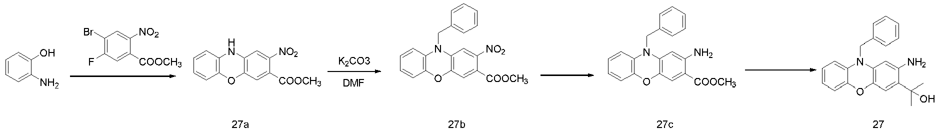

### Step 1: Preparation of methyl 2-nitro-10H-phenoxazine-3-carboxylate (27a)

2-Aminophenol (1.00 g, 9.17 mmol), cesium carbonate (4.14 g, 18.3 mmol) and methyl 4-bromo-5-fluoro-2-nitrobenzoate (2.54 g, 9.17 mmol) were dissolved in DMF (30 mL). The reaction solution was stirred overnight at 120°C, and 100 mL of water was added. The reaction solution was extracted with 100 mL ethyl acetate, and the aqueous phase was extracted twice with 100 mL ethyl acetate. The organic phases were combined, dried over anhydrous sodium sulfate, and filtered. The filtrate was concentrated under reduced pressure, and the residues were purified by silica gel column chromatography (mobile phase: ethyl acetate) to obtain 460 mg of the title product as a red solid, yield: 17.5%.

### Step 2: Preparation of methyl 10-benzyl-2-nitro-10H-phenoxazine-3-carboxylate (27b)

Methyl 2-nitro-10*H*-phenoxazine-3-carboxylate (460 mg, 1.61 mmol), cesium carbonate (727 mg, 3.22 mmol) and benzyl bromide (540 mg, 3.22 mmol) were dissolved in acetonitrile (10 mL) at room temperature, and the reaction solution was stirred at 60°C for 1 hour. The reaction solution was filtered through Celite, and the filtrate was concentrated under reduced pressure. The residues were purified by silica gel column chromatography (mobile phase: methanol) to obtain 440 mg of the title product as a brown solid, yield: 72.7%.

### Step 3: Preparation of methyl 2-amino-10-benzyl-10H-phenoxazine-3-carboxylate (27c)

Methyl 10-benzyl-2-nitro-10*H-*phenoxazine-3-carboxylate (440 mg, 1.17 mmol), iron powder (655 mg, 11.7 mmol) and NH₄Cl (861 mg, 15.3 mmol) were dissolved in ethanol (20 mL) and water (4 mL) at room temperature, and the reaction solution was stirred under reflux at 80°C for 4 hours. The reaction solution was filtered through Celite, and the filtrate was concentrated under reduced pressure. The residues were purified by silica gel column chromatography (mobile phase: methanol) to obtain 170 mg of the title product as a brown solid, yield: 42.0%.

### Step 4: Preparation of 2-(2-amino-10-benzyl-10H-phenoxazin-3-yl)propan-2-ol (27)

Methylmagnesium chloride (3 N, 0.8 mL, 2.40 mmol) was added dropwise to THF (5 mL) at 0°C under a nitrogen atmosphere, and methyl 2-amino-10-benzyl-10*H*-phenoxazine-3-carboxylate (100 mg, 0.289 mmol) dissolved in THF (5 mL) was added. The reaction solution was stirred for 3 hours at room temperature, and 50 mL of water was added. The reaction solution was extracted with 50 mL of dichloromethane, and the aqueous phase was extracted twice with 50 mL of dichloromethane. The organic phases were combined, dried over anhydrous sodium sulfate, and filtered. The filtrate was concentrated under reduced pressure, and the residues were purified by preparative liquid chromatography (column model: Gemini-C18 150*21.2 mm, 5 µm, mobile phase: acetonitrile/water 0.05% formic acid, gradient: 2 min-30% ~ 22 min-70%) to obtain 15.0 g of the title compound as a white solid, yield: 15.0%.

LC-MS: m/z 329.10 [M-17]⁺.

¹H NMR (400 MHz, CDCl₃) *δ* 7.36-7.21 (m,5 H), 6.72 (td, *J* = 7.8,1.6 Hz, 1H), 6.66 (dd, *J* = 7.8,1.6 Hz, 1H), 6.58 (td, *J* = 7.6,1.4 Hz, 1H), 6.50 (dd, *J* = 7.8,1.6 Hz, 1H), 6.18 (s, 1H), 6.09 (s, 2H), 5.05 (d, *J* = 1.4 Hz, 3H), 4.78 (s, 2H), 1.27 (s, 6H).

### Example 28: Preparation of 2-(2-amino-9-methoxy-9H-fluoren-3-yl)propan-2-ol (28)

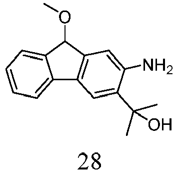

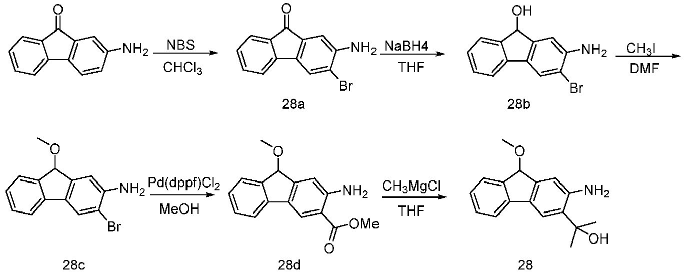

### Step 1: Preparation of 2-amino-3-bromo-9H-fluoren-9-one (28a)

2-Amino-9*H*-fluoren-9-one (1.50 g, 7.68 mmol) and N-bromosuccinimide (NBS) (1.39 g, 7.68 mmol) were dissolve in chloroform (30 mL) at room temperature under a nitrogen atmosphere. The reaction solution was stirred at room temperature for 12 hours, followed by addition of water (40 mL). The reaction solution was extracted three times with dichloromethane (30 mL). The organic phases were combined, dried over anhydrous sodium sulfate, and filtered. The filtrate was concentrated under reduced pressure, and the residues were purified by silica gel column chromatography (mobile phase: petroleum ether/ethyl acetate = 4/1) to obtain 1.30 g of the title product as a red solid, yield: 62.0%.

LC-MS: m/z = 275.1 [M+H]⁺.

### Step 2: Preparation of 2-amino-3-bromo-9H-fluoren-9-ol (28b)

2-Amino-3-bromo-9*H*-fluoren-9-one (1.30 g, 4.74 mmol) was dissolved in anhydrous tetrahydrofuran (30 mL) at 0°C under a nitrogen atmosphere, followed by the slow addition of sodium borohydride (358 mg, 9.48 mmol). The reaction solution was slowly raised to room temperature and stirred for 12 hours. The reaction solution was quenched with ice water (40 mL), and extracted three times with ethyl acetate (30 mL). The organic phases were combined, dried over anhydrous sodium sulfate, and filtered. The filtrate was concentrated under reduced pressure, and the residues were purified by silica gel column chromatography (mobile phase: petroleum ether/ethyl acetate = 4/1) to obtain 1.16 g of the title compound as a red solid, yield: 89.4%.

LC-MS: m/z = 277.1 [M+H]⁺.

### Step 3: Preparation of 3-bromo-9-methoxy-9H-fluoren-2-amine (28c)

2-Amino-3-bromo-9*H*-fluoren-9-ol (860 mg, 3.11 mmol) and potassium carbonate (434 mg, 3.14 mmol) were dissolved in *N,N-*dimethylformamide (15 mL) at room temperature under a nitrogen atmosphere, followed by the slow addition of methyl iodide (446 mg, 3.14 mmol). The reaction solution was stirred at 50°C for 12 hours, and water (40 mL) was added. The reaction was extracted three times with ethyl acetate (30 mL). The organic phases were combined, dried over anhydrous sodium sulfate, and filtered. The filtrate was concentrated under reduced pressure, and the residues were purified by silica gel column chromatography (mobile phase: petroleum ether/ethyl acetate = 4/1) to obtain 350 mg of the title compound as a white solid, yield: 38.6%.

LC-MS: m/z = 291.2 [M+H]⁺.

### Step 4: Preparation of methyl 2-amino-9-methoxy-9H-fluorene-3-carboxylate (28d)

3-Bromo-9-methoxy-9*H*-fluoren-2-amine (266 mg, 0.917 mmol), triethylamine (148 mg, 1.47 mmol) and [1,1'-Bis(diphenylphosphino)ferrocene]dichloropalladium(II) (67.1 mg, 0.0917 mmol) were dissolved in methanol (10 mL) at room temperature. The solution was placed in an autoclave, and stirred at 90°C for 12 hours under a carbon monoxide atmosphere. The solution was concentrated under reduced pressure concentration, and the residues were purified by silica gel column chromatography (mobile phase: petroleum ether/ethyl acetate = 4/1) to obtain 225 mg of the title compound as a yellow solid, yield: 92.2%.

LC-MS: m/z = 270.3 [M+H]⁺.

### Step 5: Preparation of 2-(2-amino-9-methoxy-9H-fluoren-3-yl)propan-2-ol (28)

Methyl 2-amino-9-methoxy-9*H*-fluorene-3-carboxylate (50.0 mg, 0.190 mmol) was dissolved in anhydrous tetrahydrofuran (6 mL) at 0°C under a nitrogen atmosphere, and a solution of methylmagnesium chloride (0.440 mL, 1.33 mmol) in tetrahydrofuran was added dropwise. The reaction solution was stirred at room temperature for 12 hours, followed by addition of water (40 mL), and the reaction was extracted with ethyl acetate (30 mL) for three times. The organic phases were combined, dried over anhydrous sodium sulfate, and filtered. The filtrate was concentrated under reduced pressure, and the residues were purified by silica gel column chromatography (mobile phase: petroleum ether/ethyl acetate = 4/1) to obtain a gray solid. The gray solid was purified by preparative liquid chromatography (column model: C18-4, mobile phase: acetonitrile, water, gradient: 20%-55%) to obtain 10 mg of the title compound as a white solid, yield: 7.78%.

LC-MS: m/z = 270.1 [M+H]⁺.

¹H NMR (400 MHz, DMSO-d₆) *δ* 7.54 (d, *J* = 7.5 Hz, 1H), 7.45 (d, *J* = 7.4 Hz, 2H), 7.25 (t, *J* = 7.3 Hz, 1H), 7.10 (t, *J* = 7.8 Hz, 1H), 6.79 (s, 1H), 6.64 (q, *J* = 4.9 Hz, 1H), 5.62 (d, *J* = 7.3 Hz, 1H), 5.44 (s, 1H), 5.33 (d, *J* = 7.2 Hz, 1H), 2.83 (d, *J* = 5.1 Hz, 3H), 1.58 (d, *J* = 4.2 Hz, 6H).

### Example 29: Preparation of tert-Butyl 2-amino-3-(2-hydroxypropan-2-yl)-10H-phenoxazine-10-carboxylate (29)

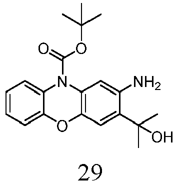

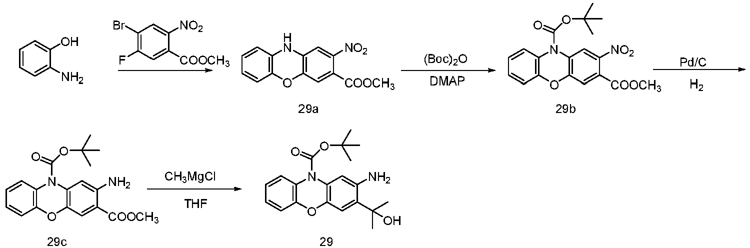

### Step 1: Preparation of methyl 2-nitro-10H-phenoxazine-3-carboxylate (29a)

2-Aminophenol (1.00 g, 9.25 mmol), methyl 4-bromo-5-fluoro-2-nitrobenzoate (2.57 g, 9.25 mmol) and cesium carbonate (6.02 g, 18.50 mmol) were dissolved in *N,N-*dimethylformamide (50 mL) at room temperature under a nitrogen atmosphere. The reaction solution was stirred at 100°C for 12 hours, followed by addition of water (40 mL), and the reaction was extracted with ethyl acetate (40 mL) for three times. The organic phases were combined, dried over anhydrous sodium sulfate, and filtered. The filtrate was concentrated under reduced pressure, and the residues were purified by silica gel column chromatography (mobile phase: petroleum ether/ethyl acetate = 4/1) to obtain 1.07 g of the title compound as a red solid, yield: 40.6%.

LC-MS: m/z = 287.2 [M+H]⁺.

### Step 2: Preparation of 10-(tert-butyl) 3-methyl 2-nitro-10H-phenoxazine-3,10-dicarboxylate (29b)

Methyl 2-nitro-10*H*-phenoxazine-3-carboxylate (800 mg, 2.79 mmol), di-*tert*-butyl dicarbonate (1.10 g, 5.02 mmol), potassium carbonate (965 mg, 6.98 mmol) and 4-dimethylaminopyridine (34.1 mg, 0.279 mmol) were dissolved in acetonitrile (50 mL) at room temperature under a nitrogen atmosphere. The reaction solution was heated and refluxed under stirring for 12 hours, followed by addition of water (40 mL), and the reaction was extracted with ethyl acetate (30 mL) for three times. The organic phases were combined, dried over anhydrous sodium sulfate, and filtered. The filtrate was concentrated under reduced pressure, and the residues were purified by silica gel column chromatography (mobile phase: petroleum ether/ethyl acetate = 10/1) to obtain 723 mg of the title compound as a yellow solid, yield: 67.0%.

### Step 3: Preparation of 10-(tert-butyl) 3-methyl 2-amino-10H-phenoxazine-3,10-dicarboxylate (29c)

10-(*tert*-Butyl) 3-methyl 2-nitro-10*H*-phenoxazine-3,10-dicarboxylate (350 mg, 0.980 mmol) and palladium carbon (35.0 mg, 10%wt) were added to methanol (50 mL) at room temperature under a nitrogen atmosphere. The reaction solution was stirred for 12 hours under a hydrogen atmosphere. The reaction solution was filtered through Celite, and the filtrate was concentrated under reduced pressure. The residues were purified by silica gel column chromatography (mobile phase: petroleum ether/ethyl acetate = 10/1) to obtain 301 mg the title compound as a white solid, yield: 86.2%.

LC-MS: m/z = 357.3 [M+H]⁺.

### Step 4: Preparation of tert-butyl 2-amino-3-(2-hydroxypropan-2-yl)-10H-phenoxazine-10-carboxylate (29)

Methylmagnesium chloride (2.25 mL, 6.76 mmol) was dissolved in anhydrous tetrahydrofuran (10 mL) at 0°C under a nitrogen atmosphere, and a soulution of 10-(*tert*-butyl) 3-methyl 2-amino-10*H*-phenoxazine-3,10-dicarboxylate (301 mg, 0.845 mmol) in tetrahydrofuran was added dropwise. The reaction solution was stirred at room temperature for 12 hours, followed by addition of water (10 mL), and the reaction was extracted with ethyl acetate (40 mL) for three times. The organic phases were combined, dried over anhydrous sodium sulfate, and filtered. The filtrate was concentrated under reduced pressure, and the residues were purified by silica gel column chromatography (mobile phase: petroleum ether/ethyl acetate = 4/1) to obtain a gray solid. The gray solid was purified by preparative liquid chromatography (column model: C18-4, mobile phase: acetonitrile, water, gradient: 20%-55%) to obtain 114 mg of the title compound as a white solid, yield: 37.9%.

LC-MS: m/z = 339.1 [M-17]⁺.

¹H NMR (400 MHz, DMSO-d₆) *δ* 7.51 - 7.47 (m, 1H), 7.14 (d, *J =* 7.5 Hz, 2H), 7.08 (t, *J* = 6.8 Hz, 2H), 6.36 (s, 1H), 5.57 (s, 2H), 5.27 (s, 1H), 1.48 (d, *J* = 6.5 Hz, 15H).

### Biological Assay

### Test Example 1: Test of the determination of aldehyde capture rate

In the aldehyde capture test disclosed in the present invention, the stable lipid metabolite, nonenal, was used as the model aldehyde to react with the compound of the Example. The specific protocol was as follows.

0.05 mM of the compounds of the Examples of the present invention were precisely weighed respectively and dissolved in 1.2 mL of a mixed solution (v/v=1:1) of triolein (Aladdin, G105172-5g) and linoleic acid (Aladdin, L100442-25g), respectively. 2.5 mL of PBS (Solarbio, P1020) solution containing 20% Captisol^{®} (Aladdin, C125030-25g) was added, and the solution was stirred at room temperature overnight. After the example compound was completely dissolved, 0.025 mM nonenal (Sigma, 255653-5G) was added, and the solution was stirred vigorously at room temperature. 200 µL of the sample was added to 800 µL of acetonitrile (Fisher, A995) at 0, 45, 90, 180, 225 and 465 minutes, respectively. The solution was mixed vigorously by vortex for 2 minutes, and centrifuged at a low speed of 1000 r/min for 3 minutes. The supernatant was transferred to an injection vial. The nonenal in the reaction mixture was analyzed quantitatively by high performance liquid chromatography. The adduct resulted from the example compound of the present invention and nonenal was analyzed qualitatively by high performance liquid chromatography-mass spectrometry.
Liquid chromatograph: Waters I Class;
Column: ACQUITY UPLC HSS T3 1.8µm;
Column temperature: 40°C;
Liquid chromatography condition: mobile phase A: acetonitrile containing 0.1% formic acid; B: water containing 0.1% formic acid;
Flow rate: 0.4 mL/mL;
Injection volume: 0.5 mL.
Gradient of the mobile phases:

| Time (min) | A% |
|---|---|
| 0 | 10 |
| 0.5 | 10 |
| 2.5 | 95 |
| 3.4 | 95 |
| 3.5 | 10 |
| 4.0 | 10 |

In the developed detection method, the retention time of the model aldehyde nonenal was 2.42 minutes.
Mass spectrometry conditions:
   Mass spectrometer: Waters TQ-S micro
   Ion source: ESI⁺
   Capillary voltage: 0.5kV
   Cone voltage: 10V
   Source temperature: 150°C
   Degassing temperature: 500°C, degassing flow: 1000L/Hr
SIM mode
   m/z: 225.24 [(m_{Example 1}-OH) + H]⁺, 365.42 [m_{adduct 1}+H]⁺

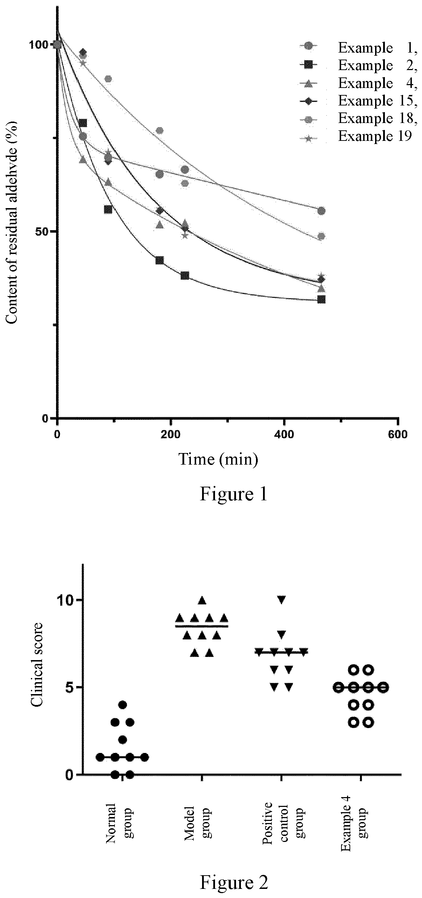

The time-varying diagram of the capture reaction of the compounds of the examples of the present invention to nonenal is shown in Figure 1. The aldehyde consumption behavior of the example compounds of the present invention is shown in Table 1 below.

**Table 1 Aldehyde consumption behavior of the example compounds of the present invention at different time points**

| Compounds | 135 minutes | | 180 minutes | |
|---|---|---|---|---|
| | Aldehyde consumption (100%) | Capture rate ^{a} | Aldehyde consumption (100%) | Capture rate ^{a} |
| Example 1 | 24.57 | -0.13 | 34.69 | -0.14 |
| Example 2 | 43.48 | -0.34 | 57.75 | -0.31 |
| Example 3 | 0.11 | -0.01 | 1.38 | -0.02 |
| Example 4 | 38.33 | -0.27 | 48.10 | -0.23 |
| Example 5 | 15.06 | -0.14 | 19.05 | -0.10 |
| Example 6 | 5.40 | -0.08 | 11.94 | -0.07 |
| Example 7 | 5.17 | -0.07 | 11.82 | -0.07 |
| Example 8 | 8.30 | -0.22 | 17.80 | -0.07 |
| Example 9 | 0.10 | -0.01 | 0.10 | -0.04 |
| Example 10 | 9.15 | -0.08 | 9.48 | -0.06 |
| Example 11 | 7.71 | -0.06 | 12.33 | -0.08 |
| Example 12 | 17.18 | -0.24 | 30.31 | -0.16 |
| Example 13 | 6.03 | -0.12 | 14.83 | -0.08 |
| Example 14 | 36.30 | -0.19 | 44.51 | -0.28 |
| Example 15 | 23.50 | -0.15 | 30.00 | -0.17 |
| Example 16 | 53.50 | -0.24 | 55.80 | -0.36 |
| Example 17 | 17.55 | -0.12 | 23.03 | -0.14 |
| Example 18 | 39.10 | -0.32 | 43.96 | -0.28 |
| Example 19 | 19.40 | -0.14 | 36.90 | -0.25 |
| Example 20 | 23.91 | -0.18 | 25.45 | -0.14 |
| Example 21 | 6.92 | -0.05 | 10.06 | -0.05 |
| Example 23 | 16.24 | -0.12 | 17.89 | -0.11 |
| Example 25 | 4.87 | -0.03 | 4.88 | -0.03 |
| Example 26 | 2.02 | -0.02 | 5.34 | -0.03 |
| Example 27 | 14.37 | -0.11 | 17.87 | -0.10 |
| Example 28 | 3.77 | -0.02 | 4.37 | -0.03 |
| Example 29 | 6.52 | -0.04 | 6.53 | -0.04 |

| | | | | |
|---|---|---|---|---|
| a. Aldehyde capture rate is represented by the slope of the capture curve per unit time, and the higher the absolute value of the slope, the higher the capture rate. | | | | |

It can be seen from Figure 1 and the above Table 1 that the example compounds of the present invention have aldehyde capture ability.

### Test Example 2: Therapeutic effect of the compound of the present invention in uveitis animal model

Female lewis rats were used as the research subjects. Lewis rats (Vital River) were randomly divided into groups (4 animals per group, a total of 8 eyes). The groups are as follows: normal control group, model control group, and example administration group. The inflammation model was established in these groups except for the normal control group.

### Formulation of eye drops:

(1) Formulation of drug vehicle stock solution: 2090 mg of Na₂HPO₄ · 12H₂O (Xilong Scientific, 9009012-01-09), 19 mg of NaH₂PO₄·2H₂O (Xilong Scientific, 9009013-01-09) and 9500 mg of β-cyclodextrin (Sigma, E1930253) were precisely weighed and dissolved in 30 mL sterile water for injection (Beijing CR Double-Crane), followed by addition of 10 mL of PEG-400 (Solarbio, 222U011). The solution was mixed well, and made up to 50 mL with water for injection.
(2) Formulation of drug solution: 10 mg of the compound of Example 2 of the present invention was weighed and dissolved in 1 mL of the vehicle stock solution, and the resulting solution was made up to 2 mL with water for injection. The content of each substance is as follows: Na₂HPO₄·12H₂O 0.83%, NaH₂PO₄·2H₂O 0.017%, β-cyclodextrin 9.5%, PEG-400 5.0%, active compound 0.5%.
(3) The pH value of the formulated drug solution was determined by pH test paper. If the pH value was not within 7.3 ± 0.05, then it was adjusted to this range with 1 N HCl or 1 N NaOH. The solution was filtered through a 0.22 µm sterile filter (Merck, Millex), and stored at 4°C.

### Handling of the animal model:

The animal model was a noninfectious uveitis animal model established by intraperitoneal injection of lipopolysaccharide (derived from *Escherichia coli,* 055:B5, Sigma, L2880-100MG, injection dose: 2 mg/kg). At the same time of modeling, drug administration was carried out according to the above grouping situation. The normal control group was administered with normal saline, the model control group was administered with blank vehicle, and the example administration group was administered with the eye drops formulated above. The drug was administered by eye instillation to both eyes of each animal, and the eye instillation dose was 20 µL/eye. After the completion of eye instillation, the eyelids were closed for 20 seconds to prevent the loss of the drug. The administration was repeated at 3, 6 and 23 hours after modeling. When the illness was severe (24 hours after modeling), the animals were anesthetized by intraperitoneal injection of 3.5% chloral hydrate. The ocular inflammation of the animals in each group was examined with a slit lamp microscope (Jiangxi Jiangfeng, LYL-S), and evaluated according to the McDonald-Shadduck scoring system (GB/T 28538-2012).

The animals were sacrificed, and the bilateral eyeballs were enucleated. The eyeball was subjected to corneal puncture. The aqueous humor of each eyeball was collected by a capillary, placed in a 1.5 mL centrifuge tube, and centrifuged at 1000 r/min. Quantitative analysis of total protein in aqueous humor was performed on the supernatant by a BCA protein quantification kit (Beyotime, P0010).

The effect of the compound of Example 2 of the present invention on the ocular inflammation score and protein concentration in aqueous humor of the ocular inflammation model rats are shown in Table 2 below.

**Table 2 Ocular inflammation score and protein concentration in aqueous humor of the model rats after treatment (mean ± standard deviation)**

| Group of the animals | Ocular score | Protein concentration in aqueous humor (µg/mL) |
|---|---|---|
| Normal control group | 4.5±0.8 | 225.5±65.4 |
| Model control group | 17.0±6.4 | 1062.7±423.6 |
| Example 2 group | 8.6±1.1^{∗} | 376.4±164.0^{∗∗∗} |

| | | |
|---|---|---|
| ^{∗}P<0.05, ^{∗∗∗}P<0.001, V.S. model control group | | |

It can be seen from the above Table 2 that in the ocular inflammation animal model, the treatment with the compound of Example 2 of the present invention can effectively reduce the ocular inflammation model score and significantly reduce the protein concentration in the aqueous humor, showing a therapeutic effect.

### Test Example 3: Therapeutic effect of the compound of the present invention on allergic conjunctivitis animal model

C48/80 is a polymer formed by the condensation of N-methyl-p-methoxyphenethylamine with formaldehyde, which acts directly on G protein and induces mast cell degranulation. After degranulation, the mast cell releases active substances such as histamine and kinin, which can cause acute type I allergic reactions such as telangiectasia and enhanced permeability. It can cause allergic conjunctivitis if applied topically to the ocular surface.

Female Wistar rats were used as the research subjects. Wistar rats (Vital River) were randomly divided into groups (5 animals per group, a total of 10 eyes). The groups are as follows: normal control group, model control group, positive drug group, and example administration group. The inflammation model was established in these groups except for the normal control group. Emedastine Difumarate Eye Drops (Emadine^{®}, Alcon, H20181192) was used as the positive drug. Model establishment, administration and evaluation processes were as follows.

The animal was subjected to inhalation anesthesia (isoflurane, Hebei Yipin pharmaceutical Co., Ltd., C002151205; anesthesia parameters: flow rate: 1.0 L/min, oxygen pressure: 0.1 MPa, solubility: 4.5%, anesthesia time: 5 minutes). The animal was grabbed with one hand and kept ventral side up. 10 µL of C48/80 solution (Sigma, C2313-100MG, 200 mg/mL, formulated with 0.9% saline) was added dropwise to the corneal surface of both eyes of the animal by a 10 µL pipette. The upper and lower eyelids were gently closed for 10 seconds to prevent the loss of the drug.

After 10 minutes of stimulation, different drug treatments were carried out according to the group settings. Both eyes of the same animal were subjected to the same drug treatment (10 µL per eye). The eyelids were closed for 10 seconds by the same method. Ophthalmological examination was performed 20 minutes after administration by a handheld slit lamp microscope (Jiangxi Jiangfeng, LYL-S). The clinical score was evaluated individually on the eyes of each animal according to the McDonald-Shadduck scoring system (GB/T 28538-2012).

The results are shown in Figure 2. According to the evaluation principle of the McDonald-Shadduck scoring system, the score value is positively correlated with the severity of ocular inflammation. The compound of Example 4 of the present invention has a good therapeutic effect on the allergic conjunctivitis in Wistar rats induced by C48/80.

## Claims

1. A compound of formula (I): 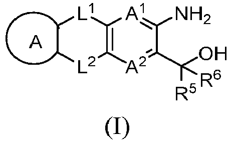
or a mesomer, racemate, enantiomer, diastereomer thereof, or mixture thereof, or a prodrug thereof, or a pharmaceutically acceptable salt thereof,
wherein,
A¹ is selected from the group consisting of N and CR¹;
A² is selected from the group consisting of N and CR²;
L¹ and L² are each independently selected from the group consisting of single bond, CR^{c}R^{d}, NR^{c}, O and S(O)ₘ, and L¹ and L² are not single bonds simultaneously;
ring A is selected from the group consisting of aromatic ring, heteroaromatic ring and heterlcyclic ring, wherein the aromatic ring, heteroaromatic ring and heterlcyclic ring are each optionally further substituted by one or more substituents selected from the group consisting of halogen, amino, nitro, cyano, oxo, hydroxy, thiol, carboxy, ester group, alkyl, alkoxy, alkenyl, alkynyl, cycloalkyl, heterocyclyl, aryl, heteroaryl, -C(O)R^{a}, -O(O)CR^{a}, -C(O)OR^{a}, -C(O)NR^{a}R^{b}, -NHC(O)R^{a}, -S(O)ₘR^{a}, -S(O)ₘNR^{a}R^{b} and -NHS(O)ₘR^{a};
R¹ and R² are each independently selected from the group consisting of hydrogen, halogen, amino, nitro, cyano, hydroxy, thiol, oxo, alkyl, alkoxy, cycloalkyl, heterocyclyl, aryl, heteroaryl, -C(O)R^{a}, -O(O)CR^{a}, -C(O)OR^{a}, -C(O)NR^{a}R^{b}, -NHC(O)R^{a}, -S(O)ₘR^{a}, -S(O)ₘNR^{a}R^{b} and -NHS(O)ₘR^{a};
R^{c} and R^{d} are each independently selected from the group consisting of hydrogen, halogen, amino, nitro, cyano, oxo, hydroxy, thiol, carboxy, ester group, alkyl, alkoxy, alkenyl, alkynyl, cycloalkyl, heterocyclyl, aryl, heteroaryl, -C(O)R^{a}, -O(O)CR^{a}, -C(O)OR^{a}, -C(O)NR^{a}R^{b}, -NHC(O)R^{a}, -S(O)ₘR^{a}, -S(O)ₘNR^{a}R^{b} and -NHS(O)ₘR^{a}, wherein the alkyl, alkoxy, alkenyl, alkynyl, cycloalkyl, heterocyclyl, aryl and heteroaryl are each optionally further substituted by one or more substituents selected from the group consisting of halogen, amino, nitro, cyano, hydroxy, thiol, oxo, alkyl, alkoxy, cycloalkyl, heterocyclyl, aryl and heteroaryl;
R⁵ and R⁶ are each independently selected from the group consisting of hydrogen, halogen, amino, cyano, hydroxy, thiol, alkyl, alkoxy, alkenyl, alkynyl, cycloalkyl, heterocyclyl, aryl and heteroaryl, wherein the alkyl, alkoxy, alkenyl, alkynyl, cycloalkyl, heterocyclyl, aryl and heteroaryl are each optionally further substituted by one or more substituents selected from the group consisting of halogen, amino, nitro, cyano, hydroxy, thiol, carboxy, ester group, oxo, alkyl, alkoxy, alkenyl, alkynyl, cycloalkyl, heterocyclyl, aryl and heteroaryl;
or, R⁵ and R⁶, together with the carbon atom to which they are attached, form a cycloalkyl or heterocyclyl, wherein the cycloalkyl or heterocyclyl is optionally further substituted by one or more substituents selected from the group consisting of halogen, amino, nitro, cyano, hydroxy, thiol, carboxy, ester group, oxo, alkyl, alkoxy, alkenyl, alkynyl, cycloalkyl, heterocyclyl, aryl and heteroaryl;
R^{a} and R^{b} are each independently selected from the group consisting of hydrogen, halogen, hydroxy, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are each optionally further substituted by one or more substituents selected from the group consisting of halogen, amino, nitro, cyano, hydroxy, thiol, carboxy, ester group, oxo, alkyl, alkoxy, alkenyl, alkynyl, cycloalkyl, heterocyclyl, aryl and heteroaryl;
or, R^{a} and R^{b}, together with the nitrogen atom to which they are attached, form a nitrogen-containing heterocyclyl, wherein the nitrogen-containing heterocyclyl is optionally further substituted by one or more substituents selected from the group consisting of halogen, amino, nitro, cyano, oxo, hydroxy, thiol, carboxy, ester group, alkyl, alkoxy, alkenyl, alkynyl, cycloalkyl, heterocyclyl, aryl and heteroaryl;
m is an integer from 0 to 2.

2. The compound of formula (I) according to claim 1,
wherein,
ring A is selected from the group consisting of aromatic ring and heteroaromatic ring, preferably 6-membered aromatic ring or 5- to 6-membered heteroaromatic ring, more preferably benzene ring or pyridine ring, wherein the aromatic ring and heteroaromatic ring are each optionally further substituted by one or more substituents selected from the group consisting of halogen, amino, nitro, cyano, oxo, hydroxy, thiol, carboxy, ester group, alkyl, alkoxy, alkenyl, alkynyl, cycloalkyl, heterocyclyl, aryl, heteroaryl, -C(O)R^{a}, -O(O)CR^{a}, -C(O)OR^{a}, -C(O)NR^{a}R^{b}, -NHC(O)R^{a}, -S(O)ₘR^{a}, -S(O)ₘNR^{a}R^{b} and -NHS(O)ₘR^{a}, preferably substituted by halogen(s);
R^{a} and R^{b} are each independently selected from the group consisting of hydrogen, halogen, hydroxy, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are each optionally further substituted by one or more substituents selected from the group consisting of halogen, amino, nitro, cyano, hydroxy, thiol, carboxy, ester group, oxo, alkyl, alkoxy, alkenyl, alkynyl, cycloalkyl, heterocyclyl, aryl and heteroaryl;
or, R^{a} and R^{b}, together with the nitrogen atom to which they are attached, form a nitrogen-containing heterocyclyl, wherein the nitrogen-containing heterocyclyl is optionally further substituted by one or more substituents selected from the group consisting of halogen, amino, nitro, cyano, oxo, hydroxy, thiol, carboxy, ester group, alkyl, alkoxy, alkenyl, alkynyl, cycloalkyl, heterocyclyl, aryl and heteroaryl;
m is an integer from 0 to 2.

3. The compound of formula (I) according to claim 1 or 2,
wherein,
A¹ is selected from CR¹; and
A² is selected from the group consisting of N and CR²;
R¹ and R² are each independently selected from the group consisting of hydrogen, halogen, amino, nitro, cyano, hydroxy, thiol, oxo, alkyl, alkoxy, cycloalkyl, heterocyclyl, aryl, heteroaryl, -C(O)R^{a}, -O(O)CR^{a}, -C(O)OR^{a}, -C(O)NR^{a}R^{b}, -NHC(O)R^{a}, -S(O)ₘR^{a}, -S(O)ₘNR^{a}R^{b} and -NHS(O)ₘR^{a};
R^{a}, R^{b} and m are as defined in claim 1.

4. The compound of formula (I) according to any one of claims 1 to 3, being a compound of formula (II) 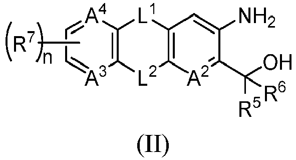 wherein,
A² is N or CH;
A³ is N or CH;
A⁴ is N or CH;
L¹ and L² are each independently selected from the group consisting of single bond, CR^{c}R^{d}, NR^{c}, O and S(O)ₘ, and L¹ and L² are not single bonds simultaneously;
each R⁷ is independently selected from the group consisting of hydrogen, halogen, amino, nitro, cyano, oxo, hydroxy, thiol, carboxy, ester group, alkyl, alkoxy, alkenyl, alkynyl, cycloalkyl, heterocyclyl, aryl, heteroaryl, -C(O)R^{a}, -O(O)CR^{a}, -C(O)OR^{a}, -C(O)NR^{a}R^{b}, -NHC(O)R^{a}, -S(O)ₘR^{a}, -S(O)ₘNR^{a}R^{b} and -NHS(O)ₘR^{a};
n is 0, 1, 2 or 3;
R⁵, R⁶, R^{a}, R^{b}, R^{c}, R^{d} and m are as defined in claim 1.

5. The compound of formula (I) according to any one of claims 1 to 4,
wherein,
L¹ is selected from the group consisting of single bond, CR^{c}R^{d}, NR^{c} and O;
L² is selected from the group consisting of single bond, NR^{c} and O;
R^{c} and R^{d} are each independently selected from the group consisting of hydrogen, halogen, C₁-C₆ alkyl, C₁-C₆ alkoxy, -O(O)CR^{a} and benzyl; R^{a} is selected from C₁-C₆ alkyl.

6. The compound of formula (I) according to any one of claims 1 to 5,
wherein,
R⁵ and R⁶ are each independently selected from the group consisting of hydrogen, halogen, amino, C₁-C₆ alkyl and C₃-C₆ cycloalkyl, wherein the C₁-C₆ alkyl is optionally further substituted by one or more substituents selected from halogen;
or, R⁵ and R⁶, together with the carbon atom to which they are attached, form a C₃-C₆ cycloalkyl or 5- to 7-membered heterocyclyl, wherein the cycloalkyl or heterocyclyl is optionally further substituted by one or more substituents selected from the group consisting of halogen, amino, nitro, cyano, hydroxy, thiol, carboxy, ester group, oxo, alkyl, alkoxy, alkenyl, alkynyl, cycloalkyl, heterocyclyl, aryl and heteroaryl;
preferably, R⁵ and R⁶ are C₁-C₆ alkyl.

7. The compound of formula (I) according to any one of claims 1 to 6, wherein the compound is selected from the group consisting of:

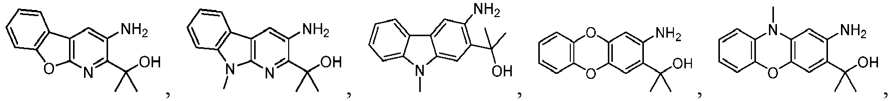

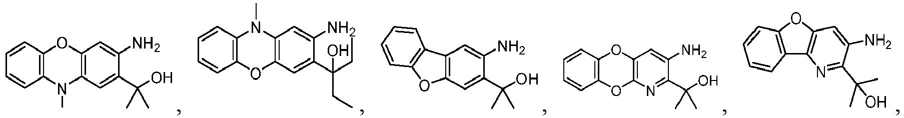

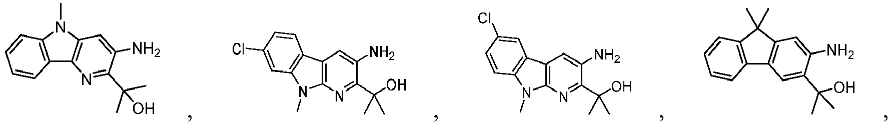

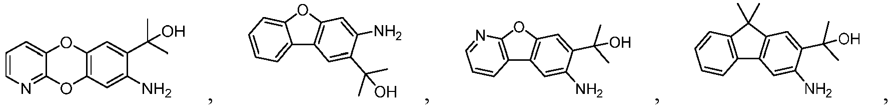

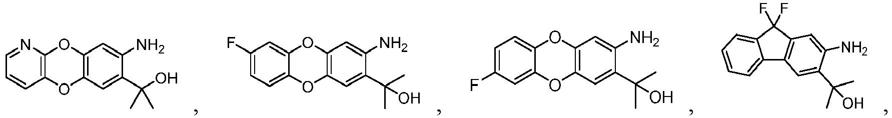

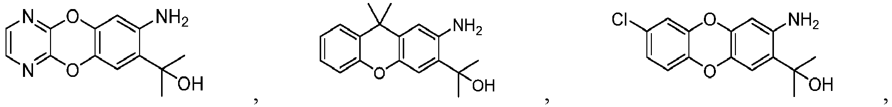

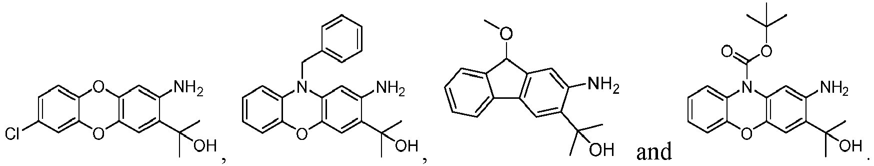

8. A method for preparing the compound of formula (I) according to any one of claims 1 to 7, comprising the following step of: 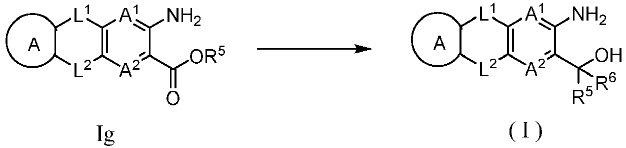
reacting compound Ig with an alkyl Grignard reagent to obtain the compound of formula (I), wherein the alkyl Grignard reagent is preferably methylmagnesium chloride or methylmagnesium bromide;
wherein A', A², ring A, L¹, L², R⁵ and R⁶ are as defined in claim 1.

9. A pharmaceutical composition comprising the compound of formula (I) according to any one of claims 1 to 7 and a pharmaceutically acceptable carrier.

10. Use of the compound of formula (I) according to any one of claims 1 to 7, or the pharmaceutical composition according to claim 9 in the preparation of a toxic aldehyde capture agent.

11. Use of the compound of formula (I) according to any one of claims 1 to 7, or the pharmaceutical composition according to claim 9 in the preparation of medicaments for the prevention and/or treatment of diseases related to active carbonyl compound; the disease is preferably ocular disease, skin disease, autoimmune disease, digestive system disease, cardiovascular disease, respiratory system disease, neurodegenerative disease, obesity, cancer, and aging-related disease; and the ocular disease is preferably noninfectious uveitis, allergic conjunctivitis and dry eye.
